# EUROPEAN PATENT APPLICATION

(11) **EP 2 272 841 A1**
(43) Date of publication of application: **12.01.2011**
(21) Application number: 09725429.6
(22) Date of filing: 26.03.2009
(51) Int. Cl.: C07D 401/06, A61K 31/437, A61K 31/438, A61K 31/44, A61K 31/444, A61K 31/5025, A61P 1/00, A61P 1/16, A61P 3/04, A61P 3/06, A61P 3/10, A61P 3/12, A61P 9/04, A61P 9/10, A61P 9/12, A61P 11/00, A61P 13/12, A61P 15/08, A61P 17/00, A61P 19/06, A61P 25/02

(54) **DIARYLMETHYLAMIDE DERIVATIVE HAVING ANTAGONISTIC ACTIVITY ON MELANIN-CONCENTRATING HORMONE RECEPTOR**

(30) Priority: 28.03.2008 JP 2008085606
(71) Applicant: Banyu Pharmaceutical Co., Ltd., Chiyoda-ku Tokyo 102-8667 (JP)
(72) Inventor: KAMIJYO, Kaori, Tsukuba-shi Ibaraki 300-2611 (JP); MORIYA, Minoru, Tsukuba-shi Ibaraki 300-2611 (JP); SUZUKI, Takao, Tsukuba-shi Ibaraki 300-2611 (JP); TAKEZAWA, Akihiro, Tsukuba-shi Ibaraki 300-2611 (JP)
(74) Representative: Buchan, Gavin MacNicol
(86) International application number: PCT/JP2009/056096
(87) International publication number: WO 2009/119726

(57) **Abstract**

[Problem]

To provide a melanin-concentrating hormone receptor antagonist useful as a pharmaceutical agent for central diseases, circulatory diseases, and metabolic diseases.

[Means for Resolution]

Provided is a diarylmethylamide derivative represented by formula (I): 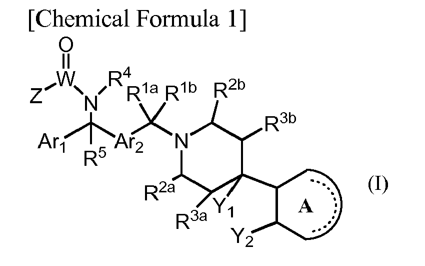 Wherein R^{1a}, R^{1b}, R^{2a}, R^{2b}, R^{3a}, and R^{3b} independently represent a hydrogen atom or the like, R⁴ represents a hydrogen atom, C₁₋₆ alkyl, or the like, R⁵ represents a hydrogen atom or the like, Z represents C₁₋₆ alkyl or the like, or R⁴ and Z together form a 4- to 6-membered nitrogen-containing hetero ring, Y₁ represents H or the like, Y₂ represents H, or Y₁ and Y₂ together form - O-CH₂-, W represents C, SO, or the like, Ar₁ represents 6-membered aryl or the like, Ar₂ represents 6-membered aryl or the like, and ring A represents a benzene ring, a pyridine ring, or the like.

## Description

### TECHNICAL FIELD

The present invention relates to a novel diarylmethylamide derivative. The compound acts as a melanin-concentrating hormone receptor antagonist, and is useful as an agent for the prevention, treatment, or remedy of various circulatory diseases, neurological diseases, metabolic diseases, reproductive system diseases, respiratory diseases, digestive diseases, and the like.

### BACKGROUND ART

Melanin-concentrating hormone (MCH) is cyclic peptide hormone/neuropeptide that was first isolated from the hypophysis of salmon by Kawauchi et al., in 1983 [see Nature, Vol. 305, 321 (1983)], and is known to functionally antagonize melanocyte-stimulating hormone in the fishes to cause condensation of melanin granules in the melanophore, thus being involved in the body color change [see International Review of Cytology, Vol. 126, 1 (1991); Trends in Endocrinology and Metabolism, Vol. 5, 120 (1994)]. Further, in the mammals, although MCH-containing neuron cell bodies are localized in the lateral hypothalamic area and the zona incerta, the nerve fibers thereof project to an extremely large area in the brain [see The Journal of Comparative Neurology, Vol. 319, 218 (1992)], and MCH is believed to be responsible for various central biological functions.

The lateral hypothalamic area has been known as the feeding center from long ago, and further, in recent years, much molecular biological/pharmacological knowledge suggesting involvement of MCH in the energy homeostasis control has been accumulated. Specifically, it has been reported that expression of mRNA, an MCH precursor, is accelerated in the brain of genetic obesity model animals, i.e., ob/ob mice, db/db mice, KKAy mice, Zucker fatty rats, and fasted mice [see Nature, Vol. 380, 243 (1996); Diabetes, Vol. 47, 294 (1998); Biochemical and Biophysical Research Communications, Vol. 268, 88 (2000); Molecular Brain Research, Vol. 92, 43 (2001)].

As a result of acute intraventricular administration of MCH to rats, an increase in food intake is observed [Nature, Vol. 380, 243 (1996)], and chronic administration leads to obesity accompanied by bulimia [see Proceedings of the National Academy of Sciences of the United States of America, Vol. 99, 3240 (2002)]. Further, in mice lacking MCH precursor gene, as compared with wild mice, reduced food consumption and increased oxygen consumption per body weight are seen, and also low body weight due to reduction in body fat is observed [see Nature, Vol. 396,670 (1998)].

Meanwhile, transgenic mice that overexpress MCH precursor develop obesity, which is accompanied by bulimia, and insulin resistance [see The Journal of Clinical Investigation, Vol. 107, 379 (2001)]. This accordingly suggests that MCH is an important factor in the development of obesity, and is also involved in metabolic disorders and respiratory diseases for which obesity is a risk factor. In addition, MCH is known to be involved in, for example, anxiogenesis, epilepsy, memory/learning, diuresis, sodium/potassium excretion, oxytocin secretion, and reproduction/reproductive function [see Peptides, Vol. 17, 171 (1996); Peptides, Vol. 18, 1095 (1997); Peptides, Vol. 15, 757 (1994); Journal of Neuroendocrinology, Vol. 8, 57 (1996) Critical Reviews in Neurobiology, Vol. 8, 221 (1994)].

MCH causes various pharmacological effects via MCH receptors existing mainly in the central nervous system. As MCH receptors, at least two types are known: type 1 receptor (MCH-1R or SLC-1) and type 2 receptor (MCH-2R or SLT) [see Nature, Vol. 400, 261 (1999); Nature, Vol. 400, 265 (1999); Biochemical and Biophysical Research Communications, Vol. 261, 622 (1999); Nature Cell Biology, Vol. 1, 267 (1999); FEBS Letters, Vol. 457, 522 (1999); Biochemical and Biophysical Research Communications, Vol. 283, 1013 (2001); The Journal of Biological Chemistry, Vol. 276, 20125 (2001); Proceedings of the National Academy of Sciences of the United States of America, Vol. 98, 7564 (2001); Proceedings of the National Academy of Sciences of the United States of America, Vol. 98, 7576 (2001); The Journal of Biological Chemistry, Vol. 276, 34664 (2001); Molecular Pharmacology, Vol. 60, 632 (2001)].

In particular, pharmacological effects observed in the rodents are caused mainly via MCH-1R [see Genomics, Vol. 79, 785 (2002)]. From the fact that chronic administration of MCH to mice lacking MCH-1R gene does not induce bulimia or obesity, the energy metabolism control by MCH is known to be caused via MCH-1R. Further, it is known that deficiency of MCH-1R gene increases the amount of activity of a mouse [see Proceedings of the National Academy of Sciences of the United States of America, Vol. 99, 3240 (2002)], and also, involvement in central diseases accompanied by behavior disorder, such as attention deficit/hyperactivity disorder, schizophrenia, depression, and the like, is strongly suggested [see Molecular Medicine Today, Vol. 6, 43 (2000); Trends in Neuroscience, Vol. 24, 527 (2001)].

In addition, the presence of an autoantibody against MCH-1R has been reported in the blood serum of vitiligo vulgaris patients [see The Journal of Clinical Investigation, Vol. 109, 923 (2002)]. MCH-1R expression in certain kinds of cancer cells has also been reported, and, in light of the MCH and MCH-1R expression sites in vivo, involvement of MCH in cancer, sleep/waking, drug dependence, and digestive diseases has also been suggested [see Biochemical and Biophysical Research Communications, Vol. 289, 44 (2001); Neuroendocrinology, Vol. 61, 348 (1995); Endocrinology, Vol. 137, 561 (1996); The Journal of Comparative Neurology, Vol. 435, 26 (2001)].

The function of MCH is expressed by MCH binding to an MCH receptor. Accordingly, inhibition of binding of MCH to its receptor will inhibit the expression of MCH activity. Therefore, substances that antagonize binding of MCH to its receptor are expected to be useful as preventive or remedy for various MCH-related diseases, including metabolic diseases such as obesity, diabetes, hormone secretion disorder, hyperlipemia, gout, fatty liver, and like; circulatory diseases such as angina pectoris, acute/congestive cardiac insufficiency, myocardial infarction, coronary arteriosclerosis, hypertension, nephropathy, electrolyte abnormality, and like; central and peripheral nervous system diseases such as bulimia, affective disorder, depression, anxiety, epilepsy, delirium, dementia, schizophrenia, attention deficit/hyperactivity disorder, dysmnesia, somnipathy, cognitive impairment, dyskinesia, dysesthesia, dysosmia, morphine resistance, drug dependence, alcohol dependence, and like; reproductive system diseases such as infertility, premature delivery, sexual dysfunction, and like; and other conditions including digestive diseases, respiratory diseases, cancer, chromatosis, and the like.

As compounds having MCH receptor antagonism, for example, WO 03/004027 (Patent Document 1) and US 2006/079683 (Patent Document 2) disclose a number ofN-benzyl-4-phenylpiperidine derivatives. However, these specifications nowhere disclose a compound having an amide group bonded to methylene between two aryl groups, which is the feature of the invention.
Patent Document 1: WO 03/004027 pamphlet
Patent Document 2: US 2006/079683 pamphlet

### DISCLOSURE OF THE INVENTION

### PROBLEMS THAT THE INVENTION IS TO SOLVE

The present inventors conducted extensive research on compounds having MCH receptor antagonism. As a result, they found that compounds having piperidine bonded through methylene to one of the aryl groups of diarylmethylamide are heretofore unknown, novel compounds, and that such compounds have MCH receptor antagonism and are effective in the prevention or treatment of various MCH-receptor-related diseases, and thus accomplished the invention.

Specifically, the invention provides:
(1) a diarylmethylamide derivative represented by formula (I) or a pharmaceutically acceptable salt thereof:

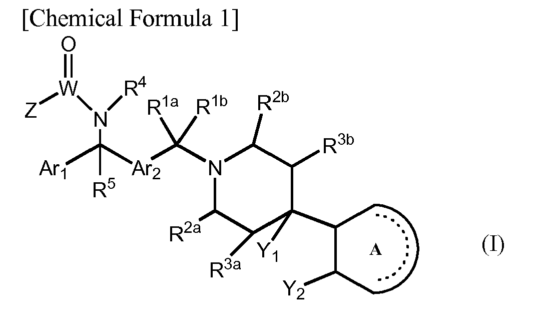

wherein:
R^{1a} and R^{1b} independently represent a hydrogen atom or C₁₋₆ alkyl,
R^{2a} and R^{2b} independently represent a hydrogen atom or C₁₋₆ alkyl,
R^{3a} and R^{3b} independently represent a hydrogen atom or C₁₋₆ alkyl,
R⁴ represents a hydrogen atom, hydroxy, C₁₋₆ alkyl, C₃₋₆ cycloalkyl, or C₁₋₆ alkoxy, wherein the alkyl, cycloalkyl, or alkoxy being optionally substituted with halogen, hydroxy, or C₁₋₆ alkoxy,
R⁵ represents a hydrogen atom, C₁₋₆ alkyl, or C₃₋₆ cycloalkyl, wherein the alkyl or cycloalkyl being optionally substituted with halogen, hydroxy, or C₁₋₆ alkoxy,
Z represents C₁₋₆ alkyl, C₃₋₆ cycloalkyl, C₁₋₆ alkoxy, aryl, heteroaryl, or N(R^{6a})(R^{6b}), wherein the alkyl, cycloalkyl, alkoxy, aryl, or heteroaryl being optionally substituted with halogen, hydroxy, C₁₋₆ alkyl, halo C₁₋₆ alkyl, C₁₋₆ alkoxy, or halo C₁₋₆ alkoxy, or,
R⁴ and Z together form, together with the nitrogen atom to which R⁴ is bonded, a 4- to 6-membered nitrogen-containing hetero ring, wherein the nitrogen-containing hetero ring optionally containing a double bond in the ring and optionally further containing a heteroatom selected from the group consisting of nitrogen, oxygen, and sulfur, the nitrogen-containing hetero ring being optionally fused with an aryl ring or a heteroaryl ring, and the nitrogen-containing hetero ring being optionally substituted with halogen, hydroxy, C₁₋₆ alkyl, halo C₁₋₆ alkyl, C₁₋₆ alkoxy, halo C₁₋₆ alkoxy, or oxo,
R^{6a} and R^{6b} independently represent a hydrogen atom or C₁₋₆ alkyl, or, R^{6a} and R^{6b} together form, together with the nitrogen atom to which they are bonded, a 5- to 6-membered nitrogen-containing hetero ring, wherein the nitrogen-containing hetero ring optionally further containing a heteroatom selected from the group consisting of nitrogen, oxygen, and sulfur and being optionally substituted with halogen, hydroxy, C₁₋₆ alkyl, halo C₁₋₆ alkyl, C₁₋₆ alkoxy, halo C₁₋ ₆ alkoxy, or oxo,
Y₁ represents H or -OR^{7a},
Y₂ represents H, or Y₁ and Y₂ together form -O-C(R^{7b})(R^{7c})-,
R^{7a}, R^{7b}, and R^{7c} each independently represent a hydrogen atom or C₁₋₆ alkyl,
W represents C, S, or SO,
Ar₁ represents 6-membered aryl or 6-membered nitrogen-containing heteroaryl, wherein the aryl or nitrogen-containing heteroaryl being optionally substituted with a substituent selected from the group consisting of group α,
Ar₂ is a divalent group and represents 6-membered aryl or 5- to 6-membered heteroaryl, wherein the aryl or heteroaryl being optionally substituted with a substituent selected from the group consisting of group α, and
   a formula:

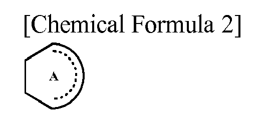

represents a 6-membered aryl ring or a 5- to 6-membered nitrogen-containing hetero ring, wherein the aryl ring or nitrogen-containing hetero ring being optionally fused with a 5- to 6-membered aryl ring or heteroaryl ring, wherein the aryl ring or nitrogen-containing hetero ring being optionally substituted with halogen, cyano, hydroxy, C₁₋₆ alkyl, halo C₁₋₆ alkyl, C₁₋₆ alkoxy, halo C₁₋₆ alkoxy, C₁₋₆ alkylcarbonylamino, or oxo.

Substituents of group α are:
halogen, cyano, hydroxy, amino, mono C₁₋₆ alkylamino, di-C₁₋₆ alkylamino, C₁₋₆ alkyl, halo C₁₋₆ alkyl, C₁₋₆ alkoxy, halo C₁₋₆ alkoxy, C₁₋₆ alkoxy C₁₋₆ alkyl, C₁₋₆ alkoxycarbonyl, C₁₋₆ alkoxycarbonylamino, C₁₋₆ alkoxycarbonyl(C₁₋₆ alkyl)amino, C₁₋₆ alkylcarbonyl, C₁₋₆ alkylcarbonyloxy, C₁₋₆ alkylcarbonylamino, C₁₋₆ alkylcarbonyl(C₁₋₆ alkyl)amino, carbamoyl, mono-C₁₋₆ alkylcarbamoyl, di-C₁₋₆ alkylcarbamoyl, carbamoylamino, mono-C₁₋₆ alkylcarbamoylamino, di-C₁₋₆ alkylcarbamoylamino, mono-C₁₋₆ alkylcarbamoyl(C₁₋₆ alkyl)amino, di-C₁₋₆ alkylcarbamoyl(C₁₋₆ alkyl)amino, carbamoyloxy, mono-C₁₋₆ alkylcarbamoyloxy, di-C₁₋₆ alkylcarbamoyloxy, C₁₋₆ alkylsulfonyl, C₁₋₆ alkylsulfonylamino, C₁₋₆ alkylsulfonyl(C₁₋₆ alkyl)amino, sulfamoyl, mono-C₁₋₆ alkylsulfamoyl, di-C₁₋₆ alkylsulfamoyl, sulfamoylamino, mono-C₁₋₆ alkylsulfamoylamino, di-C₁₋₆ alkylsulfamoylamino, mono-C₁₋₆ alkylsulfamoyl(C₁₋₆ alkyl)amino, and di-C₁₋₆ alkylsulfamoyl(C₁₋₆ alkyl)amino.

The invention also provides:
(2) a melanin-concentrating hormone receptor antagonist comprising as an active ingredient the compound according to (1),
(3) a pharmaceutical composition comprising a pharmaceutically acceptable additive and the compound according to (1),
(4) a preventive or remedy for obesity, diabetes, fatty liver, bulimia, depression, or anxiety, comprising as an active ingredient the compound according to (1), and
(5) a medicine based on melanin-concentrating hormone receptor antagonism, comprising as an active ingredient the compound according to (1).

Hereinafter, the invention will be described in further detail.

Examples of "halogen" include a fluorine atom, a chlorine atom, a bromine atom, and an iodine atom.

The term "C₁₋₆ alkyl" encompasses straight alkyl having a carbon number of 1 to 6 and branched alkyl having a carbon number of 3 to 6. Specific examples thereof include methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl, 1-ethyl-1-methylpropyl, and the like.

The term "C₃₋₆ cycloalkyl" means cycloalkyl having a carbon number of 3 to 6, examples thereof including cyclopropyl, cyclobutyl, cyclopentyl, and cyclohexyl.

The term "halo C₁₋₆ alkyl" encompasses C₁₋₆ alkyl with the hydrogen atoms thereof being partially or completely substituted with halogen, examples thereof including fluoromethyl, difluoromethyl, trifluoromethyl, 2-fluoroethyl, 1,2-difluoroethyl, and the like.

The term "C₁₋₆ alkoxy" encompasses groups having C₁₋₆ alkyl bonded to an oxygen atom. Specific examples thereof include methoxy, ethoxy, n-propyloxy, isopropyloxy, n-butoxy, isobutoxy, tert-butoxy, n-pentyloxy, and the like.

The term "halo C₁₋₆ alkoxy" encompasses groups having halo C₁₋₆ alkyl bonded to an oxygen atom. Specific examples thereof include fluoromethoxy, chloromethoxy, difluoromethoxy, dichloromethoxy, trifluoromethoxy, trichloromethoxy, 2-fluoroethoxy, 1,2-difluoroethoxy, and the like.

The term "mono-C₁₋₆ alkylamino" means a group with one of the hydrogen atoms of amino (-NH₂) being substituted with a C₁₋₆ alkyl group. Specific examples thereof include methylamino, ethylamino, n-propylamino, isopropylamino, n-butylamino, sec-butylamino, tert-butylamino, and the like.

The term "di-C₁₋₆ alkylamino" means a group with the two amino hydrogen atoms each being substituted with a C₁₋₆ alkyl group. Specific examples thereof include dimethylamino, diethylamino, ethylmethylamino, di(n-propyl)amino, methyl(n-propyl)amino, diisopropylamino, and the like.

The term "C₁₋₆ alkoxy C₁₋₆ alkyl" means C₁₋₆ alkyl substituted with C₁₋₆ alkoxy. Specific examples thereof include methoxymethyl, ethoxymethyl, n-propyloxymethyl, isopropyloxymethyl, 1-methoxyethyl, 2-methoxyethyl, and the like.

The term "C₁₋₆ alkoxycarbonyl" means a group having C₁₋₆ alkoxy bonded to carbonyl (-CO-), and encompasses alkoxycarbonyl having a carbon number of 1 to 6. Specific examples thereof include methoxycarbonyl, ethoxycarbonyl, n-propyloxycarbonyl, isopropyloxycarbonyl, n-butoxycarbonyl, isobutoxycarbonyl, tert-butoxycarbonyl, n-pentyloxycarbonyl, and the like.

The term "C₁₋₆ alkoxycarbonylamino" means a group with one of the amino hydrogen atoms being substituted with C₁₋₆ alkoxycarbonyl and encompasses alkoxycarbonylamino having a carbon number of 1 to 6. Specific examples thereof include methoxycarbonylamino, ethoxycarbonylamino, n-propyloxycarbonylamino, isopropyloxycarbonylamino, n-butoxycarbonylamino, isobutoxycarbonylamino, tert-butoxycarbonylamino, n-pentyloxycarbonylamino, and the like.

The term "C₁₋₆ alkoxycarbonyl(C₁₋₆ alkyl)amino" means a group having bonded thereto C₁₋₆ alkoxycarbonyl in place of the hydrogen atom on the nitrogen atom of mono-C₁₋₆ alkylamino. Specific examples thereof include methoxycarbonyl(methyl)amino, ethoxycarbonyl(methyl)amino, n-propyloxycarbonyl(methyl)amino, and the like.

The term "C₁₋₆ alkylcarbonyl" means groups having C₁₋₆ alkyl bonded to carbonyl, and encompasses alkylcarbonyl having a carbon number of 1 to 6. Specific examples thereof include acetyl, propionyl, butyryl, isobutyryl, valeryl, isovaleryl, pivaloyl, and the like.

The term "C₁₋₆ alkylcarbonyloxy" means a group having C₁₋₆ alkylcarbonyl bonded to an oxygen atom. Specific examples thereof include acetoxy, propionyloxy, valeryloxy, isovaleryloxy, pivaloyloxy, and the like.

The term "C₁₋₆ alkylcarbonylamino" means a group with one of the amino hydrogen atoms being substituted with C₁₋₆ alkylcarbonyl. Specific examples thereof include acetylamino, propionylamino, isobutyryl amino, valerylamino, isovalerylamino, pivaloylamino, and the like.

The term "C₁₋₆ alkylcarbonyl(C₁₋₆ alkyl)amino" means a group with the hydrogen atom on the nitrogen atom of mono-C₁₋₆ alkylamino being substituted with C₁₋₆ alkylcarbonyl, examples thereof including methylcarbonyl(methyl)amino, ethylcarbonyl(methyl)amino, n-propylcarbonyl(methyl)amino, and the like.

The term "mono-C₁₋₆ alkylcarbamoyl" means a group with one of the hydrogen atoms of carbamoyl (-CONH₂) being substituted with C₁₋₆ alkyl. Specific examples thereof include methylcarbamoyl, ethylcarbamoyl, n-propylcarbamoyl, isopropylcarbamoyl, n-butylcarbamoyl, sec-butylcarbamoyl, tert-butylcarbamoyl, and the like.

The term "di-C₁₋₆ alkylcarbamoyl" means a group with the two carbamoyl hydrogen atoms each being substituted with C₁₋₆ alkyl. Specific examples thereof include dimethylcarbamoyl, diethylcarbamoyl, ethylmethylcarbamoyl, di(n-propyl)carbamoyl, methyl(n-propyl)carbamoyl, diisopropylcarbamoyl, and the like.

The term "mono-C₁₋₆ alkylcarbamoylamino" means a group with one of the amino hydrogen atoms being substituted with mono-C₁₋₆ alkylcarbamoyl. Specific examples thereof include methylcarbamoylamino, ethylcarbamoylamino, n-propylcarbamoylamino, isopropylcarbamoylamino, n-butylcarbamoylamino, sec-butylcarbamoylamino, tert-butylcarbamoylamino, and the like.

The term "di-C₁₋₆ alkylcarbamoylamino" means a group with one of the amino hydrogen atoms being substituted with di-C₁₋₆ alkylcarbamoyl. Specific examples thereof include dimethylcarbamoylamino, diethylcarbamoylamino, di(n-propyl)carbamoylamino, diisopropylcarbamoylamino, di(n-butyl)carbamoylamino, di(sec-butyl)carbamoylamino, di(tert-butyl)carbamoylamino, and the like.

The term "mono-C₁₋₆ alkylcarbamoyl(C₁₋₆ alkyl)amino" means a group with the hydrogen atom on the nitrogen atom of mono-C₁₋₆ alkylamino being substituted with mono-C₁₋₆ alkylcarbamoyl. Specific examples thereof include monomethylcarbamoyl(methyl)amino, monoethylcarbamoyl(methyl)amino, [mono-(n-propyl)carbamoyl](methyl)amino, and the like.

The term "di-C₁₋₆ alkylcarbamoyl(C₁₋₆ alkyl)amino" means a group with the hydrogen atom on the nitrogen atom of mono-C₁₋₆ alkylamino being substituted with di-C₁₋₆ alkylcarbamoyl. Specific examples thereof include dimethylcarbamoyl(methyl)amino, diethylcarbamoyl(methyl)amino, [di(n-propyl)carbamoyl](methyl)amino, and the like.

The term "mono-C₁₋₆ alkylcarbamoyloxy" means a group having mono-C₁₋₆ alkylcarbamoyl bonded to an oxygen atom. Specific examples thereof include methylcarbamoyloxy, ethylcarbamoyloxy, n-propylcarbamoyloxy, isopropylcarbamoyloxy, n-butylcarbamoyloxy, sec-butylcarbamoyloxy, tert-butylcarbamoyloxy, and the like.

The term "di-C₁₋₆ alkylcarbamoyloxy" means a group having di-C₁₋₆ alkylcarbamoyl bonded to an oxygen atom. Specific examples thereof include dimethylcarbamoyloxy, diethylcarbamoyloxy, ethylmethylcarbamoyloxy, di(n-propyl)carbamoyloxy, methyl(n-propyl)carbamoyloxy, diisopropylcarbamoyloxy, and the like.

The term "C₁₋₆ alkylsulfonyl" means a group having C₁₋₆ alkyl bonded to sulfonyl (-SO₂-). Specific examples thereof include methanesulfonyl, ethanesulfonyl, n-propanesulfonyl, isopropanesulfonyl, n-butanesulfonyl, sec-butanesulfonyl, tert-butanesulfonyl, and the like.

The term "C₁₋₆ alkylsulfonylamino" means a group with one of the amino hydrogen atoms being substituted with C₁₋₆ alkylsulfonyl. Specific examples thereof include methanesulfonylamino, ethanesulfonylamino, n-propanesulfonylamino, isopropanesulfonylamino, n-butanesulfonylamino, sec-butanesulfonylamino, tert-butanesulfonylamino, and the like.

The term "C₁₋₆ alkylsulfonyl(C₁₋₆ alkyl)amino" means a group with the hydrogen atom on the nitrogen atom of mono-C₁₋₆ alkylamino being substituted with C₁₋₆ alkylsulfonyl. Specific examples thereof include methanesulfonyl(methyl)amino, ethanesulfonyl(methyl)amino, n-propanesulfonyl(methyl)amino, isopropanesulfonyl(methyl)amino, and the like.

The term "mono-C₁₋₆ alkylsulfamoyl" means a group with one of the hydrogen atoms of sulfamoyl (-SO₂NH₂) being substituted with C₁₋₆ alkyl. Specific examples thereof include monomethylsulfamoyl, monoethylsulfamoyl, mono(n-propyl)sulfamoyl, monoisopropylsulfamoyl, mono(n-butyl)sulfamoyl, mono(sec-butyl)sulfamoyl, mono(tert-butyl)sulfamoyl, and the like.

The term "di-C₁₋₆ alkylsulfamoyl" means a group with the two sulfamoyl hydrogen atoms each being substituted with C₁₋₆ alkyl. Specific examples thereof include dimethylsulfamoyl, diethylsulfamoyl, di(n-propyl)sulfamoyl, diisopropylsulfamoyl, di(n-butyl)sulfamoyl, di(sec-butyl)sulfamoyl, di(tert-butyl)sulfamoyl, and the like.

The term "mono-C₁₋₆ alkylsulfamoylamino" means a group with one of the amino hydrogen atoms being substituted with C₁₋₆ alkylsulfamoyl. Specific examples thereof include (monomethylsulfamoyl)amino, (monoethylsulfamoyl)amino, [mono(n-propyl)sulfamoyl]amino, (monoisopropylsulfamoyl)amino, [mono(n-butyl)sulfamoyl]amino, [mono(sec-butyl)sulfamoyl]amino, [mono(tert-butyl)sulfamoyl]amino, and the like.

The term "(di-C₁₋₆ alkylsulfamoyl)amino" means a group with one of the amino hydrogen atoms being substituted with di-C₁₋₆ alkylsulfamoyl. Specific examples thereof include (dimethylsulfamoyl)amino, (diethylsulfamoyl)amino, (ethylmethylsulfamoyl)amino, [di(n-propyl)sulfamoyl]amino, [methyl(n-propyl)sulfamoyl]amino, (diisopropylsulfamoyl)amino, and the like.

The term "mono-C₁₋₆ alkylsulfamoyl(C₁₋₆ alkyl)amino" means a group with the hydrogen atom on the nitrogen atom of mono-C₁₋₆ alkylamino being substituted with mono-C₁₋₆ alkylsulfamoyl. Specific examples thereof include monomethylsulfamoyl(methyl)amino, monoethylsulfamoyl(methyl)amino, [mono-(n-propyl)sulfamoyl](methyl)amino, and the like.

The term "di-C₁₋₆ alkylsulfamoyl(C₁₋₆ alkyl)amino" means a group with the hydrogen atom on the nitrogen atom of mono-C₁₋₆ alkylamino being substituted with di-C₁₋₆ alkylsulfamoyl. Specific examples thereof include dimethylsulfamoyl(methyl)amino, diethylsulfamoyl(methyl)amino, [di(n-propyl)sulfamoyl](methyl)amino, and the like.

Examples of "aryl" include phenyl, naphthyl, tolyl, and the like.

The term "heteroaryl" means 5-membered or 6-membered monocyclic heteroaryl containing one or more, preferably one to three, same or different heteroatoms selected from the group consisting of an oxygen atom, a nitrogen atom, and a sulfur atom, or otherwise means condensed-ring heteroaryl formed by condensation of such monocyclic heteroaryl and the above-mentioned heteroaryl or alternatively by mutual condensation of the same or different monocyclic heteroaryl groups. Examples thereof include pyrrolyl, furyl, thienyl, imidazolyl, pyrazolyl, thiazolyl, isothiazolyl, oxazolyl, isoxazolyl, triazolyl, tetrazolyl, oxadiazolyl, 1,2,3-thiadiazolyl, 1,2,4-thiadiazolyl, 1,3,4-thiadiazolyl, pyridyl, pyrazinyl, pyrimidinyl, pyridazinyl, 1,2,4-triazinyl, 1,3,5-triazinyl, indolyl, benzofuranyl, benzothienyl, benzimidazolyl, benzopyrazolyl, benzoxazolyl, benzisoxazolyl, benzothiazolyl, benzisothiazolyl, indazolyl, purinyl, quinolyl, isoquinolyl, phthalazinyl, naphthyridinyl, quinoxalinyl, quinazolinyl, cinnolinyl, pteridinyl, pyrido[3,2-b]pyridyl, and the like.

The term "nitrogen-containing hetero ring" means a saturated, partially saturated, or unsaturated, monocyclic or bicyclic ring containing three to ten atoms including nitrogen, which optionally further contains an oxygen atom or a sulfur atom. Examples thereof include pyrrolidinyl, oxazolidinyl, thiazolydinyl, piperidinyl, morpholinyl, piperazinyl, pyrrolyl, imidazolyl, thiazolyl, triazolyl, indolyl, benzimidazolyl, pyridyl, pyrazinyl, pyrimidinyl, and the like.

A "pharmaceutically acceptable salt" of a derivative represented by formula (I) may be an ordinary salt that is pharmaceutically acceptable. Examples thereof include acid addition salts of the amine moiety of the compound of formula (I); acid addition salts of the nitrogen-containing heterocycle of the compound of formula (I); in the case where the compound of formula (I) contains an acidic substituent, base addition salts of such a group; etc.

Examples of such acid addition salts include inorganic acid salts such as hydrochloride, sulfate, nitrate, phosphate, perchlorate, and like; organic acid salts such as maleate, fumarate, tartrate, citrate, ascorbate, trifluoroacetate, and like; sulfonates such as methanesulfonate, isothiocyanate, benzenesulfonate, p-toluenesulfonate, and like; etc.

Examples of base addition salts include alkali metal salts such as sodium salt, potassium salt, and like; alkaline earth metal salts such as calcium salt, magnesium salt, and like; organic amine salts such as ammonium salt, trimethylamine salt, triethylamine salt, dicyclohexylamine salt, ethanolamine salt, diethanolamine salt, triethanolamine salt, procaine salt, N,N'-dibenzylethylenediamine salt, and like; etc.

Hereinafter, for more specific disclosure of the derivative of the invention, the symbols used in formula (I) will be explained with reference to specific examples.

R^{1a} and R^{1b} independently represent a hydrogen atom or C₁₋₆ alkyl.

Specifically, R^{1a} and R^{1b} may each be a hydrogen atom, methyl, ethyl, or the like, for example. A hydrogen atom and methyl are preferably recommended.

R^{2a} and R^{2b} independently represent a hydrogen atom or C₁₋₆ alkyl.

Specifically, R^{2a} and R^{2b} may each be a hydrogen atom, methyl, ethyl, or the like, for example. A hydrogen atom is preferably recommended.

R^{3a} and R^{3b} independently represent a hydrogen atom or C₁₋₆ alkyl.

Specifically, R^{3a} and R^{3b} may each be a hydrogen atom, methyl, ethyl, or the like, for example. A hydrogen atom is preferably recommended.

R⁴ represents a hydrogen atom, hydroxy, C₁₋₆ alkyl, C₃₋₆ cycloalkyl, or C₁₋₆ alkoxy, wherein the alkyl, cycloalkyl, or alkoxy is optionally substituted with one to three substituents independently selected from halogen, hydroxy, and C₁₋₆ alkoxy.

R⁴ may be, for example, a hydrogen atom; hydroxy; C₁₋₆ alkyl such as methyl, ethyl, n-propyl, isopropyl, n-butyl, isobutyl, chloromethyl, fluoromethyl, difluoromethyl, trifluoromethyl, chloroethyl, fluoroethyl, difluoroethyl, 2-hydroxyethyl, 2-hydroxy-2-methylpropyl, 2-methoxyethyl, or like; C₃₋₆ cycloalkyl such as cyclopropyl, cyclobutyl, cyclopentyl, cyclohexyl, chlorocyclopropyl, fluorocyclopropyl, hydroxycyclopropyl, or like; or C₁₋₆ alkoxy such as methoxy, ethoxy, n-propyloxy, isopropyloxy, difluoromethoxy, 2-hydroxy-2-methylpropyloxy, 2-methoxyethoxy, or like. Preferably recommended are a hydrogen atom, methyl, 2-hydroxy-2-methylpropyl, cyclopropyl, 2-hydroxy-2-methylpropyloxy, and the like.

R⁵ represents a hydrogen atom, C₁₋₆ alkyl, or C₃₋₆ cycloalkyl, wherein the alkyl or cycloalkyl is optionally substituted with one to three substituents independently selected from halogen, hydroxy, and C₁₋₆ alkoxy.

R⁵ may specifically be, for example, a hydrogen atom; C₁₋₆ alkyl such as methyl, ethyl, n-propyl, isopropyl, n-butyl, isobutyl, chloromethyl, fluoromethyl, difluoromethyl, trifluoromethyl, chloroethyl, fluoroethyl, difluoroethyl, hydroxymethyl, methoxymethyl, or like; or C₃₋₆ cycloalkyl such as cyclopropyl, cyclobutyl, cyclopentyl, cyclohexyl, chlorocyclopropyl, fluorocyclopropyl, or like. Preferably recommended are a hydrogen atom, methyl, ethyl, cyclopropyl, fluoromethyl, difluoromethyl, trifluoromethyl, and the like.

Z represents C₁₋₆ alkyl, C₃₋₆ cycloalkyl, C₁₋₆ alkoxy, aryl, heteroaryl, or N(R^{6a})(R^{6b}), wherein the alkyl, cycloalkyl, alkoxy, aryl, or heteroaryl is optionally substituted with one to three substituents independently selected from halogen, hydroxy, C₁₋₆ alkyl, halo C₁₋₆ alkyl, C₁₋₆ alkoxy, and halo C₁₋₆ alkoxy, or
R⁴ and Z may together form, together with the nitrogen atom to which R⁴ is bonded, a 4- to 6-membered nitrogen-containing hetero ring. The nitrogen-containing hetero ring optionally contains a double bond in the ring and optionally further contains a heteroatom selected from the group consisting of nitrogen, oxygen, and sulfur. The nitrogen-containing hetero ring is optionally fused with an aryl ring or a heteroaryl ring, and the nitrogen-containing hetero ring is optionally substituted with halogen, hydroxy, C₁₋₆ alkyl, halo C₁₋₆ alkyl, C₁₋₆ alkoxy, halo C₁₋₆ alkoxy, or oxo.
R^{6a} and R^{6b} independently represent a hydrogen atom or C₁₋₆ alkyl, or R^{6a} and R^{6b} together form, together with the nitrogen atom to which they are bonded, a 5- to 6-membered nitrogen-containing hetero ring. The nitrogen-containing hetero ring optionally further contains a heteroatom selected from the group consisting of nitrogen, oxygen, and sulfur, or is optionally substituted with halogen, hydroxy, C₁₋₆ alkyl, halo C₁₋₆ alkyl, C₁₋₆ alkoxy, halo C₁₋₆ alkoxy, or oxo.

Specifically, R^{6a} and R^{6b} may independently be a hydrogen atom, methyl, ethyl, n-propyl, isopropyl, or the like, for example. The nitrogen-containing hetero ring formed by R^{6a} and R^{6b} together with the nitrogen atom to which they are bonded may be pyrrolidine, piperidine, morpholine, thiomorpholine, piperazine, 3-hydroxypyrrolidine, 3-methoxypyrrolidine, N-methylpiperazine, pyrrolidin-2-one, or the like, for example.

Z may specifically be, for example, C₁₋₆ alkyl such as methyl, ethyl, n-propyl, isopropyl, n-butyl, isobutyl, tert-butyl, 2,2-dimethylpropyl, difluoromethyl, trifluoromethyl, hydroxymethyl, 1-hydroxy-1-methylethyl, 1-hydroxy-2,2-dimethylpropyl, 2-hydroxy-2-methylpropyl, methoxymethyl, or like; C₃₋₆ cycloalkyl such as cyclopropyl, cyclobutyl, 1-hydroxycyclopropyl, 1-methylcyclopropyl, 2-methyl-2-hydroxycyclopropyl, 1-hydroxy-2,2-dimethylcyclopropyl, or like; C₁₋₆ alkoxy such as methoxy, ethoxy, n-propyloxy, isopropyloxy, tert-butoxy, or like; aryl such as phenyl, 2-trifluoromethylphenyl, 2-trifluoromethoxypheny, naphthyl, or like; heteroaryl such as pyridyl, oxazolyl, pyrrolyl, furanyl, isoxazolyl, fluoropyridyl, trifluoromethylpyridyl, difluoromethoxypyridyl, trifluoromethoxypyridyl, or like; or N(R^{6a})(R^{6b}) such as amino, methylamino, dimethylamino, ethylamino, diethylamino, ethylmethylamino, or like. Preferably recommended are methyl, ethyl, isopropyl, difluoromethyl, 1-hydroxy-1-methylethyl, 1-hydroxycyclopropyl, phenyl, isoxazolyl, and the like.

Specific examples of nitrogen-containing hetero rings formed by R⁴ and Z together with the nitrogen atom to which R⁴ is bonded are as follows:

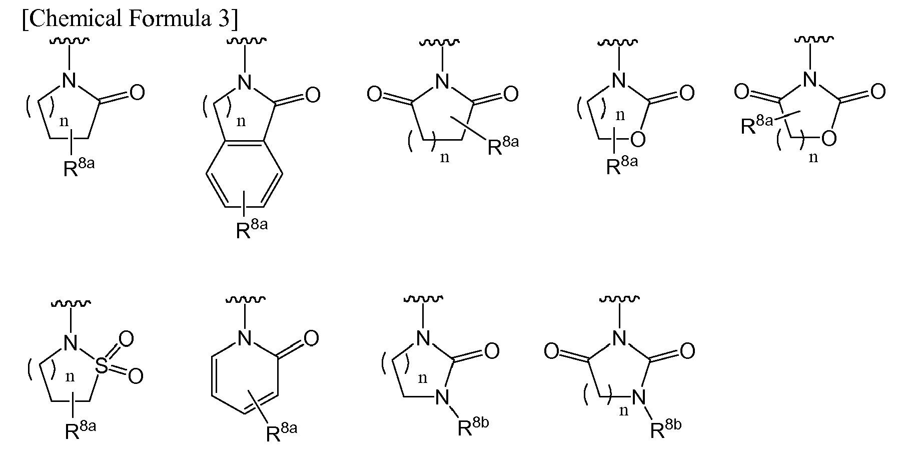

wherein R^{8a} represents a hydrogen atom, halogen, hydroxy, C₁₋₆ alkyl, halo C₁₋₆ alkyl, C₁₋₆ alkoxy, or halo C₁₋₆ alkoxy, R^{8b} represents a hydrogen atom, C₁₋₆ alkyl, or halo C₁₋₆ alkyl, and n is 0 to 2.

R^{8a} may specifically be, for example, a hydrogen atom; halogen such as a fluorine atom, a chlorine atom, a bromine atom, or like; C₁₋₆ alkyl such as hydroxy; methyl, ethyl, n-propyl, isopropyl, n-butyl, isobutyl, or like; C₁₋₆ alkyl such as fluoromethyl, chloromethyl, difluoromethyl, trifluoromethyl, fluoroethyl, chloroethyl, difluoroethyl, or like halo; C₁₋₆ alkoxy such as methoxy, ethoxy, n-propyloxy, isopropyloxy, n-butoxy, or like; or halo C₁₋₆ alkoxy such as chloromethoxy, fluoromethoxy, dichloromethoxy, difluoromethoxy, trichloromethoxy, trifluoromethoxy, chloroethoxy, fluoroethoxy, or like.

R^{8b} may specifically be, for example, a hydrogen atom; C₁₋₆ alkyl such as methyl, ethyl, n-propyl, isopropyl, n-butyl, isobutyl, or like; or halo C₁₋₆ alkyl such as fluoromethyl, chloromethyl, difluoromethyl, trifluoromethyl, fluoroethyl, chloroethyl, difluoroethyl, or like.

Preferred examples of nitrogen-containing hetero rings formed by R⁴ and Z together with the nitrogen atom to which R⁴ is bonded are:

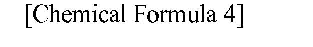

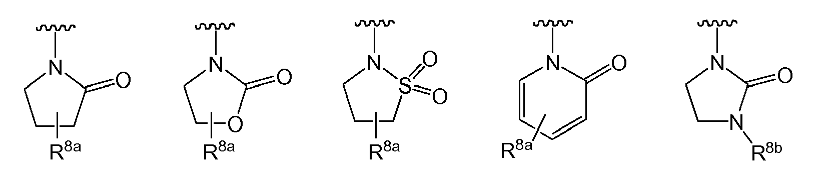

wherein R^{8a} and R^{8b} are as defined above. Among the above, a hydrogen atom, a fluorine atom, hydroxy, methoxy, and the like are particularly recommended for R^{8a}, while a hydrogen atom, methyl, and the like are particularly recommended for R^{8b}.

Y₁ represents H or -OR^{7a}, and Y₂ represents H, or Y₁ and Y₂ together form -O-C(R^{7b})(R^{7c})-.

R^{7a}, R^{7b}, and R^{7c} each independently represent a hydrogen atom or C₁₋₆ alkyl.

Specifically, R^{7a}, R^{7b}, and R^{7c} may each be a hydrogen atom; methyl, ethyl, n-propyl, isopropyl, n-butyl, isobutyl, or the like, for example. A hydrogen atom is preferably recommended.

Y₁ and Y₂ may specifically be:
Y₁ = H, Y₂ = H,
Y₁ = OR^{7a}, Y₂ = H, or
Y₁ = OCH₃, Y₂ = H, or, where Y₁ and Y₂ are taken together,
   - -O-C(R^{7b})(R^{7c})-,
   - -O-CH₂-,
   - -O-CH(CH₃)-, or-
   - -O-C(CH₃)₂-. Preferably recommended are Y₁ = H and Y₂ = H and, where Y₁ and Y₂ are taken together, -O-CH₂-.

W represents C, S, or SO, and C and SO are preferably recommended.

Ar₁ represents 6-membered aryl or 6-membered nitrogen-containing heteroaryl. The aryl or nitrogen-containing heteroaryl is optionally substituted with a substituent selected from the group consisting of group α.

Preferred examples of substituents in Ar₁ selected from the group consisting of group α include a fluorine atom, a chlorine atom, a bromine atom, or like halogen; methyl, ethyl, n-propyl, isopropyl, or like C₁₋₆ alkyl; etc. The number of substituents contained may be one to four, and preferably one to three.

Ar₁ may specifically be, for example, phenyl, 4-fluorophenyl, 3,4-difluorophenyl, 2,4,5-trifluorophenyl, 3,4,5-trifluorophenyl, 4-chloro-3,5-difluorophenyl, or the like as 6-membered aryl; or pyridyl, 5-fluoropyridin-2-yl, 5-chloropyridin-2-yl, 6-chloropyridin-3-yl, or the like as 6-membered nitrogen-containing heteroaryl. Preferred examples are 6-membered aryl (especially phenyl) and 6-membered nitrogen-containing heteroaryl (especially pyridyl) substituted with one to three fluorine atoms or chlorine atoms, and especially recommended are 3,4-difluorophenyl, 2,4,5-trifluorophenyl, 3,4,5-trifluorophenyl, 4-chloro-3,5-difluorophenyl, and 5-chloropyridin-2-yl.

Ar₂ is a group formed by removing two hydrogen atoms from 6-membered aryl or 5- to 6-membered heteroaryl, and the aryl or heteroaryl is optionally substituted with one to three substituents independently selected from the group consisting of group α.

The 6-membered aryl in Ar₂ may be a benzene ring, for example. The 5- to 6-membered heteroaryl may be a pyridine ring, a pyrazine ring, a pyrimidine ring, or a pyridazine ring, for example. In particular, a benzene ring, a pyridine ring, and a pyrimidine ring are recommended.

Preferred examples of substituents in Ar₂ selected from the group consisting of group α include a fluorine atom, a chlorine atom, methyl, ethyl, n-propyl, isopropyl, chloromethyl, fluoromethyl, methoxy, ethoxy, methylcarbonyl, methanesulfonyl, and the like.

The substituents in Ar₁ selected from the group consisting of group α and the substituents in Ar₂ selected from the group consisting of group α may be the same or different.

With respect to Ar₂, preferred 6-membered aryl is phenylenediyl, and 1,4-phenylenediyl is especially recommended; and preferred 6-membered nitrogen-containing heteroaryl is pyridinediyl or pyrimidinediyl, and especially pyridine-2,5-diyl and pyrimidine-2,5-diyl are recommended.

A formula:

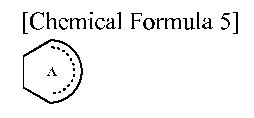

(hereinafter referred to as "ring A") represents a 6-membered aryl ring or a 5- to 6-membered nitrogen-containing hetero ring. The aryl ring or nitrogen-containing hetero ring is optionally further fused with a 5- to 6-membered aryl ring or heteroaryl ring, and the aryl ring or nitrogen-containing hetero ring is optionally substituted with one to three substituents independently selected from halogen, cyano, hydroxy, C₁₋₆ alkyl, halo C₁₋₆ alkyl, C₁₋₆ alkoxy, halo C₁₋₆ alkoxy, C₁₋₆ alkylcarbonylamino, and oxo.

Specific examples of rings A are as follows:

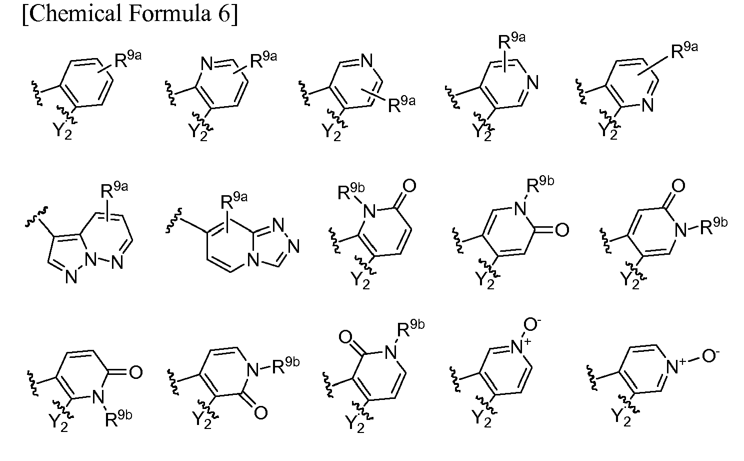

wherein R^{9a} represents a hydrogen atom, halogen, cyano, hydroxy, C₁₋₆ alkyl, halo C₁₋₆ alkyl, C₁₋₆ alkoxy, halo C₁₋₆ alkoxy, or C₁₋₆ alkylcarbonylamino, R^{9b} represents a hydrogen atom, C₁₋₆ alkyl, or halo C₁₋₆ alkyl, and Y₂ is as defined above.

R^{9a} may specifically be, for example, a hydrogen atom; halogen such as a fluorine atom, a chlorine atom, a bromine atom, or like; C₁₋₆ alkyl such as cyano; hydroxy; methyl, ethyl, n-propyl, isopropyl, n-butyl, isobutyl, or like; halo C₁₋₆ alkyl such as fluoromethyl, chloromethyl, difluoromethyl, trifluoromethyl, fluoroethyl, chloroethyl, difluoroethyl, or like; C₁₋₆ alkoxy such as methoxy, ethoxy, n-propyloxy, isopropyloxy, n-butoxy, or like; halo C₁₋₆ alkoxy such as chloromethoxy, fluoromethoxy, dichloromethoxy, difluoromethoxy, trichloromethoxy, trifluoromethoxy, chloroethoxy, fluoroethoxy, or like; or C₁₋₆ alkylcarbonylamino such as methylcarbonylamino, ethylcarbonylamino, or like.

R^{9b} may specifically be, for example, a hydrogen atom; C₁₋₆ alkyl such as methyl, ethyl, n-propyl, isopropyl, n-butyl, isobutyl, or like; or halo C₁₋₆ alkyl such as fluoromethyl, chloromethyl, difluoromethyl, trifluoromethyl, fluoroethyl, chloroethyl, difluoroethyl, or like.

Among the above, the ring A is preferably a 5- to 6-membered nitrogen-containing hetero ring, wherein the hetero ring being optionally fused with a 5- to 6-membered aryl ring or heteroaryl ring, and wherein the nitrogen-containing hetero ring being optionally substituted with halogen, cyano, hydroxy, C₁₋₆ alkyl, halo C₁₋₆ alkyl, C₁₋₆ alkoxy, halo C₁₋₆ alkoxy, or C₁₋₆ alkylcarbonylamino. Particularly recommended are:

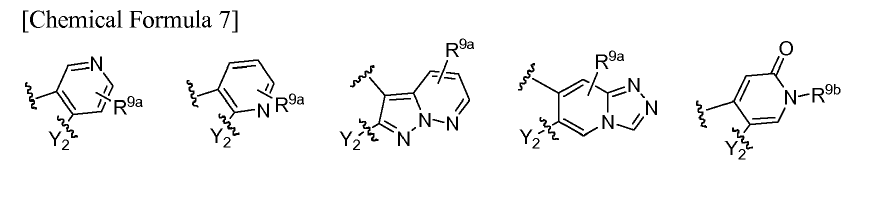

wherein the symbols are as defined above, etc.

Among the above, in particular, a hydrogen atom, a fluorine atom, and the like are recommended for R^{9a}, while a hydrogen atom, methyl, and the like are recommended for R^{9b}.

Preferred examples of compounds represented by formula (I) include:
N-{(3,4-difluorophenyl)[4-(1H,1'H-spiro[furo[3,4-c]pyridine-3,4'-piperidin]-1'-ylmethyl)phenyl]methyl}acetamide,
N-{(3,4-difluorophenyl)[5-(1H,1'H-spiro[furo[3,4-c]pyridine-3,4'-piperidin]-1'-ylmethyl)pyridin-2-yl]methyl}-N-(2-hydroxy-2-methylpropyloxy)acetamide,
N-{(3,4-difluorophenyl)[5-(1H,1'H-spiro[furo[3,4-c]pyridine-3,4'-piperidin]-1'-ylmethyl)pyridin-2-yl]methyl}-N-(2-hydroxy-2-methylpropyl)-2-methylpropanamide,
N-{(3,4-difluorophenyl)[4-(1H,1'H-spiro[furo[3,4-c]pyridine-3,4'-piperidin]-1'-ylmethyl)phenyl]methyl}benzamide,
N-{(3,4-difluorophenyl)[4-(1H,1'H-spiro[furo[3,4-c]pyridine-3,4'-piperidin]-1'-ylmethyl)phenyl]methyl}isoxazol-5-carboxamide,
3-{(3,4-difluorophenyl)[4-(1H,1'H-spiro[furo[3,4-c]pyridine-3,4'-piperidin]-1'-ylmethyl)phenyl]methyl}-1,3-oxazolidin-2-one,
1'-{4-[(3,4-difluorophenyl)(1,1-dioxideisothiazolidin-2-yl)methyl]benzyl}-5-methyl-3,5-dihydro-6H-spiro[furo[3,4-c]pyridine-1,4'-piperidin]-6-one,
1-{(3,4-difluorophenyl)[4-(1H,1'H-spiro[furo[3,4-c]pyridine-3,4'-piperidin]-1'-ylmethyl)phenyl]methyl}-3-methylimidazolidin-2-one,
(R)- or (S)-N-{(5-chloropyridin-2-yl)4-(1H,1'H-spiro[furo[3,4-c]pyridine-3,4'-piperidin]-1'-ylmethyl)phenyl]methyl}propanamide,
(R)- or (S)-N-[(3,4-difluorophenyl)(5-{[4-(6-fluoropyridin-3-yl)piperidin-1-yl]methyl}pyridin-2-yl)methyl]-2-hydroxy-N,2-dimethylpropanamide,
(R)- or (S)-N-cyclopropyl-N-{(3,4-difluorophenyl)[5-(1H,1'H-spiro[furo[3,4-c]pyridine-3,4'-piperidin]-1'-ylmethyl)pyridin-2-yl]methyl}--2-hydroxy-2-methylpropanamide, (R)- or (S)-N-{(3,4-difluorophenyl)[5-(1H,1'H-spiro[furo[3,4-c]pyridine-3,4'-piperidin]-1'-ylmethyl)pyrimidin-2-yl]methyl}-2-hydroxy-N,2-dimethylpropanamide,
(R)- or (S)-N-((3,4-difluorophenyl){4-[(4-pyrazolo[1,5-b]pyridazin-3-ylpiperidin-1-yl)methyl]phenyl}methyl)-1-hydroxycyclopropanecarboxamide,
(R)- or (S)-N-{cyclopropyl(3,4-difluorophenyl)[5-(1H,1'H-spiro[furo[3,4-c]pyridine-3,4'-piperidin]-1'-ylmethyl)pyridin-2-yl]methyl}acetamide,
(R)- or (S)-N-[1-(3,4-difluorophenyl)-1-(5-{[4-(6-fluoropyridin-3-yl)piperidin-1-yl]methyl}pyridin-2-yl)ethyl]-2,2-difluoroacetamide,
1-{(3,4-difluorophenyl)[4-(1H,1'H-spiro[furo[3,4-c]pyridine-3,4'-piperidin]-1'-ylmethyl)phenyl]methyl}pyrrolidin-2-one,
1-[[4-(1H,1'H-spiro[furo[3,4-c]pyridine-3,4'-piperidin]-1'-ylmethyl)phenyl](2,4,5-trifluorophenyl)methyl]pyrrolidin-2-one,
(3R)- or (3S)-[(R)- or (S)-1-{(3,4-difluorophenyl)[4-(1H,1'H-spiro[furo[3,4-c]pyridine-3,4'-piperidin]-1'-ylmethyl)phenyl]methyl}]-3-fluoropyrrolidin-2-one,
1-{(3,4-difluorophenyl)[4-(1H,1'H-spiro[furo[3,4-c]pyridine-3,4'-piperidin]-1'-ylmethyl)phenyl]methyl}pyridin-2(1H)-one,
1-((3,4-difluorophenyl){4-[(6-fluoro-1H,1'H-spiro[furo[3,4-c]pyridine-3,4'-piperidin]-1'-yl)methyl]phenyl}methyl)pyrrolidin-2-one,
1-((3,4-difluorophenyl){4-[(4-[1,2,4]triazolo[4,3-a]pyridin-7-ylpiperidin-1-yl)methyl]phenyl}methyl)pyrrolidin-2-one,
(R)- or (S)-N-{1-(4-chloro-3,5-difluorophenyl)-1-[5-(1H,1'H-spiro[furo[3,4-c]pyridine-3,4'-piperidin]-1'-ylmethyl)pyridin-2-yl]ethyl} acetamide,
(R)- or (S)-N-{1-(3,4-difluorophenyl)-2,2-difluoro-1-[5-(1H,1'H-spiro[furo[3,4-c]pyridine-3,4'-piperidin]-1'-ylmethyl)pyridin-2-yl]ethyl} acetamide, and
(R)- or (S)-N-{1-(3,4-difluorophenyl)-2,2,2-trifluoro-1-[5-(1H,1'H-spiro[furo[3,4-c]pyridine-3,4'-piperidin]-1'-ylmethyl)pyridin-2-yl]ethyl acetamide, and the like.

The compound of the invention may contain one or more chiral centers, and thus can exist as an optically active substance or a as racemate. Such a chiral center allows two or more independent optical isomers to exist, and all the possible optical isomers shall be encompassed, singly or as a mixture, by the scope of the invention.

### Method for preparing a compound represented by formula (I)

The compound represented by formula (I) can be prepared by the following methods, but the production method is not limited thereto.

### Production method 1

Production method 1 is a method for preparing the compound represented by formula (I).

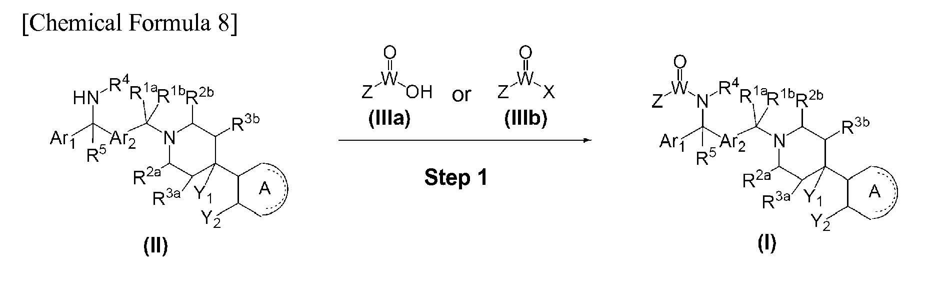

In the formulae, X represents halogen or the like, and other symbols are as defined above.

### Step 1

A compound represented by formula (II) is reacted with a compound represented by formula (IIIa) or formula (IIIb) in a reaction solvent to give the compound represented by formula (I). The amidation reaction can be performed in accordance with a conventionally known amidation method used in peptide synthesis, for example, the method described in *Peptide-Gosei no Kiso to Jikken* (Basics and Experiments of Peptide Synthesis), Nobuo Izumiya et al., Maruzen, 1983.

Examples of compounds represented by formula (IIIa) include carboxylic acids. Examples of compounds represented by formula (IIIb) include the carboxylic acids, sulfinic acids, and sulfonic acid reaction equivalents.

Acid halides, acid anhydrides, mixed acid anhydrides, activated esters, activated amides, and the like are used as carboxylic acids and sulfinic acids or sulfonic acid reaction equivalents represented by formula (IIIb). As such reaction equivalents, commercial products are usable. In addition, they can also be readily prepared with reference to a conventionally known method, for example, the above-mentioned *Peptide-Gosei no Kiso to Jikken* (Basics and Experiments of Peptide Synthesis), Nobuo Izumiya et al., Maruzen, 1983.

The amount of compound represented by formula (IIIa) or formula (IIIb) used is 1.0 mol to molar excess, for example, and preferably 1.0 mol to 1.5 mol per mol of the compound represented by formula (II).

When the compound represented by formula (IIIa) is used, the amidation reaction is preferably carried out in the presence of a condensing agent. For example, the reaction may be carried out in the presence or absence of, preferably in the presence of, 1-hydroxybenzotriazole (hereinafter referred to as "HOBT") or a like N-hydroxy compound, using N,N'-dicyclohexylcarbodiimide, 1-ethyl-3-(3-dimethylaminopropyl)carbodiimide hydrochloride (hereinafter referred to as "WSC•HCl"), or a like condensing agent.

The amount of condensing agent used is usually 1.0 mol to molar excess, for example, and preferably 1.0 mol to 1.5 mol per mol of the compound represented by formula (II).

When an N-hydroxy compound is used, the amount thereof is 1.0 mol to molar excess, for example, and preferably 1.0 mol to 1.5 mol per mol of the compound represented by formula (II).

Although amidation reaction proceeds in the absence of a base, for smooth proceeding of the reaction, the reaction is preferably performed in the presence of a base. Examples of usable bases are organic bases such as triethylamine, N,N-diisopropylethylamine, pyridine, lithium bis(trimethylsilyl)amide, and like; inorganic bases such as sodium hydroxide, potassium hydroxide, sodium carbonate, potassium carbonate, sodium hydrogen carbonate, and like.

The amount of base used is usually 1.0 mol to molar excess, for example, and preferably 1.0 mol to 4.0 mol per mol of the compound represented by formula (II). When the base is liquid, the base may be used as both solvent and base.

In the reaction using a reaction equivalent as above, dimethylaminopyridine or a like basic catalyst may be used as a catalyst for accelerating the reaction. The amount of catalyst used is 0.1 mol to 5.0 mol, for example, and preferably 0.1 mol to 0.5 mol per mol of the compound represented by formula (II).

Examples of reaction solvents include halogenated hydrocarbons such as methylene chloride, chloroform, and like; ethers such as diethylether, tetrahydrofuran (hereinafter referred to as "THF"), 1,4-dioxane (hereinafter referred to as "dioxane"), and like; acetonitrile, dimethylformamide (hereinafter referred to as "DMF"), dimethyl sulfoxide (hereinafter referred to as "DMSO"), pyridine, and the like; mixed solvents thereof; etc.

The reaction temperature is usually -50°C to 100°C, for example, and is preferably 0°C to 50°C.

The reaction time is usually 5 minutes to 7 days, for example, and is preferably 30 minutes to 24 hours.

Examples of compounds represented by formula (IIIa) include isobutyric acid, 1-methylcyclopropanecarboxylic acid, 1-hydroxycyclopropanecarboxylic acid, cyclobutanecarboxylic acid, 3-hydroxy-3-methylbutanoic acid, (2S)-2-hydroxy-3,3-dimethylbutanoic acid, and the like.

Examples of compounds represented by formula (IIIb) include acetyl chloride, 2,2-dimethylpropanoyl chloride, 2-chloro-1,1-dimethyl-2-oxoethyl acetate, cyclopropanecarbonyl chloride, benzoyl chloride, pyridine-2-carbonyl chloride, 2-furoyl chloride, isoxazole-5-carbonyl chloride, succinyl dichloride, 4-chlorobutanoyl chloride, methanesulfonyl chloride acid, cyclopropanesulfonyl chloride, 3-chloropropanesulfonyl chloride, and like acid halides; acetic anhydride, propionic anhydride, trimethylacetic anhydride, difluoroacetic anhydride, and like acid anhydrides; methyl chloroformate and like activated esters; dimethycarbamoyl chloride, 1-pyrrolidinecarbonyl chloride, 4-morpholinecarbonyl chloride, and like activated amides; etc.

The compound represented by formula (II) can be prepared by the below-mentioned method.

### Production method 2-1

Production method 2-1 is a method for preparing a compound represented by formula (I') having formula (I) wherein R⁵ is a hydrogen atom.

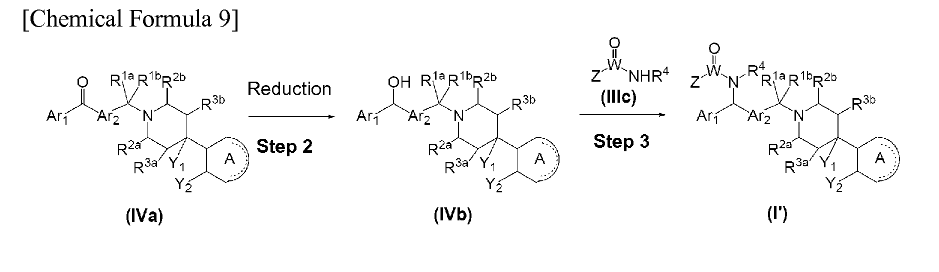

In the formulae, the symbols are as defined above.

### Step 2

A compound represented by formula (IVb) is obtained from a compound represented by formula (IVa) in accordance with a known method, for example, by reduction reaction in an alcohol solvent using sodium borohydride.

### Step 3

] The compound represented by formula (IVb) is reacted with a compound represented by formula (IIIc) in an acid solvent to give a compound represented by formula (I').

The amount of the compound represented by formula (IIIc) used is 1.0 mol to molar excess, for example, and preferably 3.0 mol to 5.0 mol per mol of the compound represented by formula (IVb).

Examples of acids include trifluoroacetate (hereinafter sometimes referred to as "TFA") and a mixture of concentrated sulfuric acid and acetic acid.

The reaction temperature is 20°C to 200°C, for example, and is preferably 100°C to 180°C. The reaction is usually completed within 10 minutes to 12 hours.

For accelerating the reaction, the reaction may be effected using a microwave.

Examples of the compounds represented by formula (IIIc) include:

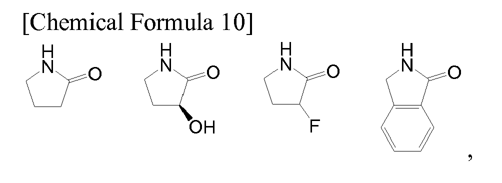

etc.

The compound represented by (IVa) can be prepared in accordance with the methods described in WO2008/038692 and PCT/JP08/067406.

### Production method 2-2

Production method 2-2 is another method for preparing a compound represented by formula (I').

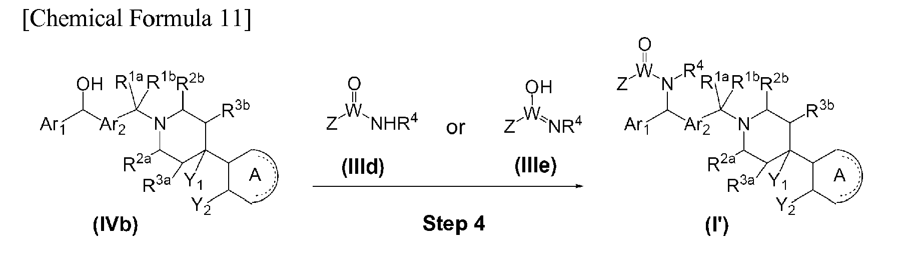

In the formulae, the symbols are as defined above.

### Step 4

The compound represented by formula (IVb) is reacted with a compound represented by formula (IIId) or a compound represented by formula (IIIe) under Mitsunobu reaction conditions to give a compound represented by formula (I').

Specifically, in a reaction solvent, in the presence of an azo compound, such as dialkyl azodicarboxylate or azodicarboxamide, and an organic phosphorous compound, such as triarylphosphine or trialkylphosphine, a compound represented by formula (IIId) or the compound represented by formula (IIIe) is reacted with a compound represented by formula (IVb) to give the compound represented by formula (I').

Examples of azo compounds include dimethyl azodicarboxylate, diethyl azodicarboxylate, diisopropyl azodicarboxylate, 1,1'-(azodicarbonyl)dipiperidide, N,N,N',N'-tetramethylazodicarboxamide, and the like. Examples of triarylphosphines include triphenylphosphine, tritolylphosphine, and the like, and examples of trialkylphosphines include triethylphosphine, tri-n-butylphosphine, and the like. In particular, a combination of N,N,N',N'-tetramethylazodicarboxamide and tri-n-butylphosphine is recommended.

With respect to the amounts of azo compound and organic phosphorous compound used, the amount of azo compound is 1.0 mol to 3.0 mol, for example, and preferably 1.0 mol to 2.0 mol per mol of the compound represented by formula (IVb), while the amount of organic phosphorous compound is 1.0 mol to 3.0 mol, for example, and preferably 1.0 mol to 2.0 mol per mol of the compound represented by formula (IVb).

The amount of the compound represented by formula (IIId) or the compound represented by formula (IIIe) used is 1.0 mol to 10 mol, for example, and preferably 1.0 mol to 3.0 mol per mol of the compound represented by formula (IVb).

Examples of reaction solvents include halogenated carbons such as methylene chloride, dichloroethane, and like; aliphatic hydrocarbons such as n-heptane (hereinafter referred to as "heptane"), n-hexane (hereinafter referred to as "hexane"), and like; aromatic hydrocarbons such as benzene, toluene, xylene, and like; ethers such as diethylether, THF, dioxane, and like; acetonitrile; mixed solvents thereof; etc.

The reaction temperature is 0°C to 100°C, for example, and is preferably 0°C to 50°C. The reaction is usually completed within 1 to 24 hours.

Examples of the compounds represented by formula (IIId) and the compounds represented by formula (IIIe) are as follows:

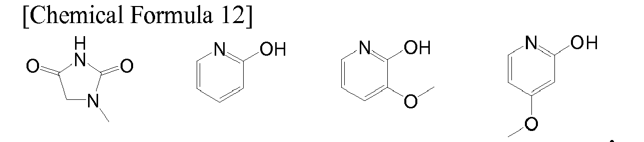

### Production method 3

Production method 3 is a method for preparing the compound represented by formula (I') from the compound represented by formula (Va).

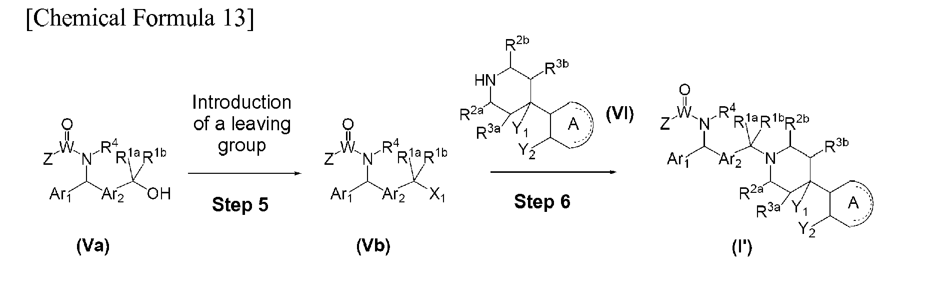

In the formulae, X₁ represents mesyl, tosyl, or a like leaving group, and other symbols are as defined above.

### Step 5

A leaving group is introduced into a compound represented by formula (Va) by mesylation, tosylation, or the like, to give the compound represented by formula (Vb). For reaction conditions, the methods described in WO2008/038692 and PCT/JP08/067406 can be referred to.

### Step 6

The compound represented by formula (Vb) is reacted with a compound represented by formula (VI) in a reaction solvent and preferably in the presence of a base to give a compound represented by formula (I').

The amount of compound represented by formula (VI) used is 1.0 mol to 2.0 mol, for example, and preferably 1.0 to 1.5 mol per mol of the compound represented by formula (Vb).

Examples of bases include inorganic bases such as sodium carbonate, sodium hydrogen carbonate, potassium carbonate, potassium hydrogen carbonate, lithium carbonate, and like; organic amines such as trimethylamine, triethylamine, N,N-diisopropylethylamine, pyridine, and like; etc. The amount of base used is 1.0 mol to 5.0 mol, for example, and preferably 1.1 mol to 2.0 mol per mol of the compound represented by formula (Vb).

Examples of reaction solvents include halogenated hydrocarbons such as methylene chloride, chloroform, carbon tetrachloride, and like; ethers such as diethylether, THF, dioxane, and like; DMF, DMSO, and the like; mixed solvents thereof; etc.

The reaction temperature is 0°C to 100°C, for example, and is preferably 10°C to 30°C. The reaction is usually completed within 1 hour to 24 hours.

Examples of compounds represented by formula (VI) include 6-fluoro-1H-spiro[furo[3,4-c]pyridine-3,4'-piperidine] hydrochloride, 7-piperidin-4-yl[1,2,4]triazolo[4,3-a]pyridine hydrochloride, 3-piperidin-4-ylpyrazolo[1,5-b]pyridazine hydrochloride, and the like. These compounds can be prepared in accordance with the methods described in WO2008/038692 and PCT/JP08/067406 or a similar method.

The compound represented by formula (Va) can be prepared by the below-mentioned method.

### Production method 4-1

Production method 4-1 is a method for preparing a compound represented by formula (IIa) having formula (II) wherein R⁵ is a hydrogen atom.

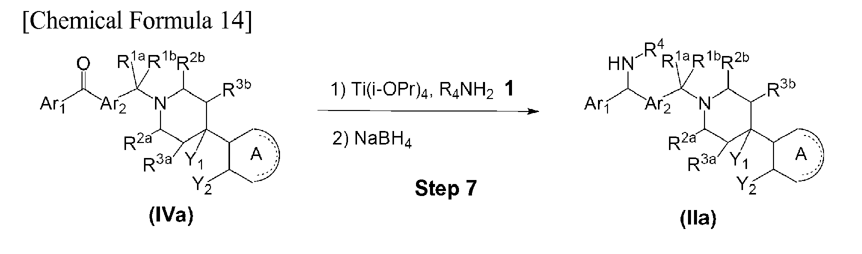

In the formulae, the symbols are as defined above.

### Step 7

1) The compound represented by formula (IVa) is reacted with a compound 1 in a reaction solvent in the presence of titanium tetraisopropoxide.

The amount of titanium tetraisopropoxide used is 1.0 mol to molar excess, for example, and preferably 1.3 mol to 2.2 mol per mol of the compound represented by formula (IVa).

The amount of compound 1 used is 1.0 mol to molar excess, for example, and preferably 2.0 mol to 5.0 mol per mol of the compound represented by formula (IVa).

Examples of reaction solvents include alcohols such as methanol, ethanol, propanol, and like; ethers such as diethylether, THF, dioxane, and like; halogenated hydrocarbons such as methylene chloride, chloroform, dichloroethane, and like; aromatic hydrocarbons such as benzene, toluene, xylene, and like; DMF, acetonitrile, and the like; mixed solvents thereof; etc.

The reaction temperature is usually -20°C to 100°C, for example, and is preferably 0°C to room temperature. The reaction is usually completed within 30 minutes to 24 hours, and preferably 1 hour to 6 hours.

2) Sodium borohydride is added to the reaction solution obtained in 1) to effect reduction reaction, to give the compound represented by formula (IIa).

The amount of sodium borohydride used is 1.0 mol to molar excess, for example, and preferably 1.2 mol to 2.4 mol per mol of the compound represented by formula (IVa).

The reaction temperature is usually -20°C to 100°C, for example, and is preferably 0°C to room temperature. The reaction is usually completed within 30 minutes to 24 hours, and preferably 1 hour to 6 hours.

Examples of compounds represented by compound 1 include ammonia, methylamine, ethylamine, cyclopropylamine, 1-amino-2-methylpropan-2-ol, and the like.

### Production method 4-2

Production method 4-2 is a method for preparing the compound represented by formula (IIa) from the compound represented by formula (IVb).

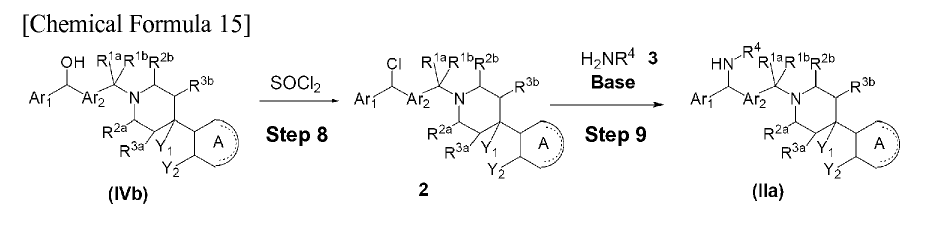

In the formulae, the symbols are as defined above.

### Step 8

The compound represented by formula (IVb) is chlorinated at 0°C in accordance with a conventionally known method using an excessive amount of thionyl chloride to give a compound 2.

### Step 9

The compound 2 is reacted with a compound 3 in a reaction solvent in the presence of a base to give the compound represented by formula (IIa).

The amount of compound 3 used is 1.0 mol to molar excess, for example, and preferably 1.5 mol to 5.0 mol per mol of the compound 2.

Examples of bases include triethylamine, N,N-diisopropylethylamine, pyridine, and the like. The amount of base used is 1.0 mol to molar excess, for example, and preferably 3.0 mol to 10 mol per mol of the compound 2.

Examples of reaction solvents include methylene chloride, THF, acetonitrile, and the like, mixed solvents thereof, etc.

The reaction temperature is 50°C to 150°C, for example, and is preferably 90°C to 100°C. The reaction is usually completed within 1 day to 4 days.

For accelerating the reaction, a molecular sieve 4A or the like may be added.

Examples of compounds represented by compound 3 include O-methylhydroxyamine hydrochloride, (aminooxy)(tert-butyl)dimethylsilane, 1-(aminooxy)-2-methylpropan-2-ol, and the like.

### Production method 5

Production method 5 is a method for preparing the compound represented by formula (IIb) having formula (IIa) wherein R⁴ is a hydrogen atom.

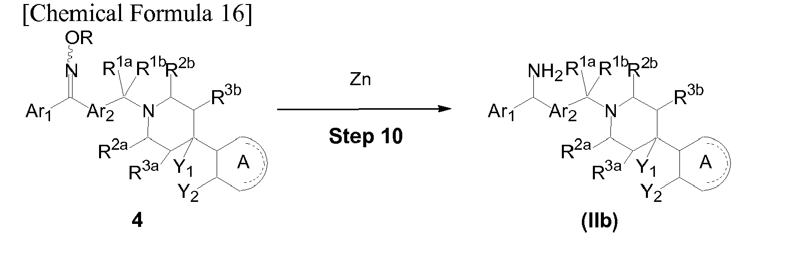

In the formulae, R represents a hydrogen atom, methyl, ethyl, 2-hydroxy-2-methylpropyl, or the like, and other symbols are as defined above.

### Step 10

A compound 4 is reduced in a reaction solvent using zinc to give the compound represented by formula (IIb).

The amount of zinc used is 5.0 mol to molar excess, for example, and preferably 5.0 mol to 6.0 mol per mol of the compound 4.

Examples of reaction solvents include TFA, formic acid, acetic acid, and the like.

The reaction temperature is -10°C to 50°C, for example, and is preferably 0°C to room temperature. The reaction is usually completed within 30 minutes to 2 hours.

The compound 4 can be prepared in accordance with the methods described in WO2008/038692 and PCT/JP08/067406.

This step may also be carried out by, in addition to achieve reduction using zinc, achieve reduction under hydrogen atmosphere using palladium-carbon, platinum oxide, Raney nickel, or the like, or by a known method using tin chloride, lithium hydride aluminum, or a like reducing agent.

### Production method 6

Production method 6 is a method for preparing the compound represented by formula (IIb) from known compounds 5 and 6.

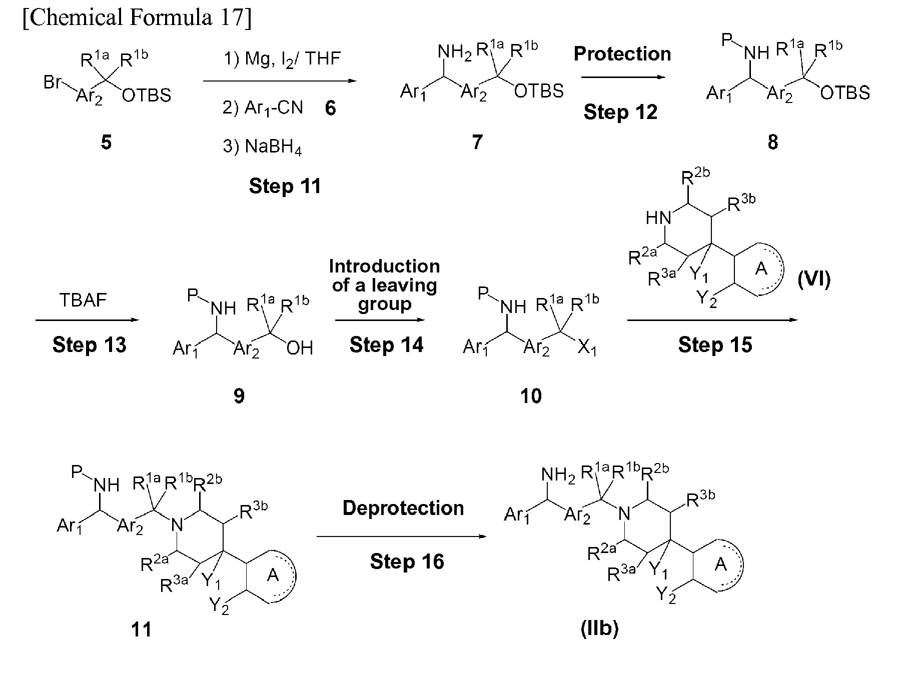

In the formulae, P represents an amino-protecting group, and other symbols are as defined above.

### Step 11

1) The compound 5 is reacted with a piece of magnesium in THF in the presence of iodine to prepare a Grignard reagent.
2) To the reaction solution obtained in 1) is added 1.0 equivalent of compound 6, and the reaction is carried out for 1 hour to 4 hours at 20°C to a temperature of heating under reflux.
3) Sodium borohydride is added to the reaction solution obtained in 2) to effect reduction, to give a compound 7.

These reactions can be performed in accordance with a conventionally known method.

### Step 12

The amino group of the compound 7 is protected to give a compound 8. Examples of protecting groups include tert-butyloxycarbonyl, benzyloxycarbonyl, and the like. For a method for introducing a protecting group, the below-mentioned *Protective Groups in Organic Synthesis* can be referred to.

### Step 13

The TBS (tert-butyldimethylsilyl) group of the compound 8 is removed with tetrabutylammonium fluoride to give a compound 9. For a method for deprotection, *Protective Groups in Organic Synthesis* can be referred to.

### Step 14

A leaving group is introduced into the compound 9 according to Step 5 to give a compound 10.

### Step 15

The compound 10 is reacted with the compound represented by formula (VI) according to Step 6 to give a compound 11.

### Step 16

The amino-protecting group of the compound 11 is removed in accordance with a conventionally known method to give the compound represented by formula (IIb).

Examples of compounds represented by compound 5 include [(4-bromobenzyl)oxy](tert-butyl)dimethylsilane and the like.

Examples of compounds represented by compound 6 include 3,4-difluorobenzonitrile and the like.

In addition to the above methods, the compound represented by formula (II) and the compound represented by formula (Va) can be prepared in accordance with the method shown in the following chart. These methods can be performed under the reaction conditions described in Examples and Production Examples.

### Production method 7

Production method 7 is a method for preparing the compound represented by formula (IIc) having formula (II) wherein R⁴ is a hydrogen atom.

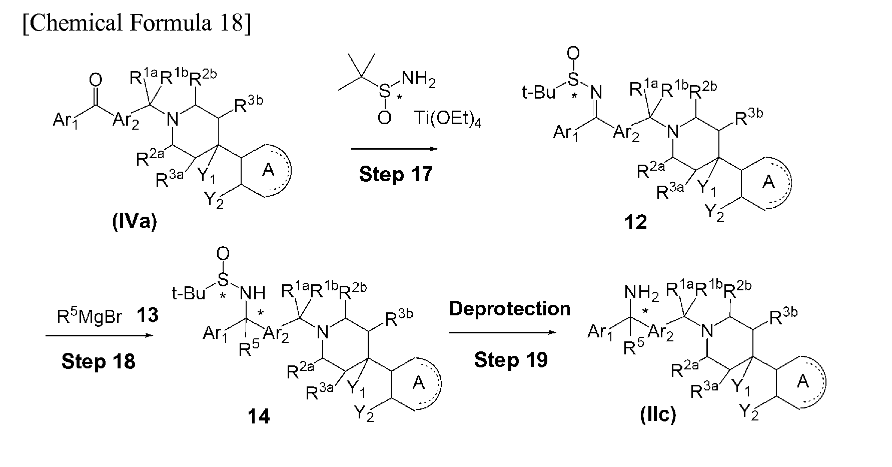

In the formulae, the symbols are as defined above.

### Step 17

The compound represented by formula (IVa) is condensed with tert-butylsulfinylamide in a reaction solvent in the presence of titanium tetraethoxide to give a compound 12.

The amount of tert-butylsulfinylamide used is 1.0 mol to 2.0 mol, for example, and preferably 1.0 mol to 1.2 mol per mol of the compound represented by formula (IVa). The amount of titanium tetraethoxide used is 1.0 mol to 3.0 mol, for example, and preferably 1.0 mol to 2.0 mol per mol of the compound represented by formula (IVa).

Examples of reaction solvents include THF, diethylether, and the like. The reaction temperature is 20°C to a temperature of heating under reflux, for example, and the reaction is usually completed within 1 hour to 12 hours.

### Step 18

The compound 12 is reacted with a compound 13 in a reaction solvent to give a compound 14.

The amount of compound 13 used is 1.0 mol to 5.0 mol, for example, and preferably 2.0 mol to 3.0 mol per mol of the compound 12.

Examples of reaction solvents include THF, diethylether, toluene, and the like. The reaction temperature is -78°C to room temperature, for example, and is preferably -78°C to 0°C. The reaction is usually completed within 1 hour to 12 hours.

In Step 17, when optically active tert-butylsulfinylamide is used, R⁵ can be stereoselectively introduced into the compound 12.

### Step 19

The tert-butylsulfinyl group of the compound 14 is removed at 0°C to room temperature using TFA or an aqueous hydrochloric acid solution to give the compound represented by formula (IIc).

Examples of compounds represented by compound 13 include methylmagnesium bromide, ethylmagnesium bromide, cyclopropylmagnesium bromide, and the like.

### Production method 8

Production method 8 is a method for preparing a compound represented by formula (IIb) from a known compound 15. For the reaction method and reaction conditions, known methods and those described in the above production methods 1 to 7 can be referred to.

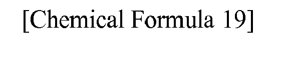

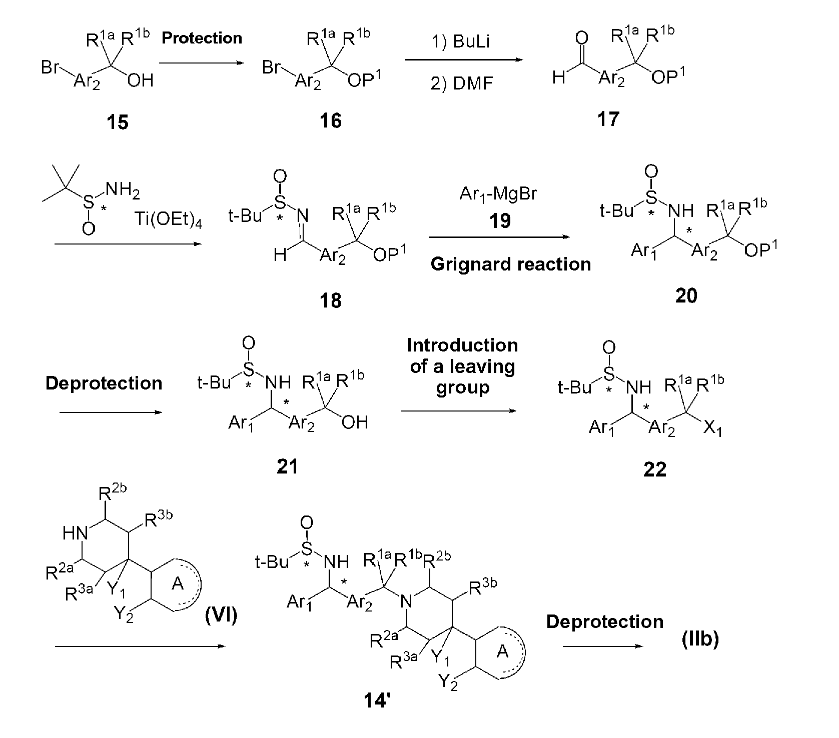

In the formulae, P¹ represents a tert-butyldimethylsilyl group or like hydroxy-protecting group, and other symbols are as defined above.

Specifically, the hydroxy group of the compound 15 is protected to give a compound 16. The compound 16 is reacted with n-butyllithium at -78°C to prepare a lithium reagent, and DMF is added dropwise to the obtained solution, to give a compound 17. The compound 17 is reacted according to Step 17 to give a compound 18. The compound 18 is subjected to Grignard reaction with a compound 19 at -78°C to give a compound 20. Subsequently, the compound 20 is reacted according to the Steps 13 to 16 of the production method 6, to give the compound (IIb).

Examples of compounds represented by compound 15 include (6-bromopyridin-3-yl)methanol and the like.

Examples of compounds represented by compound 19 include 3,4-difluorophenylmagnesium bromide and the like.

### Production method 9

Production method 9 is a method for preparing a compound 20' from a known compound 23 (a compound described in WO2008/038692). For the reaction method and reaction conditions, known methods and those described in the above production method 7 can be referred to. By reactions in accordance with the production method 8, the compound 20' can be converted into a compound represented by formula (IIc).

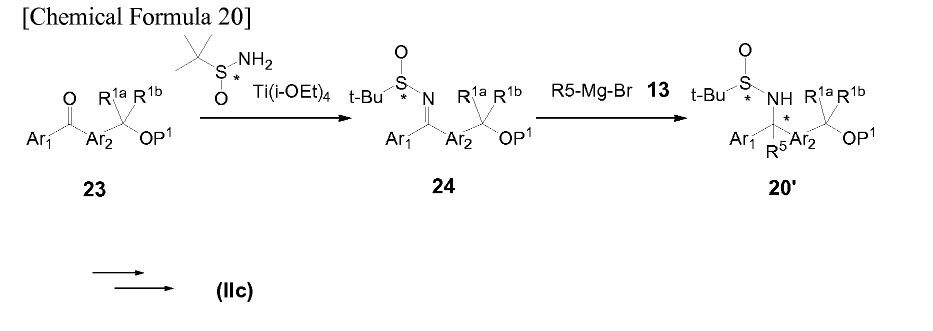

In the formulae, the symbols are as defined above.

### Production method 10

Production method 10 is a method for preparing a compound represented by formula (Va) using known compounds 23 and 27 (compounds both described in WO2008/038692). For the reaction method and reaction conditions, known methods and those described in the above production methods 1 to 8 can be referred to.

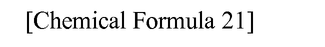

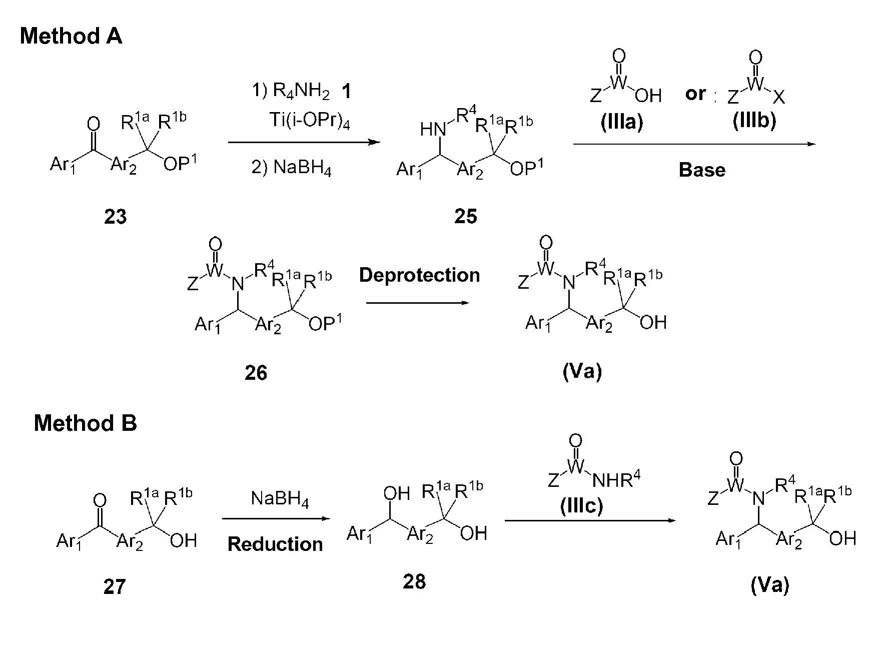

In the formulae, the symbols are as defined above.

In the above-mentioned production method, when the reactant has an amino group, a hydroxy group, a carboxyl group, an oxo group, a carbonyl group, and the like that are not involved in the reaction, then the amino group, the hydroxy group, the carboxyl group, the oxo group, and the carbonyl group may be suitably protected with an amino-protecting group, a hydroxy-protecting group, a carboxyl-protecting group, or an oxo- or carbonyl-protecting group prior to each reaction of the above production methods, followed by removal of the protecting groups after the reaction.

Although this depends on the type of the protecting group, the stability of the target compound, and the like, the protecting group can be introduced and removed, for example, according to the method described in the literature [see Protective Groups in Organic Synthesis, T.W. Greene, John Wiley & Sons, (1981)] or a similar method, by, for example, solvolysis with acid or base, i.e., for example, with 0.01 mol to a large excess of acid, preferably TFA, formic acid, hydrochloric acid, or the like, or with an equimolar amount to a large excess of base, preferably sodium hydroxide, potassium hydroxide, or the like; chemical reduction using a metal hydride complex; catalytic reduction using a palladium-carbon catalyst, a Raney nickel catalyst, or the like; etc.

The amino-protecting group is not limited as long as it has such a function. Examples thereof include aralkyl such as benzyl, p-methoxybenzyl, or like; C₁₋₆ alkanoyl such as acetyl, propionyl, or like; benzoyl; arylalkanoyl such as phenylacetyl; C₁₋₆ alkoxycarbonyl such as methoxycarbonyl, ethoxycarbonyl, tert-butoxycarbonyl, or like; alkoxycarbonyl such as benzyloxycarbonyl, p-nitrobenzyloxycarbonyl, or like; C₁₋₆ alkylsilyl such as trimethylsilyl, tert-butyldimethylsilyl, or like; tetrahydropyranyl; trimethylsilylethoxymethyl; C₁₋₆ alkylsulfonyl such as methylsulfonyl, ethylsulfonyl, or like; arylsulfonyl such as benzenesulfonyl, toluenesulfonyl, or like; etc. In particular, acetyl, benzoyl, tert-butoxycarbonyl, trimethylsilylethoxymethyl, methylsulfonyl, and the like are preferable.

The hydroxy-protecting group is not limited as long as it has such a function. Examples thereof include C₁₋₆ alkyl such as methyl, ethyl, propyl, or like; C₁₋₆ alkylsilyl such as trimethylsilyl, tert-butyldimethylsilyl, or like; C₁₋₆ alkoxymethyl such as methoxymethyl, 2-methoxyethoxymethyl, or like; tetrahydropyranyl; trimethylsilylethoxymethyl; aralkyl such as benzyl, p-methoxybenzyl, trityl, or like; formyl, acetyl, or like acyl; etc. In particular, methyl, methoxymethyl, tetrahydropyranyl, trityl, trimethylsilylethoxymethyl, tert-butyldimethylsilyl, acetyl, and the like are preferable.

The carboxyl-protecting group is not limited as long as it has such a function. Examples thereof include C₁₋₆ alkyl such as methyl, ethyl, propyl, tert-butyl, or like; halo C₁₋₆ alkyl such as 2,2,2-trichloroethyl or like; C₁₋₆ alkenyl such as 2-propenyl or like; aralkyl such as benzyl, p-methoxybenzyl, p-nitrobenzyl, trityl, or like; etc. In particular, methyl, ethyl, tert-butyl, 2-propenyl, benzyl, p-methoxybenzyl, and the like are preferable.

The oxo- and carbonyl-protecting group is not limited as long as it has such a function. Examples thereof include acetals and ketals such as ethylene ketal, dimethyl ketal, S,S'-dimethyl ketal, and like.

The thus-obtained compound of formula (I) can be readily isolated and purified by an ordinary isolation procedure, such as solvent extraction, recrystallization, column chromatography, preparative thin-layer chromatography, high-performance liquid chromatography, or the like.

The effects of the compound of the invention as an MCH receptor antagonist are demonstrated, for example, by the pharmacological test example given below.

### Pharmacological test example: MCH binding inhibition test

A cDNA sequence encoding human MCH-1R [FEBS Letters, Vol. 398, 253 (1996); Biochimica et Biophisica Acta, Vol. 1401, 216 (1998)] was cloned to a plasmid vector pEF/myc/cyto (manufactured by Invitrogen). The obtained expression vector was transfected to a host cell CHO-K1 (American Type Culture Collection) using Lipofectamine Plus reagent (manufactured by Life Technology) to provide MCH-1R expression cells.

Membrane samples prepared from the MCH-1R expression cells were incubated with a test compound and 50 pM [¹²⁵I]MCH (manufactured by NEN)in an assay buffer (50 mM Tris buffer containing 10 mM magnesium chloride, 2 mM ethylenediamine tetraacetate, 0.01 % bacitracin, and 0.2% bovine serum albumin; pH 7.4) at 25°C for one hour, followed by filtration through a glass filter GF/C (manufactured by Wattman). The glass filter was washed with 50 mM Tris buffer (pH 7.4) containing 10 mM magnesium chloride, 2 mM ethylenediamine tetraacetate, and 0.04% Tween-20, and then the radioactivity on the glass filter was determined. Non-specific binding was measured in the presence of 1 µM human MCH, and, with respect to each test compound, 50% inhibition concentration (IC₅₀ value) for specific [¹²⁵I]MCH binding was determined. The results are shown in Table 1.

**[Table 1]**

| **Example No.** | **Structure** | IC50(nM) |
|---|---|---|
| **Example 1-20** | 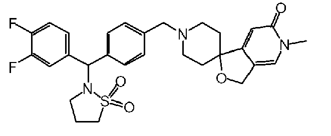 | 2.9 |
| **Example 1-37** | 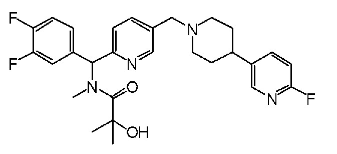 | 17 |
| **Example 1-58** | 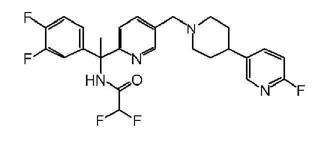 | 1.7 |
| **Example 2-1** | 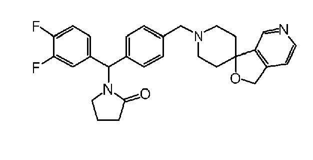 | 0.26 |
| **Example 2-5** | 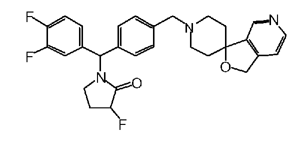 | 0.18 |

As above, compounds of the invention potently inhibited binding of MCH to MCH-1R, and acted as an excellent MCH-1R antagonist.

Therefore, the compound of the invention is effective as a preventive or a remedy for various MCH-related diseases, such as metabolic diseases such as obesity, diabetes, hormone secretion disorder, hyperlipemia, gout, fatty liver, and like; circulatory diseases such as angina pectoris, acute/congestive cardiac insufficiency, myocardial infarction, coronary arteriosclerosis, hypertension, nephropathy, electrolyte abnormality, and like; central and peripheral nervous system diseases such as bulimia, affective disorder, depression, anxiety, epilepsy, delirium, dementia, schizophrenia, attention deficit/hyperactivity disorder, dysmnesia, somnipathy, cognitive impairment, dyskinesia, dysesthesia, dysosmia, morphine resistance, drug dependence, alcohol dependence, and like; reproductive system diseases such as infertility, premature delivery, sexual dysfunction, and like; and other conditions including digestive diseases, respiratory diseases, cancer, and chromatosis. The compound of the invention is especially useful as a preventive or a remedy for obesity, diabetes, fatty liver, bulimia, depression, or anxiety.

### Pharmaceutical composition comprising the compound represented by formula (I)

Compounds of the invention may be administered orally or parenterally. As formulated into a dosage form suitable for the administration route, the compound of the invention can be used as a pharmaceutical composition for the prevention, treatment, or remedy of the above diseases.

In clinical use of the compound of the invention, usually, the compound is formulated into various preparations together with pharmaceutically acceptable additives according to the dosage form, and may then be administered. As such additives, various additives ordinarily used in the field of pharmaceutical preparations are usable. Specific examples thereof include gelatin, lactose, sucrose, titanium oxide, starch, crystalline cellulose, hydroxypropyl methylcellulose, carboxymethylcellulose, corn starch, microcrystalline wax, white petrolatum, magnesium metasilicate aluminate, anhydrous calcium phosphate, citric acid, trisodium citrate, hydroxypropylcellulose, sorbitol, sorbitan fatty acid ester, polysorbate, sucrose fatty acid ester, polyoxyethylene, hardened castor oil, polyvinylpyrrolidone, magnesium stearate, light silicic acid anhydride, talc, vegetable oil, benzyl alcohol, gum arabic, propylene glycol, polyalkylene glycol, cyclodextrin, hydroxypropyl cyclodextrin, and the like.

Preparations to be formed with those additives include, for example, solid preparations such as tablets, capsules, granules, powders, suppositories et al; and liquid preparations such as syrups, elixirs, injections et al. These may be formulated according to conventional methods known in the field of pharmaceutical preparations. The liquid preparations may also be in such a form that may be dissolved or suspended in water or in any other suitable medium in their use. Especially for injections, if desired, the preparations may be dissolved or suspended in physiological saline or glucose liquid, and a buffer or a preservative may be optionally added thereto.

The pharmaceutical compositions may contain the compound of the invention in an amount of from 1 to 99.9 % by weight, preferably from 1 to 60 % by weight of the composition. The compositions may further contain any other therapeutically-effective compounds.

In case where the compounds of the invention are used for prevention or treatment for the above-mentioned diseases, the dose and the dosing frequency may be varied, depending on the sex, the age, the body weight and the disease condition of the patient and on the type and the range of the intended remedial effect. In general, when orally administered, the dose may be from 0.001 to 50 mg/kg of body weight/day, and it may be administered at a time or in several times. The dose is preferably from about 0.01 to about 25 mg/kg/day, more preferably from about 0.05 to about 10 mg/kg/day.

As combination therapy, the compounds of the invention can be used in combination with drugs effective for hypertension, obesity-associated hypertension, hypertension-associated diseases, hypertrophy, left ventricular hypertrophy, metabolic disorders, obesity, obesity-associated diseases and the like (hereafter referred to as "co-drug"). Such drugs can be administered simultaneously, separately or in succession, for prevention or treatment of the above-mentioned diseases. When a compound of the invention is used simultaneously with one, two or more of co-drugs, they may be formulated into a medical preparation suited for single administration form. However, in combination therapy, a composition containing the compound of the invention and co-drugs may be administered to the object of medication in different packages, either simultaneously, separately or successively. They may be administered at time intervals.

The dose of the co-drug may be determined in accordance with the clinically adopted dose thereof, which can be suitably selected according to the individual object of medication, the administration route, the specific disease, the combination of drugs, and the like. The form of the co-drug for administration is not specifically limited, it may be combined with the compound of the invention when they are administered.

The administration mode includes, for example, the following: (1) A compound of the invention is combined with a co-drug to give a single preparation for single administration; (2) a compound of the invention and a co-drug are separately formulated into different two preparations, and the two preparations are simultaneously administered in one administration route; (3) a compound of the invention and a co-drug are separately formulated into different two preparations, and they are administered at different times in one and the same administration route; (4) a compound of the invention and a co-drug are separately formulated into different two preparations, and they are administered at the same time in two different administration routes; (5) a compound of the invention and a co-drug are separately formulated into different two preparations, and they are administered at different times in different administration routes (for example, a compound of the invention and a co-drug are administered in that order, or in an order contrary to this). The blend ratio of the compound of the invention and the co-drug may be suitably determined depending on the administration object, the administration route, and the disease for the administration.

The co-drug usable in the invention include, for example, remedy for diabetes, remedy for hyperlipidemia, remedy for hypertension, anti-obesity drug. Two or more such co-drugs may be combined in an adequate ratio and used.

The remedy for diabetes include, for example, 1) PPAR-γ agonists such as glitazones (e.g., ciglitazone, darglitazone, englitazone, isaglitazone (MCC-555) et al), pioglitazone, rosiglitazone, troglitazone, BRL49653, CLX-0921, 5-BTZD, GW-0207, LG-100641, LY-300512et al; 2) biguanides such as metformin, buformin, phenformin et al; 3) protein tyrosine phosphatase 1B inhibitors; 4) sulfonylureas such as acetohexamide, chloropropamide, diabinese, glibenclamide, glipizide, glyburide, glimepiride, gliclazide, glipentide, gliquidone, glisolamide, trazamide, tolubutamide et al; 5) meglitinides such as repaglinide, nateglinide et al; 6) α-glucoside hydroxylase inhibitors such as acarbose, adiposine, camiglibose, emiglitate, miglitol, voglibose, pradimicin-Q, salbostatin, CKD-711, MDL-25, 673, MDL-73, 945, MOR14 et al; 7) α-amylase inhibitors such as tendamistat, trestatin, A13688 et al; 8) insulin secretion promoters such as linogliride, A-4166 et al; 9) fatty acid oxidation inhibitors such as clomoxir, etomoxir et al; 10) A2 antagonists such as midaglizole, isaglidole, deriglidole, idazoxan, earoxan, fluparoxan et al; 11) insulin or insulin mimetics such as biota, LP-100, novalapid, insulindetermir, insulin lispro, insulin glargine, insulin zinc, Lys-Pro-insulin, GLP-1 (73-7), GLP1 amide (7-36) et al; 12) non-thiazolidinediones such as JT-501, farglitazar et al; 13) PPARα/γ dual-agonists such as MK-0767, CLX-0940, GW-1536, GW-1929, GW-2433, KRP-297, L-796449, LR-90 and SB219994 et al.

The remedy for hyperlipidemia include, for example, 1) bile acid absorption promoters such as cholesterylamine, colesevelem, colestipol, crosslinked dextran dialkylaminoalkyl derivatives, Colestid^{™}, LoCholest^{™}, Questran^{™} et al; 2) HMG-CoA reductase inhibitors such as atorvastatin, itavastatin, fluvastatin, lovastatin, pravastatin, rivastatin, rosuvastatin, simvastatin, ZD-4522 et al; 3) HMG-CoA synthase inhibitors; 4) cholesterol absorption inhibitors such as snatol ester, β-sitosterol, sterol glucoside, ezetimibe et al; 5) acyl-coenzyme A·cholesterol acyl transferase inhibitors such as avasimibe, eflucimibe, KY-505, SMP-709 et al; 6) CETP inhibitors such as JTT705, torcetrapib, CP532632, BAY-63-2149, SC-591, SC-795 et al; 7) squalane synthesis inhibitors; 8) antioxidants such as probucol; 9) PPAR-α agonists such as beclofibrate, benzafibrate, ciprofibrate, clofibrate, etofibrate, fenofibrate, gemcabene, gemfibrozil, GW-7647, BM-170744, LY-518674, fibric acid derivatives (e.g., Atromid^{™}, Lopid^{™}, Tricor^{™}) et al; 10) FXR receptor antagonists such as GW-4064, SR-103912 et al; 11) LXR receptor agonists such as GW3965, T9013137, XTCO-179628 et al; 12) lipoprotein synthesis inhibitors such as niacin; 13) renin-angiotensin system inhibitors; 14) microsome-triglyceride transport inhibitors; 15) bile acid resorption inhibitors such as BARA1453, SC435, PHA384640, S-435, AZD7706 et al; 16) PPAR-δ agonists such as GW501516, GW590735 et al; 17) triglyceride synthesis inhibitors; 18) MTTP inhibitors such as LAB687, CP346086 et al; 19) low-density lipoprotein receptor inducers; 20) squalane epoxidase inhibitors; 21) platelet agglutination inhibitors; 22) 5-lipoxygenase activated protein inhibitors such as MK-591.

The remedy for hypertension include, for example, 1) thiazide diuretics such as chlorothialidon, chlorothiazide, dichlorofenamide, hydrofluorothiazide, indapamide, hydrochlorothiazide et al; loop diuretics such as bumetanide, ethacrynic acid, flosemide, tolusemide et al; sodium diuretics such as amyloride, triamterene et al; aldosterone antagonist diuretics such as spironolactone, epilenone et al; 2) β-adrenaline blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, probanolol, sotalol, tertatolol, tilisolol, timolol et al; 3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, verapamil et al; 4) angiotensin converting enzyme inhibitors such as benazepril, captopril, cilazapril, delapril, enalapril, fosinopril, imidapril, rosinopril, moexipril, quinapril, quinaprilat, ramipril, perindopril, perindoropril, quanipril, spirapril, tenocapril, trandolapril, zofenopril et al; 5) neutral endopeptidase inhibitors such as omapatrilat, cadoxatril, ecadotril, fosidotril, sampatrilat, AVE7688, ER4030 et al; 6) endotheline antagonists such as tezosentan, A308165, YM62899 et al; 7) vasodilators such as hydralazine, clonidine, minoxidil, nicotinyl alcohol et al; 8) angiotensin II antagonists such as candesartan, eporsartan, iribesartan, losartan, pratosartan, tasosartan, telmisartan, valsartan, EXP-3137, FI6828K, RNH6270 et al; 9) α/β adrenaline blockers such as nipradilol, arotinolol, amoslalol et al; 10) α1 blockers such as terazosin, urapidil, prazosin, bunazosin, trimazosin, doxazosin, naphthopidil, indolamin, WHIP 164, XEN010 et al; 11) α2 agonists such as lofexidine, tiamenidine, moxonidine, rilmenidine, guanobenz et al; and 12) aldosterone inhibitors.

The anti-obesity drugs include, for example, 1) 5HT (serotonin) transporter inhibitors such as paroxetine, fluoxetine, fenfluramine, fluvoxamine, sertraline, imipramine et al; 2) norepinephrine transporter inhibitors such as GW320659, desipramine, talsupram, nomifensin et al; 3) cannabinoid-1 receptor 1 (CB-1) antagonists/inverse-agonists such as limonabant (Sanofi Synthelabo), SR-147778 (Sanofi Synthelabo), BAY-65-2520 (Bayer), SLV-319 (Solvey), as well as compounds disclosed in USP 5,532,237, USP 4,973,587, USP 5,013,837, USP 5,081,122, USP 5,112,820, USP 5,292,736, USP 5,624,941, USP 6,028,084, WO96/33159, WO98/33765, WO98/43636, WO98/43635, WO01/09120, WO01/96330, WO98/31227, WO98/41519, WO98/37061, WO00/10967, WO00/10968, WO97/29079, WO99/02499, WO01/58869, WO02/076949, WO01/64632, WO01/64633, WO01/64634, WO03/006007, WO03/007887 and EP-658546 et al; 4) ghrelin antagonists such as compounds disclosed in WO01/87355, WO02/08250 et al; 5) histamine(H3) antagonists/inverse-agonists such as thioperamide, 3-(1H-imidazol-4-yl)propyl N-(pentenyl)carbonate, clobenpropit, iodofenpropit, imoproxyfen, GT2395, A331440, compounds disclosed in WO02/15905, O-[3-(1H-imidazol-4-yl)propanol] carbamate, piperazine-containing H3-receptor antagonists (Lazewska, D. et al., Pharmazie, 56: 927-32 (2001), benzophenone derivatives (Sasse, A. et al., Arch. Pharm. (Weinheim) 334: 45-52 (2001)), substituted N-phenylcarbamates (Reidemeister, S. et al., Pharmazie, 55: 83-6 (2000)), proxyfen derivatives (Sasse, A. et al., J. Med. Chem., 43: 3335-43 (2000)) et al; 6) MCH-1R antagonists such as T-226296 (Takeda), SNP-7941 (Synaptic), other compounds disclosed in WO01/82925, WO01/87834, WO02/051809, WO02/06245, WO02/076929, WO02/076947, WO02/04433, WO02/51809, WO02/083134, WO02/094799, WO03/004027 and JP-A-2001-226269 et al; 7) MCH-2R agonists/antagonists; 8) NPY1 antagonists such as isopropyl 3-chloro-5-(1-(6-[2-(5-ethyl-4-methyl-thiazol-2-yl)-ethyl]-4-morpholinyl-4-yl-piridin-2-ylamino)-ethyl)phenyl]carbamate, BIBP3226, BIBO3304, LY-357897, CP-671906, GI-264879, and other compounds disclosed in USP 6,001,836, WO96/14307, WO01/23387, WO99/51600, WO01/85690, WO01/85098, WO01/85173 and WO01/89528 et al; 9) NPY5 antagonists such as 152804, GW-569180A, GW-594884A, GW-587081X, GW-548118X, FR235,208, FR226928, FR240662, FR252384, 1229U91, GI-264879A, CGP71683A, LY-377897, LY366377, PD-160170, SR-120562A, SR-120819A, JCF-104, H409/22, and other compounds disclosed in USP 6,140,354, USP 6,191,160, USP 6,258,837, USP 6,313,298, USP 6,337,332, USP 6,329,395, USP 340,683, USP 6,326,375, USP 6,329,395, USP 6,337,332, USP 6,335,345, EP-01010691, EP-01044970, WO97/19682, WO97/20820, WO97/20821, WO97/20822, WO97/20823, WO98/27063, WO00/107409, WO00/185714, WO00/185730, WO00/64880, WO00/68197, WO00/69849, WO01/09120, WO01/14376, WO01/85714, WO1/85730, WO01/07409, WO01/02379, WO01/02379, WO01/23388, WO01/23389, WO01/44201, WO01/62737, WO01/62738, WO01/09120, WO02/20488, WO02/22592, WO02/48152, WO02/49648, WO02/094789, and compounds disclosed in Norman et al., J. Med. Chem., 43:4288-4312(2000) et al; 10) leptins such as human recombinant leptin (PEG-OB, Hoffman La Roche), recombinant methionylleptin (Amgen) et al; 11) leptin derivatives such as compounds disclosed in USP 5,552,524, USP 5,552,523, USP 5,552,522, USP 5,521,283, WO96/23513, WO96/23514, WO96/23515, WO96/23516, WO96/23517, 96/23518, WO96/23519 and WO96/23520 et al; 12) opioid antagonists such as nalmefen (Revex^{™}), 3-methoxynaltorexone, naloxone, naltorexone, compounds disclosed in WO00/21509 et al; 13) orexin antagonists such as SB-334867A, and other compounds disclosed in WO01/96302, WO01/68609, WO02/51232, WO02/51838 and WO03/023561 et al; 14) bombesin receptor subtype-3 agonists; 15) cholecystokinin A (CCK-A) agonists such as AR-R15849, GI-181771, JMV-180, A-71378, A-71623, SR-146131, and other compounds disclosed in USP 5,739,106 et al; 16) CNTF (ciliary neurotrophic factors) such as GI-181771 (Glaxo-Smith Kline), SR146131 (Sanofi Synthelabo), butabindide, PD170,292, PD149164 (Pfizer) et al; 17) CNTF derivatives such as axokine (Regeneron), and other compounds disclosed in WO94/09134, WO98/22128, WO99/43813 et al; 18) growth hormone secretion receptor agonists such as NN703, hexarelin, MK-0677, SM-130686, CP-424,391, L-692,429, L-163,255, and compounds disclosed in USP 6,358,951, US Patent Application Nos. 2002/049196, 2002/022637, WO01/56592, WO02/32888 et al; 19) serotonin receptor-2C agonists such as BVT933, DPCA37215, IK264, PNU22394, WAY161503, R-1065, YM348, and other compounds disclosed in USP 3,914,250, WO02/36596, WO02/48124, WO02/10169, WO01/66548, WO02/44152, WO02/51844, WO02/40456 and WO02/40457 et al; 20) melanocortin-3 receptor agonists; 21) melanocortin-4 receptor agonists such as CHIR86036 (Chiron), ME-10142, ME-10145 (Melacure), and other compounds disclosed in WO99/64002, WO00/74679, WO01/991752, WO01/74844, WO01/70708, WO01/70337, WO01/91752, WO02/059095, WO02/059107, WO02/059108, WO02/059117, WO02/12166, WO02/11715, WO02/12178, WO02/15909, WO02/068387, WO02/068388, WO02/067869, WO03/007949 and WO03/009847 et al; 22) monoamine resorption inhibitors such as sibutramine (Meridia^{™}/Reductil^{™}) and its salts, and other derivatives disclosed in USP 4,746,680, USP 4,806,570, USP 5,436,272, US Patent Application No. 2002/0006964, WO01/27068 and WO01/62341 et al; 23) serotonin re-uptake inhibitors such as dexfenfluramine, fluoxetine, and other compounds disclosed in USP 6,365,633, WO01/27060 and WO01/162341 et al; 24) glucagon-like peptide-1 agonists; 25) topiramate (Topimax^{™}); 26) phytopharm compound 57 (e.g., CP644,673); 27) acetyl CoA carboxylase-2 (ACC2) inhibitors; 28) β-adrenalin receptor-3 agonists such as AD9677/TAK677 (Dai-Nippon Pharmaceutical/Takeda Chemical), CL-316,243, SB418790, BRL-37344, L-796568, BMS-196085, BRL-35135A, CGP12177A, BTA-243, W427353, Trecadrine, Zeneca D7114, SR59119A, and other compounds disclosed in USP 5,705,515, USP 5,451,677, WO01/74782 and WO02/32897 et al; 29) diacylglycerol acyltransferase-1 inhibitors; 30) diacylglycerol acyltransferase-2 inhibitors, 31) fatty acid synthesis inhibitors such as carulenin, C75; 32) phosphodiesterase inhibitors such as theophylline, pentoxiphylline zaprinast, sildenafil, amrinone, milrinone, cilostamide, rolipram and cilomilast et al; 33) thyroid hormone-β agonists such as KB-2611 (KaroBio BMS), and other compounds disclosed in WO02/15845, JP-A-2000-256190 et al; 34) UCP (uncoupling protein)-1, 2, or 3 activators such as phytanic acid, 4-[(E)-2-(5,6,7,8-tetrahydro-5,5,8,8-tetramethyl-2-naphthalenyl)-1-propenyl]benzoic acid (TTNPB), retinoic acid, and other compounds disclosed in WO99/00123 et al; 35) acylestrogens such as oleoylestrone, and other compounds disclosed in del Mar-Grasa, M. et al., Obesity Research, 9:202-9 (2001) et al; 36) glucocorticoid antagonists; 37) 11-β-hydroxysteroid dehydrogenase-1 inhibitors such as BVT3498, BVT2733, and other compounds disclosed in WO01/90091, WO01/90090, WO01/90092 et al; 38) stearoyl-CoA desaturase-1 inhibitors; 39) dipeptidyl peptidase-IV inhibitors such as isoleucine thiazolidide, valine pyrrolidide, NVP-DPP728, AF237, P93/01, TSL225, TMC-2A/2B/2C, FE999011, P9310/K364, VIP0177, SDZ274-444, and other compounds disclosed in WO03/004498, WO03/004496, EP1258476, WO02/083128, WO02/062764, WO03/000250, WO03/002530, WO03/002531, WO03/002553, WO03/002593, WO03/000180 and WO03/000181 et al; 40) lipase inhibitors such as tetrahydroliptatin (Orlistat/Xenical^{™}), Triton WR1339, RHC80267, lipstatin, teasaponin, diethylumbelliferyl phosphate, FL-386, WAY-121898, Bay-N-3176, valilactone, esteracin, ebelactone A, ebelactone B, RHC80267, and other compounds disclosed in WO01/77094, USP 4,598,089, USP 4,452,813, USP 5,512,565, USP 5,391,571, USP 5,602,151, USP 4,405,644, USP 4,189,438 and USP 4,242,453 et al; 41) fatty acid transporter inhibitors; 42) dicarboxylate transporter inhibitors; 43) glucose transporter inhibitors; 44) phosphate transporter inhibitors.

Those combined drugs are obtained by combining a compound of the invention with one, two or more of the above co-drugs. Furthermore, the combined drugs are useful for prevention or treatment of metabolic disorders, when combined with one, two or more drugs selected from the group consisting of remedy for diabetes and remedy for hyperlipidemia. Combinations containing, in particular, remedy for hypertension and anti-obesity agent are useful for prevention or treatment of metabolic disorders with synergistic effect, when remedy for diabetes and/or remedy for hyperlipidemia are added thereto.

On the other hand, the compound of the invention may be combined with an antipsychotic. An antipsychotic, especially an atypical antipsychotic is known to have a side effect of body weight increase; and the compound of the invention, when combined with such an antipsychotic, is useful for retarding the side effect. The antipsychotic includes, for example, olanzapine, Risperidone, quetiapine, Ziprasidone, aripiprazole, Paliperidone, Clozapine et al. Using an antipsychotic, as combined with a compound of the invention, may improve the level of metabolic parameters such as the level of blood pressure, glucose and lipid level that may be elevated by the antipsychotic. The above-mentioned methods may apply to the conditions of dose, administration subj ect, administration route and administration form.

### BEST MODE FOR CARRYING OUT THE INVENTION

Hereinafter, the invention will be described in further detail with reference to the Examples; however, the invention is not these examples. As silica gel for the column, Wakogel™ C-200 (Wako Pure Chemical Industries) was used. As filled silica gel columns, FLASH+™ cartridge, KP-Sil or FPNH, FLASH 12+M, FLASH 25+S, FLASH 25+M, FLASH 40+M (Biotage Japan), and the like were used. Kieselgel 60 F254 (Merck) was used for preparative thin-layer chromatography, and PLC 05 NH (FUJI Silysia) was used for basic preparative thin-layer chromatography. The ¹H NMR spectra were measured using JNM-AL 400 (manufactured by JEOL), MERCURY vx 400 (manufactured by VARIAN), ^{UNITY}INOVA 400 (manufactured by VARIAN), or Avance 300 (Bruker), and MS spectra were measured using ZQ 2000 (Waters).

### EXAMPLES

### Reference Example 1: Synthesis of amine (IIb)

### Reference Example 1-1

### Synthesis of 1-[4-({[tert-butyl(dimethyl)silyl]oxy}methyl)phenyl]-1-(3,4-difluorophenyl)methanamine

To a THF solution (2.00 mL) of [(4-bromobenzyl)oxy](tert-butyl)dimethylsilane (1.50 g) were added magnesium metal (1.45 g) and a catalytic amount of iodine, and the mixture was heated under reflux for 1 hour. After cooling to room temperature, a THF solution (4.00 mL) of 3,4-difluorobenzonitrile (533 mg) was added to the reaction mixture, and then heated under reflux for 1 hour and a half.
After cooling to 0°C, methanol (10.0 mL) and sodium borohydroxide (283 mg) were added thereto, and stirred at room temperature for 1 hour. The reaction mixture was filtered through Celite, and the filtrate was condensed under reduced pressure. The residue was purified by silica gel column chromatography (chloroform:methanol = 100:0 to 80:20) to give the title compound (897 mg) as a yellow oil.
ESI-MS Found: m/z 364[M+H]⁺

### Reference Example 1-2

### Synthesis of tert-butyl[[4-({tert-butyl(dimethyl)silyl]oxy}methyl)phenyl](3,4-difluorophenyl)methyl]carbamate

To a DMF solution (20.0 mL) of the compound obtained in Reference Example 1-1 (1.92 g) was added triethylamine (3.66 mL). Subsequently, di-tert-butyl dicarbonate (1.38 mL) was added thereto, and the mixture was stirred at room temperature for 2 hours. An aqueous sodium hydrogen carbonate solution was added to the reaction mixture, followed by extraction with ethyl acetate. The organic layer was washed with water, and then dried over anhydrous magnesium sulfate. The organic layer was condensed under reduced pressure to give a crude product of the title as a yellow oil.

### ESI-MS Found: m/z 486[M+Na]⁺

### Reference Example 1-3

### Synthesis of tert-butyl{(3,4-difluorophenyl)[4-(hydroxymethyl)phenyl]methyl}carbamate

To a THF solution (20.0 mL) of the crude product obtained in Reference Example 1-2 was added a 1.00 M THF solution (1.45 mL) of tetrabutylammonium fluoride, and the mixture was stirred at room temperature for 30 minutes. An aqueous sodium hydrogen carbonate solution was added to the reaction mixture, followed by extraction with ethyl acetate, and the organic layer was dried over anhydrous sodium sulfate. The organic layer was concentrated under reduced pressure, and then the residue was purified by silica gel column chromatography (hexane:ethyl acetate = 100:0 to 50:50) to give the title compound (1.43 g) as a yellow oil. ESI-MS Found: m/z 350[M+H]⁺

### Reference Example 1-4

### Synthesis of tert-butyl{(3,4-difluorophenyl)[4-(1H,1'H-spiro[furo[3,4-c]pyridine-3,4'-piperidin]-1'-ylmethyl)phenyl]methyl}carbamate

To the compound obtained in Reference Example 1-3 (822 mg) and an ethyl acetate solution (10.0 mL) of N,N-diisopropylethylamine (1.23 mL) was added methanesulfonyl chloride (220 µL) at 0°C, and the mixture was stirred for 1 hour. An aqueous sodium hydrogen carbonate solution was added to the reaction mixture, followed by extraction with ethyl acetate. The organic layer was dried over anhydrous magnesium sulfate. The organic layer was condensed under reduced pressure, to give a crude product of the corresponding mesylate as a colorless oil.
N,N-Diisopropylethylamine (4.11 mL) was added to a chloroform solution (10.0 mL) of 1H-spiro[furo[3,4-c]pyridine-3,4'-piperidine] dihydrochloride (619 mg) at 0°C. A chloroform solution (10.0 mL) of the mesylate obtained above was added thereto, and stirred at room temperature overnight. An aqueous sodium hydrogen carbonate solution was added to the reaction mixture, followed by extraction with chloroform. The organic layer was dried over anhydrous magnesium sulfate, and then concentrated under reduced pressure. The residue was purified by silica gel column chromatography (chloroform:methanol = 100:0 to 80:20) to give the title compound (980 mg) as a white amorphous substance.
ESI-MS Found: m/z 522[M+H]⁺

### Reference Example 1-5

### Synthesis of 1-(3,4-difluorophenyl)-1-[4-(1H,1'H-spiro[furo[3,4-c]pyridine-3,4'-piperidin]-1'-ylmethyl)phenyl]methanamine trihydrochloride

To an ethyl acetate solution (10.0 mL) of the compound obtained in Reference Example 1-4 (980 mg) was added a 5.00 M aqueous hydrochloric acid solution (4.00 mL) at room temperature, and the mixture was stirred for 2 hour. The reaction mixture was concentrated under reduced pressure, followed by azeotropy with ethyl acetate. The residue was solidified from methanol/ethyl acetate to give the title compound (902 mg) as a white solid. ESI-MS Found: m/z 422[M+H]⁺

### Reference Example 1-6

### Synthesis of 1-(3,4-difluorophenyl)-1-(4-{[4-(6-fluoropyridin-3-yl)piperidin-1-yl]methyl}phenyl)methanamine dihydrochloride

The same operation as in Reference Examples 1-4 and 1-5 was performed using the compound obtained in Reference Example 1-3 (751 mg) and 2-fluoro-5-piperidin-4-ylpyridine hydrochloride (538 mg), to give the title compound (792 mg) as a white solid.

### ESI-MS Found: m/z 412[M+H]⁺

### Reference Example 1-7

### Synthesis of 1-(3,4-difluorophenyl)-1-{4-[(4-pyrazolo[1,5-b]pyridazin-3-ylpiperidin-1-yl)methyl]phenyl}methanamine dihydrochloride

The same operation as in Reference Example 1-6 was performed using the compound obtained in Reference Example 1-3 (751 mg) and 3-piperidin-4-ylpyrazolo[1,5-b]pyridazine hydrochloride (538 mg), to give the title compound (770 mg) as a white solid. ESI-MS Found: m/z 434[M+H]⁺

### Reference Example 1-8

### Synthesis of tert-butyl((3,4-difluorophenyl){4-[(5-methyl-6-oxo-5,6-dihydro-1'H,3H-spiro[furo[3,4-c]pyridine-1,4'-piperidin]-1'-yl)methyl]phenyl}methyl)carbamate

To an ethyl acetate solution (1.00 mL) of the compound obtained in Reference Example 1-3 (166 mg) and N,N-diisopropylethylamine (208 µL) was added methanesulfonyl chloride (41.0 µL) at 0°C, and the mixture was stirred for 1 hour. An aqueous sodium hydrogen carbonate solution was added to the reaction mixture, followed by extraction with ethyl acetate. The organic layer was dried over anhydrous magnesium sulfate. The organic layer was condensed under reduced pressure, to give a crude product of the corresponding mesylate as a colorless oil.
TFA (3.00 mL) was added to tert-butyl 5-methyl-6-oxo-5,6-dihydro-1'H,3H-spiro[furo[3,4-c]pyridine-1,4'-piperidine]-1'-carboxylate (152 mg)at 0°C, and stirred for 20 minutes. The reaction mixture was concentrated under reduced pressure, and chloroform (500 µL) was added to the residue. Subsequently, a chloroform (500 µL) solution ofN,N-diisopropylethylamine (831 µL) and a the above mesylate crude product were added dropwise thereto at 0°C. The mixture was stirred at 0°C for 1 hour, and then stirred at room temperature overnight. An aqueous sodium hydrogen carbonate solution was added to the reaction mixture, followed by extraction with ethyl acetate. The organic layer was washed with saturated brine and then dried over anhydrous sodium sulfate. The organic layer was concentrated under reduced pressure, and then the residue was purified by preparative thin-layer chromatography (chloroform:methanol = 90:10) to give the title compound (219 mg) as a white amorphous substance.

### ESI-MS Found: m/z 552[M+H]⁺

### Reference Example 1-9

### Synthesis of 1'-{4-[amino(3,4-difluorophenyl)methyl]benzyl}-5-methyl-3,5-dihydro-6H-spiro[furo[3,4-c]pyridine-1,4'-piperidin]-6-one dihydrochloride

The same operation as in Reference Example 1-5 was performed using the compound obtained in Reference Example 1-8 (219 mg), to give the title compound (182 mg) as a white solid.
ESI-MS Found: m/z 452[M+H]⁺

### Reference Example 2: Synthesis of amine (IIb)

### Reference Example 2-1

### Synthesis of (Z)-(3,4-difluorophenyl)[5-(1H,1'H-spiro[furo[3,4-c]pyridine-3,4'-piperidin]-1'-ylmethyl)pyridin-2-yl]methanone O-(2-hydroxy-2-methylpropyl)oxime

The same operation as in Reference Example 1-4 was performed using (Z)-(3,4-difluorophenyl)[5-(hydroxymethyl)pyridin-2-yl]methanone O-(2-hydroxy-2-methylpropyl)oxime (1.68 g), to give the title compound (2.15 g) as a pale yellow amorphous substance.
ESI-MS Found: m/z 509[M+H]⁺

### Reference Example 2-2

### Synthesis of 1-(3,4-difluorophenyl)-1-[5-(1H,1'H-spiro[furo[3,4-c]pyridine-3,4'-piperidin]-1'-ylmethyl)pyridin-2-yl]methanamine tetrahydrochloride

To a TFA solution (20.0 mL) of the compound obtained in Reference Example 2-1 (2.15 g) was slowly added zinc (1.38 g) under ice cooling, and the reaction mixture was stirred at room temperature for 1 hour and a half. The reaction mixture was filtered through Celite. The solvent was concentrated under reduced pressure, followed by azeotropy with ethyl acetate. Ethyl acetate was added to the residue, and a saturated aqueous sodium hydrogen carbonate solution was then added under ice cooling. The mixed solution was extracted with ethyl acetate. The organic layer was washed with water and saturated brine, and dried over anhydrous sodium sulfate.
The organic layer was concentrated under reduced pressure, and then the residue was purified by basic silica gel column chromatography (chloroform:methanol = 100:0 to 80:20). A dioxane solution (5.00 mL) of 4.00 M hydrogen chloride was added to a mixed solution of the obtained pale yellow amorphous in chloroform (10.0 mL) and methanol (10.0 mL), and stirred at room temperature for 5 minutes. The reaction mixture was concentrated under reduced pressure. The residue was crystallized from methanol/ethyl acetate to give the title compound (1.46 mg) as a white solid.
ESI-MS Found: m/z 423[M+H]⁺

### Reference Example 2-3

### Synthesis of 1-(3,4-difluorophenyl)-1-(5-{[4-(6-fluoropyridin-3-yl)piperidin-1-yl]methyl}pyridin-2-yl)methanamine dihydrochloride

The same operation as in Reference Examples 1-4 and 2-2 was performed using (Z)-(3,4-difluorophenyl)[5-(hydroxymethyl)pyridin-2-yl]methanone O-(2-hydroxy-2-methylpropyl)oxime (1.00 g) and 2-fluoro-5-piperidin-4-ylpyridine hydrochloride (752 mg), to give the title compound (845 mg) as a white solid.
ESI-MS Found: m/z 413[M+H]⁺

### Reference Example 3: Synthesis of amine (IIa)

### Reference Example 3-1

### Synthesis of 1-(3,4-difluorophenyl)-N-methyl-1-[4-(1H,1'H-spiro[furo[3,4-c]pyridine-3,4'-piperidin]-1'-ylmethyl)phenyl]methanamine

To a 0.87 M methanol solution (2.74 mL) of methylamine was added titanium tetraisopropoxide (307 µL) at room temperature, and the mixture was stirred for 5 minutes. A methanol solution (2.00 mL) of (3,4-difluorophenyl)[4-(1H,1'H-spiro[furo[3,4-c]pyridine-3,4'-piperidin]-1'-ylmethyl)phenyl]methanone (200 mg) was added to the reaction mixture, and then stirred at room temperature overnight. Titanium tetraisopropoxide (307 µL) was added to a 0.87 M methanol solution (2.74 mL) of methylamine at room temperature and then stirred for 5 minutes. To the thus-obtained solution was added the above reaction mixture, and the mixture was further stirred at room temperature for 2 hours and a half. Sodium borohydride (21.6 mg) was added to the reaction mixture, and stirred at room temperature for 2 hours. Sodium borohydride (21.6 mg) was further added thereto, and stirred at room temperature for two days. A 5% aqueous sodium hydrogen carbonate solution was added to the reaction mixture, and then filtered through Celite, followed by washing with chloroform and water. The filtrate was extracted with chloroform. The organic layer was washed with water and saturated brine, and dried over anhydrous sodium sulfate. The organic layer was concentrated under reduced pressure, and then the residue was purified by basic silica gel column chromatography (hexane:ethyl acetate = 100:0 to 0:100) to give the title compound (88.1 mg) as a colorless oil. ESI-MS Found: m/z 436[M+H]⁺

### Reference Example 3-2

### Synthesis of 1-(3,4-difluorophenyl)-N-methyl-1-[5-(1H,1'H-spiro[furo[3,4-c]pyridine-3,4'-piperidin]-1'-ylmethyl)pyridin-2-yl]methanamine

The same operation as in Reference Example 3-1 was performed using (3,4-difluorophenyl)[5-(1H,1'H-spiro[furo[3,4-c]pyridine-3,4'-piperidin]-1'-ylmethyl)pyridin-2-yl]methanone (200 mg), to give the title compound (148 mg) as a colorless oil.
ESI-MS Found: m/z 437[M+H]⁺

### Reference Example 3-3

### Synthesis of 1-(3,4-difluorophenyl)-1-(5-{[4-(6-fluoropyridin-3-yl)piperidin-1-yl]methyl}pyridin-2-yl)-N-methylmethanamine

The same operation as in Reference Example 3-1 was performed using (3,4-difluorophenyl)(5-{[4-(6-fluoropyridin-3-yl)piperidin-1-yl]methyl}pyridin-2-yl)methanone (44.4 mg), to give the title compound (35.2 mg) as a colorless oil.
ESI-MS Found: m/z 427[M+H]⁺

### Reference Example 3-4

### Synthesis of 1-(3,4-difluorophenyl)-N-methyl-1-[5-(1H,1'H-spiro[furo[3,4-c]pyridine-3,4'-piperidin]-1'-ylmethyl)pyrimidin-2-yl]methanamine

The same operation as in Reference Example 3-1 was performed using (3,4-difluorophenyl)[5-(1H,1'H-spiro [furo[3,4-c]pyridine-3,4'-piperidin]-1'-ylmethyl)pyrimidin-2-yl]methanone (87.0 mg), to give the title compound (40.9 mg) as a pale yellow solid.
ESI-MS Found: m/z 438[M+H]⁺

### Reference Example 3-5

### Synthesis of N-{(3,4-difluorophenyl) [5-(1H,1'H-spiro[furo[3,4-c]pyridine-3,4'-piperidin]-1'-ylmethyl)pyridin-2-yl]methyl}ethanamine

The same operation as in Reference Example 3-1 was performed using (3,4-difluorophenyl)[5-(1H,1'H-spiro[furo[3,4-c]pyridine-3,4'-piperidin]-1'-ylmethyl)pyridin-2-yl]methanone (50.0 mg) and a 0.59 M methanol solution of ethylamine, to give the title compound (46.3 mg) as a colorless oil.
ESI-MS Found: m/z 451[M+H]⁺

### Reference Example 3-6

### Synthesis of 1-({(3,4-difluorophenyl)[4-(1H,1'H-spiro[furo[3,4-c]pyridine-3,4'-piperidin]-1'-ylmethyl)phenyl]methyl}amino)-2-methylpropan-2-ol

The same operation as in Reference Example 3-1 was performed under heating conditions using (3,4-difluorophenyl)[4-(1H,1'H-spiro[furo[3,4-c]pyridine-3,4'-piperidin]-1'-ylmethyl)phenyl]methanone (50.0 mg) and 1-amino-2-methylpropan-2-ol, to give the title compound (42.0 mg) as a colorless oil.
ESI-MS Found: m/z 494[M+H]⁺

### Reference Example 3-7

### Synthesis of 1-({(3,4-difluorophenyl)[5-(1H,1'H-spiro[furo[3,4-c]pyridine-3,4'-piperidin]-1'-ylmethyl)pyridin-2-yl]methyl}amino)-2-methylpropan-2-ol

The same operation as in Reference Example 3-6 was performed using (3,4-difluorophenyl)[5-(1H,1'H-spiro[furo[3,4-c]pyridine-3,4'-piperidin]-1'-ylmethyl)pyridin-2-yl]methanone (200 mg), to give the title compound (113 mg) as a pale yellow-green amorphous substance.
ESI-MS Found: m/z 495[M+H]⁺

### Reference Example 3-8

### Synthesis of N-{(3,4-difluorophenyl)[5-(1H,1'H-spiro[furo[3,4-c]pyridine-3,4'-piperidin]-1'-ylmethyl)pyridin-2-yl]methyl}cyclopropanamine

To a methanol solution (3.00 mL) of cyclopropylamine (167 µL) was added titanium tetraisopropoxide (306 µL) at room temperature, and the mixture was stirred for 5 minutes. (3,4-Difluorophenyl)[5-(1H,1'H-spiro[furo[3,4-c]pyridine-3,4'-piperidin]-1'-ylmethyl)pyridin-2-yl]methanone (200 mg) was added to the reaction mixture at 0°C, and then stirred at room temperature overnight. The resulting reaction mixture was concentrated under reduced pressure, and then the residue was purified by basic preparative thin-layer chromatography (hexane:ethyl acetate = 40:60), to give a mixture of N-{(1E)-(3,4-difluorophenyl)[5-(1H,1'H-spiro[furo[3,4-c]pyridine-3,4'-piperidin]-1'-ylmethyl)pyridin-2-yl]methylene}cyclopropanamine and N-{(1Z)-(3,4-difluorophenyl)[5-(1H,1'H-spiro[furo[3,4-c]pyridine-3,4'-piperidin]-1'-ylmethyl)pyridin-2-yl]methylene}cyclopropanamine as a colorless oil (210 mg). Zinc (21.3 mg) was added a TFA solution (10.0 mL) of the obtained mixture (30.0 mg), and the reaction mixture was stirred at room temperature for 30 minutes. The reaction mixture was filtered through Celite. The solvent was concentrated under reduced pressure, followed by azeotropy with ethyl acetate. Ethyl acetate was added to the residue, and a saturated aqueous sodium hydrogen carbonate solution was then added under ice cooling. The mixed solution was extracted with ethyl acetate. The organic layer was washed with water and saturated brine, and dried over anhydrous magnesium sulfate. The organic layer was concentrated under reduced pressure, and then the residue was purified by silica gel column chromatography (chloroform:methanol = 100:0 to 80:20) to give the title compound (30.1 mg) as a colorless oil.
ESI-MS Found: m/z 463[M+H]⁺

### Reference Example 3-9

### Synthesis of 1-(5-chloropyridin-2-yl)-1-[4-(1H,1'H-spiro[furo[3,4-c]pyridine-3,4'-piperidin]-1'-ylmethyl)phenyl]methanamine

The same operation as in Reference Example 3-1 was performed using (5-chloropyridin-2-yl) [4-(1H,1'H-spiro[furo[3,4-c]pyridine-3,4'-piperidin]-1'-ylmethyl)phenyl]methanone (95.0 mg) and a 7.00 M methanol solution of ammonia, to give the title compound (67.6 mg) as a pale yellow oil.
ESI-MS Found: m/z 421, 423[M+H]⁺

### Reference Example 4: Synthesis of alcohol (IVb)

### Reference Example 4-1

### Synthesis of (3,4-difluorophenyl)[4-(1H,1'H-spiro[furo[3,4-c]pyridine-3,4'-piperidin]-1'-ylmethyl)phenyl]methanol

To a methanol solution (32.0 mL) of (3,4-difluorophenyl)[4-(1H,1'H-spiro[furo[3,4-c]pyridine-3,4'-piperidin]-1'-ylmethyl)phenyl]methanone (1.60 g) was added sodium borohydride (144 mg) at room temperature, and the mixture was stirred for 1 hour. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by silica gel column chromatography (chloroform:methanol = 40:0 to 8:1) to give the title compound (1.28 g) as a white amorphous substance.
ESI-MS Found: m/z 423[M+H]⁺

### Reference Example 4-2

### Synthesis of (3,4-difluorophenyl)[5-(1H,1'H-spiro[furo[3,4-c]pyridine-3,4'-piperidin]-1'-ylmethyl)pyridin-2-yl]methanol

The same operation as in Reference Example 4-1 was performed using (3,4-difluorophenyl)[5-(1H,1'H-spiro [furo [3,4-c]pyridine-3,4'-piperidin]-1'-ylmethyl)pyridin-2-yl]methanone (501 mg), to give the title compound (460 mg) as a white amorphous substance. ESI-MS Found: m/z 424[M+H]⁺

### Reference Example 5: Synthesis of alcohol (IVb)

### Reference Example 5-1

### Synthesis of 2,4,5-trifluoro-N-methoxy-N-methylbenzamide

To a chloroform solution (150 mL) of 2,4,5-trifluorobenzoic acid (10.0 g) were added N,O-dimethylhydroxylamine hydrochloride (11.1 g), 1-hydroxybenzotriazole hydrate (HOBT·H₂O) (13.0 g), WSC·HCl (16.3 g), and triethylamine (40.0 mL) at 0°C, and the mixture was stirred at room temperature overnight. A saturated aqueous sodium hydrogen carbonate solution was added to the reaction mixture, followed by extraction with chloroform.
The organic layer was washed with saturated brine and then dried over anhydrous sodium sulfate. The organic layer was concentrated under reduced pressure, and then the residue was purified by silica gel column chromatography (hexane:ethyl acetate = 80:20 to 70:30) to give the title compound (11.6 g) as a colorless oil.
ESI-MS Found: m/z 220[M+H]⁺

### Reference Example 5-2

### Synthesis of [4-(hydroxymethyl)phenyl](2,4,5-trifluorophenyl)methanone

To a THF solution (22.0 mL) of 4-bromobenzyl alcohol (2.05 g) was added a 1.00 M heptane solution (5.70 mL) of di-n-butylmagnesium at 0°C, and the mixture was stirred for 2 hour. The reaction mixture was cooled to -15°C. A 1.60 M hexane solution (6.30 mL) of n-butyllithium was added thereto, and stirred for 1 hour. A THF solution (8.00 mL) of the compound obtained in Reference Example 5-1 (2.00 g) was added to the reaction mixture at - 15°C, stirred for 2 hours and a half, and then stirred at 0°C for 4 hours. To the reaction mixture were added a 2.00 M aqueous hydrochloric acid solution and then a saturated aqueous sodium hydrogen carbonate solution, followed by extraction with ethyl acetate. The organic layer was washed with saturated brine and then dried over anhydrous sodium sulfate. The organic layer was concentrated under reduced pressure, and then the residue was purified by silica gel column chromatography (hexane:ethyl acetate = 85:15 to 50:50) to give a colorless oil containing the title compound (272 mg) as the main product.

### Reference Example 5-3

### Synthesis of [4-(1H,1'H-spiro[furo[3,4-c]pyridine-3,4'-piperidin]-1'-ylmethyl)phenyl](2,4,5-trifluorophenyl)methanone

The same operation as in Reference Example 1-4 was performed using the compound obtained in Reference Example 5-2 (272 mg), to give the title compound (151 mg) as a colorless oil.
ESI-MS Found: m/z 439[M+H]⁺

### Reference Example 5-4

### Synthesis of [4-(1H,1'H-spiro[furo[3,4-c]pyridine-3,4'-piperidin]-1'-ylmethyl)phenyl](2,4,5-trifluorophenyl)methanol

The same operation as in Reference Example 4-1 was performed using the compound obtained in Reference Example 5-3 (150 mg), to give the title compound (119 mg) as a white solid.
ESI-MS Found: m/z 441[M+H]⁺

### Reference Example 6: Synthesis of alkoxyamine (IIa)

### Reference Example 6-1

### Synthesis of 1'-{4-[chloro(3,4-difluorophenyl)methyl]benzyl}-1H-spiro[furo[3,4-c]pyridine-3,4'-piperidine]

To the compound (80.0 mg) obtained in Reference Example 4-1 was added thionyl chloride (2.00 mL) at 0°C, and the mixture was stirred for 30 minutes. The reaction mixture was concentrated under reduced pressure, followed by azeotropy with toluene. Ethyl acetate was added to the resulting residue, and saturated sodium hydrogen carbonate was then added under ice cooling. The mixed solution was extracted with ethyl acetate. The organic layer was washed with water and saturated brine, and then dried over anhydrous sodium sulfate. The organic layer was concentrated under reduced pressure to give the title compound (81.3 mg) as a pale brown oil.
ESI-MS Found: m/z 441, 443[M+H]⁺

### Reference Example 6-2

### Synthesis of 1'-({6-[chloro(3,4-difluorophenyl)methyl]pyridin-3-yl}methyl)-1H-spiro[furo[3,4-c]pyridine-3,4'-piperidine]

The same operation as in Reference Example 6-1 was performed using the compound obtained in Reference Example 4-2 (200 mg), to give the title compound (209 mg) as a pale yellow solid.
ESI-MS Found: m/z 442, 444[M+H]⁺

### Reference Example 6-3

### Synthesis of 1-(3,4-difluorophenyl)-N-methoxy-1-[4-(1H,1'H-spiro[furo[3,4-c]pyridine-3,4'-piperidin]-1'-ylmethyl)phenyl]methanamine

To an acetonitrile solution (1.00 mL) of o-methylhydroxyamine hydrochloride (119 mg) and N,N-diisopropylethylamine (616 µL) was added an acetonitrile solution (1.00 mL) of the compound obtained in Reference Example 6-1 (52.1 mg) at 0°C. The mixture was stirred at 90°C for 4 days, and further stirred at 100°C overnight. The reaction mixture was cooled, and a saturated aqueous sodium hydrogen carbonate solution was added thereto, followed by extraction with ethyl acetate. The organic layer was washed with saturated brine and then dried over anhydrous sodium sulfate.
The organic layer was concentrated under reduced pressure, and then the residue was purified by preparative thin-layer chromatography (chloroform:methanol = 90:10) to give the title compound (50.2 mg) as a colorless oil.
ESI-MS Found: m/z 452[M+H]⁺

### Reference Example 6-4

### Synthesis of N-{[tert-butyl(dimethyl)silyl]oxy}-1-(3,4-difluorophenyl)-1-[4-(1H,1'H-spiro [furo[3,4-c]pyridine-3,4'-piperidin]-1'-ylmethyl)phenyl]methanamine

The same operation as in Reference Example 6-3 was performed using the compound obtained in Reference Example 6-1 (81.3 mg) and (tert-butyl)(aminooxy)dimethylsilane, to give the title compound (18.6 mg) as a colorless oil.
ESI-MS Found: m/z 552[M+H]⁺

### Reference Example 6-5

### Synthesis of 1-[({(3,4-difluorophenyl)[4-(1H,1'H-spiro[furo[3,4-c]pyridine-3,4'-piperidin]-1'-ylmethyl)phenyl]methyl}amino)oxy]-2-methylpropan-2-ol

The same operation as in Reference Example 6-3 was performed using the compound obtained in Reference Example 6-1 (80.1 mg) and 1-(aminooxy)-2-methylpropan-2-ol hydrochloride, to give the title compound (31.1 mg) as a colorless oil.
ESI-MS Found: m/z 510[M+H]⁺

### Reference Example 6-6

### Synthesis of 1-[({(3,4-difluorophenyl)[5-(1H,1'H-spiro[furo[3,4-c]pyridine-3,4'-piperidin]-1'-ylmethyl)pyridin-2-yl]methyl}amino)oxy]-2-methylpropan-2-ol

The same operation as in Reference Example 6-5 was performed using the compound obtained in Reference Example 6-2 (105 mg), to give the title compound (8.40 mg) as a colorless oil.
ESI-MS Found: m/z 511[M+H]⁺

### Reference Example 7: Synthesis of amines (IIa), (IIb), and (IIc)

### Reference Example 7-1

### Synthesis of 2-bromo-5-({[tert-butyl(dimethyl)silyl]oxy}methyl)pyridine

To a DMF solution (20.0 mL) of (6-bromopyridin-3-yl)methanol (5.00 g) and imidazole (4.53 g) was added tert-butyldimethylsilyl chloride (4.41 g), and the mixture was stirred at room temperature for 2 hours. Water was added to the reaction mixture, followed by extraction with ethyl acetate. The organic layer was washed with water three times, and dried over anhydrous sodium sulfate. The organic layer was concentrated under reduced pressure, and then the residue was purified by silica gel column chromatography (hexane:ethyl acetate = 100:0 to 50:50) to give the title compound (7.71 g) as a colorless oil.
ESI-MS Found: m/z 301, 303[M]⁺

### Reference Example 7-2

### Synthesis of 5-({[tert-butyl(dimethyl)silyl]oxy}methyl)pyridine-2-carbaldehyde

To a THF solution (200 mL) of the compound obtained in Reference Example 7-1 (5.00 g) was slowly added a 2.64 M hexane solution (6.89 mL) of n-butyllithium at -78°C, and the mixture was stirred for 1 hour. DMF (1.41 mL) was slowly added thereto at -78°C, and stirred for 1 hour. Saturated brine was added to the reaction mixture, followed by extraction with ethyl acetate, and the organic layer was dried over anhydrous magnesium sulfate. The organic layer was concentrated under reduced pressure, and then the residue was purified by silica gel column chromatography (hexane:ethyl acetate = 100:0 to 50:50) to give the title compound (3.06 g) as a colorless oil.
ESI-MS Found: m/z 252[M+H]⁺

### Reference Example 7-3

### Synthesis of N-{[5-({[tert-butyl(dimethyl)silyl]oxy}methyl)pyridin-2-yl]methylene}-(R)-2-methylpropane-2-sulfinamide

To a THF solution (30.0 mL) of titanium tetraethoxide (1.65 mL) were added the compound obtained in Reference Example 7-2 (1.00 g) and (R)-(+)-2-methyl-2-propanesulfinamide (482 mg), and, in a nitrogen atmosphere, the mixture was heated under reflux for 3 hours. Saturated brine was added to the reaction mixture at 0°C, and the mixed solution was filtered through Celite. The filtrate was extracted with ethyl acetate. The organic layer was washed with saturated brine, and then dried over anhydrous magnesium sulfate. The organic layer was concentrated under reduced pressure, and then the residue was purified by silica gel column chromatography (hexane:ethyl acetate = 100:0 to 50:50) to give the title compound (849 mg) as a colorless oil.
ESI-MS Found: m/z 355[M+H]⁺

### Reference Example 7-4

### Synthesis of (R)- or (S)-N-[[5-({[tert-butyl(dimethyl)silyl]oxy}methyl)pyridin-2-yl](3,4-difluorophenyl)methyl-(R)-2-methylpropane-2-sulfinamide

To a toluene solution (4.00 mL) of the compound obtained in Reference Example 7-3 (200 mg) was added a 0.50 M THF solution (2.26 mL) of 3,4-difluorophenylmagnesium bromide at -40°C, and the mixture was stirred for 4 hour. An aqueous sodium hydrogen carbonate solution was added to the reaction mixture, followed by extraction with ethyl acetate, and the organic layer was dried over anhydrous sodium sulfate.
The organic layer was concentrated under reduced pressure, and then the residue was purified by silica gel column chromatography (hexane:ethyl acetate = 100:0 to 50:50) to give the title compound (259 mg) as a colorless oil.
ESI-MS Found: m/z 469[M+H]⁺

### Reference Example 7-5

### Synthesis of (R)- or (S)-N-{(3,4-difluorophenyl)[5-(hydroxymethyl)pyridin-2-yl]methyl}-(R)-2-methylpropane-2-sulfinamide

The same operation as in Reference Example 1-3 was performed using the compound obtained in Reference Example 7-4 (234 mg), to give the title compound (167 mg) as a colorless oil.
ESI-MS Found: m/z 355[M+H]⁺

### Reference Example 7-6

### Synthesis of (R)- or (S)-N-{(3,4-difluorophenyl)[5-(1H,1'H-spiro[furo[3,4-c]pyridine-3,4'-piperidin]-1'-ylmethyl)pyridin-2-yl]methyl}-(R)-2-methylpropane-2-sulfinamide

The same operation as in Reference Example 1-4 was performed using the compound obtained in Reference Example 7-5 (100 mg), to give the title compound (102 mg) as a white amorphous substance.
ESI-MS Found: m/z 527[M+H]⁺

### Reference Example 7-7

### Synthesis of (R)- or (S)-1-(3,4-difluorophenyl)-1-[5-(1H,1'H-spiro[furo[3,4-c]pyridine-3,4'-piperidin]-1'-ylmethyl)pyridin-2-yl]methanamine tetrahydrochloride

To the methanol solution (500 µL) of the compound obtained in Reference Example 7-6 (24.0 mg) was added a 2.00 M dioxane solution (500 µL) of hydrogen chloride at room temperature, and the mixture was stirred for 1 hour. The solvent was concentrated under reduced pressure, and then the residue was crystallized from methanol/ethyl acetate to give the title compound (14.4 mg) as a white solid.
ESI-MS Found: m/z 423[M+H]⁺

### Reference Example 7-8

### Synthesis of N-[[5-({[tert-butyl(dimethyl)silyl]oxy}methyl)pyridin-2-yl](3,4-difluorophenyl)methylene]-(R)-2-methylpropane-2-sulfinamide

The same operation as in Reference Example 7-3 was performed using [5-({[tert-butyl(dimethyl)silyl]oxy}methyl)pyridin-2-yl](3,4-difluorophenyl)methanone (1.00 g), to give the title compound (1.39 g) as a yellow oil.
ESI-MS Found: m/z 467[M+H]

### Reference Example 7-9

### Synthesis of (R)- or (S)-N-[1-[5-({[tert-butyl(dimethyl)silyl]oxy}methyl)pyridin-2-yl]-1-(3,4-difluorophenyl)ethyl]-(R)-2-methylpropane-2-sulfinamide

To a THF solution (15.0 mL) of the compound obtained in Reference Example 7-8 (1.39 g) was added a 3.00 M THF solution (2.99 mL) of methylmagnesium bromide at -78°C, and the mixture was stirred for 1 hour. An aqueous sodium hydrogen carbonate solution was added to the reaction mixture, followed by extraction with ethyl acetate, and the organic layer was dried over anhydrous sodium sulfate. The organic layer was concentrated under reduced pressure, and then the residue was purified by silica gel column chromatography (hexane:ethyl acetate = 100:0 to 50:50) to give the title compound (259 mg) as a colorless oil.
ESI-MS Found: m/z 483[M+H]

### Reference Example 7-10

### Synthesis of (R)- or (S)-N-{1-(3,4-difluorophenyl)-1-[5-(hydroxymethyl)pyridin-2-yl]ethyl}-(R)-2-methylpropane-2-sulfinamide

The same operation as in Reference Example 1-3 was performed using the compound obtained in Reference Example 7-9 (447 mg), to give the title compound (316 mg) as a yellow oil.
ESI-MS Found: m/z 369[M+H]

### Reference Example 7-11

### Synthesis of (R)- or (S)-N-{1-(3,4-difluorophenyl)-1-[5-(1H,1'H-spiro[furo[3,4-c]pyridine-3,4'-piperidin]-1'-ylmethyl)pyridin-2-yl]ethyl}-(R)-2-methylpropane-2-sulfinamide

The same operation as in Reference Example 1-4 was performed using the compound obtained in Reference Example 7-10 (284 mg), to give the title compound (334 mg) as a yellow oil.
ESI-MS Found: m/z 541 [M+H]

### Reference Example 7-12

### Synthesis of (R)- or (S)-1-(3,4-difluorophenyl)-1-[5-(1H,1'H-spiro[furo[3,4-c]pyridine-3,4'-piperidin]-1'-ylmethyl)pyridin-2-yl]ethanamine

The same operation as in Reference Example 7-7 was performed using the compound obtained in Reference Example 7-11 (334 mg). The reaction mixture was concentrated under reduced pressure, then ethyl acetate was added to the residue, and a saturated aqueous sodium hydrogen carbonate solution was added thereto under ice cooling. The mixed solution was extracted with ethyl acetate. The organic layer was washed with saturated brine and dried over anhydrous magnesium sulfate. The organic layer was concentrated under reduced pressure, and then the residue was purified by silica gel column chromatography (chloroform:methanol = 100:0 to 80:20) to give the title compound (138 mg) as a yellow oil. ESI-MS Found: m/z 437[M+H]

### Reference Example 7-13

### Synthesis of (R)- or (S)-1'-({6-[1-amino-1-(3,4-difluorophenyl)ethyl]pyridin-3-yl}methyl)-5-methyl-3,5-dihydro-6H-spiro[furo[3,4-c]pyridine-1,4'-piperidin]-6-one

The same operation as in Reference Examples 1-8 and 7-12 was performed using the compound obtained in Reference Example 7-10 (43.8 mg) and tert-butyl 5-methyl-6-oxo-5,6-dihydro-1'H,3H-spiro[furo[3,4-c]pyridine-1,4'-piperidine]-1'-carboxylate (57.1 mg), to give the title compound (26.1 mg) as a yellow oil.
ESI-MS Found: m/z 467[M+H]

### Reference Example 7-14

### Synthesis of 4-chloro-3,5-difluoro-N-methoxy-N-methylbenzamide

The same operation as in Reference Example 5-1 was performed using 4-chloro-3,5-difluorobenzoic acid (10.0 g), to give the title compound (11.6 g) as a white solid.
ESI-MS Found: m/z 236, 238[M+H]⁺

### Reference Example 7-15

### Synthesis of [5-({[tert-butyl(dimethyl)silyl]oxy}methyl)pyridin-2-yl](4-chloro-3,5-difluorophenyl)methanone

To a THF solution (50.0 mL) of the compound obtained in Reference Example 7-1 (4.80 g) was slowly added a 2.50 M hexane solution (5.70 mL) of n-butyllithium in a nitrogen atmosphere at -78°C, and the mixture was stirred at -78°C for 1 hour. A THF solution (50.0 mL) of the compound obtained in Reference Example 7-14 (3.80 g) was added to the reaction mixture at -78°C, then heated to room temperature, and stirred for 30 minutes. A saturated aqueous ammonium chloride solution was added to the reaction mixture, followed by extraction with ethyl acetate. The organic layer was washed with saturated brine and then dried over anhydrous sodium sulfate. The organic layer was concentrated under reduced pressure, and then the residue was purified by silica gel column chromatography (hexane:ethyl acetate = 97:3) to give the title compound (3.20 g) as a colorless oil.
ESI-MS Found: m/z 398, 400[M+H]⁺

### Reference Example 7-16

### Synthesis of N-[[5-({[tert-butyl(dimethyl)silyl]oxy}methyl)pyridin-2-yl](4-chloro-3,5-difluorophenyl)methylene]-(R)-2-methylpropane-2-sulfinamide

The same operation as in Reference Example 7-3 was performed using the compound obtained in Reference Example 7-15 (3.20 g), to give the title compound (2.10 g) as a yellow oil.
ESI-MS Found: m/z 501, 503[M+H]⁺

### Reference Example 7-17

### Synthesis of (R)- or (S)-N-[1-[5-({[tert-butyl(dimethyl)silyl]oxy}methyl)pyridin-2-yl]-1-(4-chloro-3,5-difluorophenyl)ethyl]-(R)-2-methylpropane-2-sulfinamide

The same operation as in Reference Example 7-9 was performed using the compound obtained in Reference Example 7-16 (500 mg), to give the title compound (400 mg) as a colorless oil.
ESI-MS Found: m/z 517, 519[M+H]⁺

### Reference Example 7-18

### Synthesis of (R)- or (S)-N-{1-(4-chloro-3,5-difluorophenyl)-1-[5-(hydroxymethyl)pyridin-2-yl]ethyl}-(R)-2-methylpropane-2-sulfinamide

The same operation as in Reference Example 1-3 was performed using the compound obtained in Reference Example 7-17 (200 mg), to give the title compound (150 mg) as a colorless oil.
ESI-MS Found: m/z 403, 405[M+H]⁺

### Reference Example 7-19

### Synthesis of (R)- or (S)-N-{1-(4-chloro-3,5-difluorophenyl)-1-[5-(1H,1'H-spiro[furo[3,4-c]pyridine-3,4'-piperidin]-1'-ylmethyl)pyridin-2-yl]ethyl}-(R)-2-methylpropane-2-sulfinamide

The same operation as in Reference Example 1-4 was performed using the compound obtained in Reference Example 7-18 (200 mg), to give the title compound (150 mg) as a colorless oil.
ESI-MS Found: m/z 575, 577[M+H]⁺

### Reference Example 7-20

### Synthesis of (R)- or (S)-1-(4-chloro-3,5-difluorophenyl)-1-[5-(1H,1'H-spiro[furo[3,4-c]pyridine-3,4'-piperidin]-1'-ylmethyl)pyridin-2-yl]ethanamine

The same operation as in Reference Example 7-12 was performed using the compound obtained in Reference Example 7-19 (30.0 mg), to give the title compound (20.0 mg) as a colorless oil.
ESI-MS Found: m/z 471, 473[M+H]⁺

### Reference Example 7-21

### Synthesis of (R)- or (S)-N-[1-[5-({[tert-butyl(dimethyl)silyl]oxy}methyl)pyridin-2-yl]-1-(3,4-difluorophenyl)-2,2-difluoro-2-(phenylsulfonyl)-ethyl]-(R)-2-methylpropane-2-sulfinamide

To a THF solution (10.0 mL) of the compound obtained in Reference Example 7-8 (200 mg) and difluoromethyl phenyl sulfone (77.0 mg) was added a 1.00 M THF solution (0.60 mL) of lithium bis(trimethylsilyl)amide in a nitrogen atmosphere at -78°C. The mixture was stirred for 1 hour, and then the temperature was slowly raised to -30°C. Saturated brine was added to the reaction mixture, followed by extraction with ethyl acetate. The organic layer was washed with saturated brine and then dried over anhydrous sodium sulfate. The organic layer was concentrated under reduced pressure, and then the residue was purified by silica gel column chromatography (hexane:ethyl acetate = 83:17) to give the title compound (200 mg) as a colorless oil.
ESI-MS Found: m/z 659[M+H]⁺

### Reference Example 7-22

### Synthesis of (R)- or (S)-N-[1-[5-({[tert-butyl(dimethyl)silyl]oxy}methyl)pyridin-2-yl]-1-(3,4-difluorophenyl)-2,2-difluoroethyl]-(R)-2-methylpropane-2-sulfinamide

To a DMF solution (7.00 mL) of the compound obtained in Reference Example 7-21 (200 mg) was added a buffer (3.00 mL) of 8.00 M acetic acid:sodium acetate (1:1) at room temperature. Magnesium (720 mg) was gradually added to the reaction mixture, and stirred at room temperature overnight. The reaction mixture was added dropwise to ice water, followed by extraction with diethylether. The organic layer was washed with a saturated aqueous sodium hydrogen carbonate solution, water, and saturated brine, and then dried over anhydrous sodium sulfate. The organic layer was concentrated under reduced pressure, and then the residue was purified by silica gel column chromatography (hexane:ethyl acetate = 67:33) to give the title compound (100 mg) as a colorless oil.
ESI-MS Found: m/z 519[M+H]⁺

### Reference Example 7-23

### Synthesis of (R)- or (S)-N-{1-(3,4-difluorophenyl)-2,2-difluoro-1-[5-(hydroxymethyl)pyridin-2-yl]ethyl}-(R)-2-methylpropane-2-sulfinamide

The same operation as in Reference Example 1-3 was performed using the compound obtained in Reference Example 7-22 (450 mg), to give the title compound (380 mg) as a colorless oil.
ESI-MS Found: m/z 405[M+H]⁺

### Reference Example 7-24

### Synthesis of (R)- or (S)-N-{1-(3,4-difluorophenyl)-2,2-difluoro-1-[5-(1H,1'H-spiro[furo[3,4-c]pyridine-3,4'-piperidin]-1'-ylmethyl)pyridin-2-yl]ethyl}-(R)-2-methylpropane-2-sulfinamide

The same operation as in Reference Example 1-4 was performed using the compound obtained in Reference Example 7-23 (380 mg), to give the title compound (190 mg) as a colorless oil.
ESI-MS Found: m/z 577[M+H]⁺

### Reference Example 7-25

### Synthesis of (R)- or (S)-1-(3,4-difluorophenyl)-2,2-difluoro-1-[5-(1H,1'H-spiro[furo[3,4-c]pyridine-3,4'-piperidin]-1'-ylmethyl)pyridin-2-yl]ethanamine

The same operation as in Reference Example 7-12 was performed using the compound obtained in Reference Example 7-24 (180 mg), to give the title compound (120 mg) as a colorless oil.
ESI-MS Found: m/z 473[M+H]⁺

### Reference Example 7-26

### Synthesis of N-[1-[5-({[tert-butyl(dimethyl)silyl]oxy}methyl)pyridin-2-yl]-1-(3,4-difluorophenyl)-2,2,2-trifluoroethyl]-(R)-2-methylpropane-2-sulfinamide

To a THF solution (20.0 mL) of the compound obtained in Reference Example 7-8 (500 mg) and trifluoromethyltrimethylsilane (450 mg) was added a THF solution (20.0 mL) of tetra-n-butylammonium difluorotriphenylsilicate (1.10 g) in a nitrogen atmosphere at -78°C. The mixture was stirred for 1 hour, and then the temperature was slowly raised to -30°C. Saturated brine was added to the reaction mixture, followed by extraction with ethyl acetate. The organic layer was washed with saturated brine and then dried over anhydrous sodium sulfate. The organic layer was concentrated under reduced pressure to give a diastereomer mixture of the title compound (550 mg, 1:2) as a colorless oil.
ESI-MS Found: m/z 537[M+H]⁺

### Reference Example 7-27

### Synthesis of N-{1-(3,4-difluorophenyl)-2,2,2-trifluoro-1-[5-(hydroxymethyl)pyridin-2-yl]ethyl}-(R)-2-methylpropane-2-sulfinamide

The same operation as in Reference Example 1-3 was performed using the diastereomer mixture (550 mg, 1:2) obtained in Reference Example 7-26, to give a diastereomer mixture of the title compound (220 mg, 1:2) as a colorless oil.
ESI-MS Found: m/z 423[M+H]⁺

### Reference Example 7-28

### Synthesis of (R) or (S)-N-{1-(3,4-difluorophenyl)-2,2,2-trifluoro-1-[5-(1H,1'H-spiro[furo[3,4-c]pyridine-3,4'-piperidin]-1'-ylmethyl)pyridin-2-yl]ethyl}-(R)-2-methylpropane-2-sulfinamide

The same operation as in Reference Example 1-4 was performed using the diastereomer mixture (900 mg, 1:2) obtained in Reference Example 7-27, to give a crude product containing the title compound. The product was purified by reversed-phase high-performance liquid chromatography (YMC-ODS, 0.1% TFA-acetonitrile:0.1% TFA-water = 30:70 to 60:40) to give the title compound (80.0 mg, faster) as a colorless oil.
ESI-MS Found: m/z 595[M+H]⁺

### Reference Example 7-29

### Synthesis of (R)- or (S)-1-(3,4-difluorophenyl)-2,2,2-trifluoro-1-[5-(1H,1'H-spiro[furo[3,4-c]pyridine-3,4'-piperidin]-1'-ylmethyl)pyridin-2-yl]ethanamine

The same operation as in Reference Example 7-12 was performed using the compound obtained in Reference Example 7-28 (80.0 mg), to give the title compound (50.0 mg) as a colorless oil.
ESI-MS Found: m/z 491[M+H]⁺

### Reference Example 7-30

### Synthesis of (R)- or (S)-N-[1-[5-({[tert-butyl(dimethyl)silyl]oxy}methyl)pyridin-2-yl]-1-(3,4-difluorophenyl)ethyl]-N-methyl-(R)-2-methylpropane-2-sulfinamide

To a DMF solution (15.0 mL) of the compound obtained in Reference Example 7-9 (590 mg), molecular sieve 4A (5.90 g), and methyl iodide (382 µL) was added sodium hydride (58.7 mg) in a nitrogen atmosphere at 0°C, and the mixture was stirred at room temperature for 2 hours. Chloroform and water were added to the reaction mixture at 0°C, the mixed solution was filtered through Celite, and the filtrate was extracted with chloroform. The organic layer was washed with water and then dried over anhydrous magnesium sulfate. The organic layer was concentrated under reduced pressure, and then the residue was purified by silica gel column chromatography (hexane:ethyl acetate = 100:0 to 50:50) to give the title compound (504 mg) as a colorless oil.
ESI-MS Found: m/z 497[M+H]

### Reference Example 7-31

### Synthesis of (R)- or (S)-N-{1-(3,4-difluorophenyl)-1-[5-(hydroxymethyl)pyridin-2-yl]ethyl}-N-methyl-(R)-2-methylpropane-2-sulfinamide

The same operation as in Reference Example 1-3 was performed using the compound obtained in Reference Example 7-30 (504 mg), to give the title compound (333 mg) as a yellow oil.
ESI-MS Found: m/z 383[M+H]

### Reference Example 7-32

### Synthesis of (R)- or (S)-1-(3,4-difluorophenyl)-N-methyl-1-[5-(1H,1'H-spiro[furo[3,4-c]pyridine-3,4'-piperidin]-1'-ylmethyl)pyridin-2-yl]ethanamine

The same operation as in Reference Examples 1-4 and 7-12 was performed using the compound obtained in Reference Example 7-31 (165 mg), to give the title compound (104 mg) as a yellow oil.
ESI-MS Found: m/z 45 1 [M+H]

### Reference Example 7-33

### Synthesis of N-{(3,4-difluorophenyl)[5-(1H,1'H-spiro[furo[3,4-c]pyridine-3,4'-piperidin]-1'-ylmethyl)pyridin-2-yl]methylene}-(R)-2-methylpropane-2-sulfinamide

The same operation as in Reference Example 7-3 was performed using (3,4-difluorophenyl)[5-(1H,1'H-spiro[furo[3,4-c]pyridine-3,4'-piperidin]-1'-ylmethyl)pyridin-2-yl]methanone (2.00 g), to give the title compound (1.78 g) as a yellow oil.
ESI-MS Found: m/z 525[M+H]

### Reference Example 7-34

### Synthesis of (R)- or (S)-N-{1-(3,4-difluorophenyl)-1-[5-(1H,1'H-spiro[furo[3,4-c]pyridine-3,4'-piperidin]-1'-ylmethyl)pyridin-2-yl]propyl}-(R)-2-methylpropane-2-sulfinamide

The same operation as in Reference Example 7-9 was performed using the compound obtained in Reference Example 7-33 (379 mg) and a 3.00 M diethylether solution (722 µL) of ethylmagnesium bromide, to give the title compound (120 mg) as a yellow oil.
ESI-MS Found: m/z 555[M+H]

### Reference Example 7-35

### Synthesis of (R)- or (S)-1-(3,4-difluorophenyl)-1-[5-(1H,1'H-spiro[furo[3,4-c]pyridine-3,4'-piperidin]-1'-ylmethyl)pyridin-2-yl]propan-1-amine

The same operation as in Reference Example 7-12 was performed using the compound obtained in Reference Example 7-34 (30.0 mg), to give the title compound (31.7 mg) as a colorless oil.
ESI-MS Found: m/z 45 1 [M+H]

### Reference Example 7-36

### Synthesis of (R)- or (S)-1-cyclpropyl-1-(3,4-difluorophenyl)-1-[5-(1H,1'H-spiro[furo[3,4-c]pyridine-3,4'-piperidin]-1'-ylmethyl)pyridin-2-yl]methanamine

The same operation as in Reference Examples 7-9 and 7-12 was performed using the compound obtained in Reference Example 7-33 (100 mg) and a 0.50 M THF solution (381 µL) of cyclopropylmagnesium bromide, to give the title compound (51.2 mg) as a yellow oil. ESI-MS Found: m/z 463[M+H]

### Reference Example 7-37

### Synthesis of N-[(3,4-difluorophenyl)(5-{[4-(6-fluoropyridin-3-yl)piperidin-1-yl]methyl}pyridin-2-yl)methylene]-(R)-2-methylpropane-2-sulfinamide

The same operation as in Reference Example 7-3 was performed using (3,4-difluorophenyl)(5-{[4-(6-fluoropyridin-3-yl)piperidin-1-yl]methyl}pyridin-2-yl)methanone (1.07 g), to give the title compound (1.07 g) as a yellow oil.
ESI-MS Found: m/z 515[M+H]

### Reference Example 7-38

### Synthesis of (R)- or (S)-N-[1-(3,4-difluorophenyl)-1-(5-{[4-(6-fluoropyridin-3-yl)piperidin-1-yl]methyl}pyridin-2-yl)ethyl]-(R)-2-methylpropane-2-sulfinamide

The same operation as in Reference Example 7-9 was performed using the compound obtained in Reference Example 7-37 (1.07 g), to give the title compound (758 mg) as a yellow oil.
ESI-MS Found: m/z 531 [M+H]

### Reference Example 7-39

### Synthesis of (R)- or (S)-1-(3,4-difluorophenyl)-1-(5-{[4-(6-fluoropyridin-3-yl)piperidin-1-yl]methyl}pyridin-2-yl)ethanamine

The same operation as in Reference Example 7-12 was performed using the compound obtained in Reference Example 7-38 (758 mg), to give the title compound (466 mg) as a colorless oil.
ESI-MS Found: m/z 427[M+H]

### Reference Example 8: Synthesis of benzyl alcohol (Va)

### Reference Example 8-1

### Synthesis of (3,4-difluorophenyl)[4-(hydroxymethyl)phenyl]methanol

The same operation as in Reference Example 4-1 was performed using (3,4-difluorophenyl)[4-(hydroxymethyl)phenyl]methanone (2.52 g), to give the title compound (2.08 g) as a colorless oil.
ESI-MS Found: m/z 251[M+H]⁺

### Reference Example 8-2

### Synthesis of 1-{(3,4-difluorophenyl)[4-(hydroxymethyl)phenyl]methyl}pyrrolidin-2-one

To an acetic acid solution (2.00 mL) of the compound obtained in Reference Example 8-1 (200 mg) were added 2-pyrrolidone (182 µL) and concentrated sulfuric acid (200 µL) at room temperature, and the mixture was stirred at 130°C overnight. The reaction mixture was concentrated under reduced pressure, the residue was diluted with ethyl acetate, and then a 5.00 M aqueous sodium hydroxide solution was added thereto to make it alkaline. The mixed solution was extracted with ethyl acetate, and the organic layer was dried over anhydrous sodium sulfate. The organic layer was concentrated under reduced pressure to give a crude product of 4-[(3,4-difluorophenyl)(2-oxopyrrolidin-1-yl)methyl]benzyl acetate as a yellow oil. A 5.00 M aqueous sodium hydroxide solution (200 µL) was added to a methanol solution (2.00 mL) of the obtained crude product at room temperature, and stirred for 30 minutes. The reaction mixture was diluted with water, followed by extraction with ethyl acetate, and the organic layer was dried over anhydrous sodium sulfate. The organic layer was concentrated under reduced pressure, and then the residue was purified by silica gel column chromatography (hexane:ethyl acetate = 100:0 to 0:100) to give the title compound (105 mg) as a colorless oil.
ESI-MS Found: m/z 318[M+H]⁺

### Reference Example 8-3

### Synthesis of 1-[5-({[tert-butyl(dimethyl)silyl]oxy}methyl)pyridin-2-yl]-1-(3,4-difluorophenyl)-N-methylmethanamine

The same operation as in Reference Example 3-1 was performed using [5-({[tert-butyl(dimethyl)silyl]oxy}methyl)pyridin-2-yl](3,4-difluorophenyl)methanone (500 mg), to give the title compound (408 mg) as a colorless oil.
ESI-MS Found: m/z 379[M+H]⁺

### Reference Example 8-4

### Synthesis of 2-[[[5-({[tert-butyl(dimethyl)silyl]oxy}methyl)pyridin-2-yl](3,4-difluorophenyl)methyl](methyl)amino]-1,1-dimethyl-2-oxoethyl acetate

To a chloroform solution (20.0 mL) of the compound obtained in Reference Example 8-3 (391 mg) was added triethylamine (432 µL). Subsequently, 2-chloro-1,1-dimethyl-2-oxoethyl acetate (224 µL) was added thereto in a nitrogen atmosphere at 0°C, and stirred at room temperature overnight. An aqueous sodium hydrogen carbonate solution was added to the reaction mixture, followed by extraction with chloroform. The organic layer was washed with water and saturated brine, and then dried over anhydrous sodium sulfate. The organic layer was concentrated under reduced pressure, and then the residue was purified by silica gel column chromatography (hexane:ethyl acetate = 100:0 to 0:100) to give the title compound (526 mg) as a colorless oil.
ESI-MS Found: m/z 507[M+H]⁺

### Reference Example 8-5

### Synthesis of 2-[{(3,4-difluorophenyl)[5-(hydroxymethyl)pyridin-2-yl]methyl}(methyl)amino]-1,1-dimethyl-2-oxoethyl acetate

The same operation as in Reference Example 1-3 was performed using the compound obtained in Reference Example 8-4 (526 mg), to give the title compound (317 mg) as a colorless oil.
ESI-MS Found: m/z 393[M+H]⁺

### Reference Example 9: Synthesis of 3-fluoropyrrolidin-2-one

### Reference Example 9-1

### Synthesis of 1-benzoylpyrrolidin-2-one

To a THF solution (100 mL) of 2-pyrrolidone (5.00 g) were added triethylamine (24.6 mL) and then benzoyl chloride (7.50 mL), and the mixture was stirred at room temperature for 1 hours. Water was added to the reaction mixture, followed by extraction with ethyl acetate, and the organic layer was dried over anhydrous magnesium sulfate. The organic layer was concentrated under reduced pressure. The residue was then purified by silica gel column chromatography (hexane:ethyl acetate = 10:90 to 30:70), and further crystallized from ethyl acetate/hexane to give the title compound (5.70 g) as a white solid.
ESI-MS Found: m/z 190[M+H]⁺

### Reference Example 9-2

### Synthesis of 1-benzoyl-3-fluoropyrrolidin-2-one

To a THF solution (10.0 mL) of diisopropylamine (4.07 mL) was slowly added a 2.64 M hexane solution (9.61 mL) of n-butyllithium at -78°C, and the mixture was stirred for 30 minutes. A THF solution (10.0 mL) of the compound obtained in Reference Example 9-1 (3.00 g) was slowly added thereto at -78°C, and stirred for 30 minutes. A THF solution (10.0 mL) of N-fluorobenzenesulfonimide (7.50 g) was then slowly added at -78°C, and stirred at -40°C for 1 hour. An aqueous sodium hydrogen carbonate solution was added to the reaction mixture, followed by extraction with ethyl acetate, and the organic layer was dried over anhydrous magnesium sulfate. The organic layer was concentrated under reduced pressure. The residue was then purified by silica gel column chromatography (hexane:ethyl acetate = 90:10 to 40:60), and further crystallized from ethyl acetate/hexane to give the title compound (1.17 g) as a white solid. ESI-MS Found: m/z 208[M+H]⁺

### Reference Example 9-3

### Synthesis of 3-fluoropyrrolidin-2-one

To a THF solution (2.00 mL) of the compound obtained in Reference Example 9-2 (100 mg) was added n-octylamine (88.0 µL), and the mixture was stirred at room temperature for 8 hours. The reaction mixture was concentrated under reduced pressure, and then the residue was purified by silica gel column chromatography (hexane:ethyl acetate = 100:0 to 0:100) to give the title compound (43.0 mg) as a white solid.

### Example 1-1

### Synthesis of N-{(3,4-difluorophenyl)[4-(1H,1'H-spiro[furo[3,4-c]pyridine-3,4'-piperidin]-1'-ylmethyl)phenyl]methyl}acetamide

To a methylene chloride solution (1.00 mL) of the compound obtained in Reference Example 1-5 (40.0 mg) was added triethylamine (105 µL). Subsequently, acetyl chloride (5.90 µL) was added thereto at 0°C, and the mixture was stirred at room temperature for 30 minutes. An aqueous sodium hydrogen carbonate solution was added to the reaction mixture, followed by extraction with chloroform. The organic layer was washed with water and saturated brine, and then dried over anhydrous sodium sulfate. The organic layer was concentrated under reduced pressure, and then the residue was purified by preparative thin-layer chromatography (chloroform:methanol = 90:10) to give the title compound(15.6 mg) as a white amorphous substance.
¹HNMR (400 MHz, CDCl₃, δppm): 1.76-1.83 (2H, m), 1.98-2.07 (2H, m), 2.08 (3H, s), 2.39-2.49 (2H, m), 2.80-2.87 (2H, m), 3.58 (2H, s), 5.05 (2H, s), 6.06 (1H, d, J = 7.5 Hz), 6.18 (1H, d, J = 7.5 Hz), 6.95-7.00 (1H, m), 7.05 (1H, ddd, J = 11.1, 7.7, 2.4 Hz), 7.08-7.13 (1H, m), 7.16 (2H, d, J = 8.0 Hz), 7.18 (1H, d, J = 5.1 Hz), 7.35 (2H, d, J = 8.0 Hz), 8.43 (1H, s), 8.50 (1H, d, J = 5.1 Hz).
ESI-MS Found: m/z 464[M+H]⁺

### Example 1-2

### Synthesis of N-{(3,4-difluorophenyl)[4-(1H,1'H-spiro[furo[3,4-c]pyridine-3,4'-piperidin]-1'-ylmethyl)phenyl]methyl}-N-methylacetamide

The same operation as in Example 1-1 was performed using the compound obtained in Reference Example 3-1 (104 mg), to give the title compound (58.7 mg) as a white amorphous substance.
¹HNMR (400 MHz, CDCl₃, δppm): 1.77-1.84 (2H, m), 2.00-2.09 (2H, m), 2.21 (2H, s), 2.23 (1H, s), 2.44-2.47 (2H, m), 2.72 (1H, s), 2.81 (2H, s), 2.82-2.89 (2H, m), 3.59 (2H, s), 5.06 (2H, s), 6.90-6.96 (1H, m), 6.97-7.04 (1H, m), 7.08-7.20 (5H, m), 7.34-7.41 (2H, m), 8.46 (1H, s), 8.51 (1H, d, J = 4.9 Hz).
ESI-MS Found: m/z 478[M+H]⁺

### Example 1-3

### Synthesis of N-{(3,4-difluorophenyl)[5-(1H,1'H-spiro[furo[3,4-c]pyridine-3,4'-piperidin]-1'-ylmethyl)pyridin-2-yl]methyl}-N-(2-hydroxy-2-methylpropoxy)acetamide

The same operation as in Example 1-1 was performed using the compound obtained in Reference Example 6-6 (8.40 mg), acetic anhydride, and pyridine, to give the title compound (2.90 mg) as a colorless oil.
¹HNMR (400 MHz, CDCl₃, δppm): 1.07 (3H, s), 1.08 (3H, s), 1.78-1.83 (2H, m), 1.98-2.07 (2H, m), 2.25 (3H, s), 2.45-2.53 (2H, m), 2.78-2.85 (2H, m), 3.30-3.40 (2H, m), 3.62 (2H, s), 5.06 (2H, s), 6.69 (1H, br s), 7.10-7.30 (5H, m), 7.75 (1H, dd, J = 6.4, 1.6 Hz), 8.45 (1H, s), 8.51 (1H, d, J = 4.8 Hz), 8.59 (1H, d, J = 1.6 Hz).
ESI-MS Found: m/z 553[M+H]⁺

### Example 1-4

### Synthesis of N-{(3,4-difluorophenyl)[4-(1H,1'H-spiro[furo[3,4-c]pyridine-3,4'-piperidin]-1'-ylmethyl)phenyl]methyl}-N-hydroxyacetamide

The same operation as in Example 1-3 was performed using the compound obtained in Reference Example 6-4 (18.6 mg), to give N-{[tert-butyl(dimethyl)silyl]oxy}-N-{(3,4-difluorophenyl)[4-(1H,1'H-spiro[furo[3,4-c]pyridine-3,4'-piperidin]-1'-ylmethyl)phenyl]methyl}acetamide. Further, the same operation as in Reference Example 1-3 was performed to give the title compound (8.00 mg) as a colorless oil.
¹HNMR (400 MHz, CDCl₃, δppm): 1.80-1.87 (2H, m), 1.89-1.98 (2H, m), 2.17 (3H, s), 2.45-2.55 (2H, m), 2.78-2.85 (2H, m), 3.60 (2H, s), 5.05 (2H, s), 6.92-7.01 (1H, m), 7.03-7.09 (1H, m), 7.10-7.22 (4H, m), 7.23-7.32 (4H, m), 8.38 (1H, d, J = 4.8 Hz).
ESI-MS Found: m/z 480[M+H]⁺

### Example 1-5

### Synthesis of N-{(3,4-difluorophenyl)[5-(1H,1'H-spiro[furo[3,4-c]pyridine-3,4'-piperidin]-1'-ylmethyl)pyridin-2-yl]methyl}-N-(2-hydroxy-2-methylpropyl)-2-methylpropanamide

The same operation as in Example 1-3 was performed under heating conditions using the compound obtained in Reference Example 3-7 (37.8 mg) and isobutyric anhydride, to give the title compound (9.2 mg) as a colorless oil.
¹HNMR (400 MHz, CDCl₃, δppm): 0.89 (3/5H, d, J = 6.8 Hz), 1.01 (12/5H, d, J = 6.8 Hz), 1.08 (3/5H, d, J = 6.8 Hz), 1.16 (12/5H, d, J = 6.8 Hz), 1.28 (3H, s), 1.30 (3H, s), 1.78-1.83 (2H, m), 1.95-2.07 (2H, m), 2.40-2.53 (2H, m), 2.78-2.90 (3H, m), 3.34 (1H, d, J = 15.2 Hz), 3.55-3.65 (3H, m), 5.06 (2H, s), 6.11 (4/5H, s), 6.49 (1/5H, s), 6.90-7.30 (5H, m), 7.65 (4/5H, d, J = 8.0 Hz), 7.78 (1/5H, d, J = 8.0 Hz), 8.43-8.45 (4/5H, s), 8.46 (1H, s), 8.51 (1H, d, J = 4.8 Hz), 8.60-8.62 (1/5H, m).
ESI-MS Found: m/z 565[M+H]⁺

### Example 1-6

### Synthesis of N-{(3,4-difluorophenyl)[4-(1H,1'H-spiro[furo[3,4-c]pyridine-3,4'-piperidin]-1'-ylmethyl)phenyl]methyl}-2,2-dimethylpropanamide

The same operation as in Example 1-1 was performed using the compound obtained in Reference Example 1-5 (20.0 mg) and trimethylacetic anhydride, to give the title compound (17.5 mg) as a colorless oil.
¹HNMR (400 MHz, CDCl₃, δppm): 1.25 (9H, s), 1.78-1.83 (2H, m), 1.99-2.09 (2H, m), 2.40-2.49 (2H, m), 2.81-2.89 (2H, m), 3.58 (2H, s), 5.06 (2H, s), 6.10-6.14 (2H, m), 6.93-7.05 (2H, m), 7.09-7.15 (1H, m), 7.13 (2H, d, J = 7.6 Hz), 7.18 (1H, d, J = 4.8 Hz), 7.35 (2H, d, J = 7.6 Hz), 8.45 (1H, s), 8.51 (1H, d, J = 4.8 Hz).
ESI-MS Found: m/z 506[M+H]⁺

### Example 1-7

### Synthesis of N-{(3,4-difluorophenyl)[4-(1H,1'H-spiro[furo[3,4-c]pyridine-3,4'-piperidin]-1'-ylmethyl)phenyl]methyl}benzamide

The same operation as in Example 1-1 was performed using the compound obtained in Reference Example 1-5 (40.0 mg) and benzoyl chloride, to give the title compound (39.6 mg) as a white amorphous substance.
¹HNMR (400 MHz, CDCl₃, δppm): 1.76-1.83 (2H, m), 1.98-2.08 (2H, m), 2.40-2.49 (2H, m), 2.81-2.88 (2H, m), 3.59 (2H, s), 5.05 (2H, s), 6.38 (1H, d, J = 7.6 Hz), 6.65 (1H, d, J = 7.6 Hz), 7.03-7.19 (4H, m), 7.23 (2H, d, J = 7.7 Hz), 7.38 (2H, d, J = 7.7 Hz), 7.42-7.48 (2H, m), 7.49-7.56 (1H, m), 7.80-7.84 (2H, m), 8.44 (1H, s), 8.48-8.51 (1H, m).
ESI-MS Found: m/z 526[M+H]⁺

### Example 1-8

### Synthesis of N-{(3,4-difluorophenyl)[4-(1H,1'H-spiro[furo[3,4-c]pyridine-3,4'-piperidin]-1'-ylmethyl)phenyl]methyl}pyridine-2-carboxamide

The same operation as in Example 1-1 was performed using the compound obtained in Reference Example 1-5 (20.0 mg) and pyridine-2-carbonyl chloride, to give the title compound (27.5 mg) as a white amorphous substance.
¹HNMR (400 MHz, CDCl₃, δppm): 1.76-1.83 (2H, m), 1.97-2.07 (2H, m), 2.39-2.48 (2H, m), 2.81-2.88 (2H, m), 3.58 (2H, s), 5.06 (2H, s), 6.37 (1H, d, J = 8.3 Hz), 7.05-7.20 (4H, m), 7.27 (2H, d, J = 8.3 Hz), 7.37 (2H, d, J = 8.3 Hz), 7.44-7.48 (1H, m), 7.84-7.90 (1H, m), 8.19-8.23 (1H, m), 8.45 (1H, s), 8.51 (1H, d, J = 5.4 Hz), 8.55-8.57 (1H, m), 8.67 (1H, d, J = 8.3 Hz). ESI-MS Found: m/z 527[M+H]⁺

### Example 1-9

### Synthesis of N-{(3,4-difluorophenyl)[4-(1H,1'H-spiro[furo[3,4-c]pyridine-3,4'-piperidin]-1'-ylmethyl)phenyl]methyl}-2-furamide

The same operation as in Example 1-1 was performed using the compound obtained in Reference Example 1-5 (20.0 mg) and 2-furoyl chloride, to give the title compound (23.2 mg) as a white amorphous substance.
¹HNMR (400 MHz, CDCl₃, δppm): 1.76-1.83 (2H, m), 1.98-2.07 (2H, m), 2.40-2.48 (2H, m), 2.80-2.88 (2H, m), 3.58 (2H, s), 5.06 (2H, s), 6.34 (1H, d, J = 7.8 Hz), 6.52-6.53 (1H, m), 6.87 (1H, d, J = 7.8 Hz), 7.03-7.07 (1H, m), 7.08-7.19 (4H, m), 7.23 (2H, d, J = 8.0 Hz), 7.37 (2H, d, J = 8.0 Hz), 7.45-7.46 (1H, m), 8.45 (1H, s), 8.51 (1H, d, J = 5.4 Hz).
ESI-MS Found: m/z 516[M+H]⁺

### Example 1-10

### Synthesis of N-{(3,4-difluorophenyl)[4-(1H,1'H-spiro[furo[3,4-c]pyridine-3,4'-piperidin]-1'-ylmethyl)phenyl]methyl}isoxazol-5-carboxamide

The same operation as in Example 1-1 was performed using the compound obtained in Reference Example 1-5 (20.0 mg) and isoxazole-5-carbonyl chloride, to give the title compound (21.2 mg) as a colorless oil.
¹HNMR (400 MHz, CDCl₃, δppm): 1.77-1.83 (2H, m), 1.98-2.07 (2H, m), 2.40-2.48 (2H, m), 2.81-2.87 (2H, m), 3.59 (2H, s), 5.06 (2H, s), 6.33 (1H, d, J = 7.8 Hz), 6.96-6.97 (1H, m), 7.03-7.08 (1H, m), 7.08-7.20 (4H, m), 7.23 (2H, d, J = 8.3 Hz), 7.39 (2H, d, J = 8.3 Hz), 8.35-8.36 (1H, m), 8.44-8.45 (1H, m), 8.51 (1H, d, J = 4.9 Hz).
ESI-MS Found: m/z 517[M+H]⁺

### Example 1-11

### Synthesis of 1-{(3,4-difluorophenyl)[4-(1H,1'H-spiro[furo[3,4-c]pyridine-3,4'-piperidin]-1'-ylmethyl)phenyl]methyl}pyrrolidine-2,5-dione

The same operation as in Example 1-1 was performed under heating conditions using the compound obtained in Reference Example 1-5 (20.0 mg) and succinyl dichloride, to give the title compound (7.30 mg) as a white amorphous substance.
¹HNMR (400 MHz, CDCl₃, δppm): 1.77-1.83 (2H, m), 1.98-2.07 (2H, m), 2.40-2.48 (2H, m), 2.76 (4H, s), 2.81-2.87 (2H, m), 3.58 (2H, s), 5.06 (2H, s), 6.48 (1H, s), 7.01-7.06 (1H, m), 7.07-7.19 (3H, m), 7.23-7.27 (2H, m), 7.34 (2H, d, J = 7.8 Hz), 8.45 (1H, s), 8.51 (1H, d, J = 5.1 Hz).
ESI-MS Found: m/z 504[M+H]⁺

### Example 1-12

### Synthesis of methyl{(3,4-difluorophenyl)[4-(1H,1'H-spiro[furo[3,4-c]pyridine-3,4'-piperidin]-1'-ylmethyl)phenyl]methyl}carbamate

The same operation as in Example 1-1 was performed using the compound obtained in Reference Example 1-5 (20.0 mg) and methyl chloroformate, to give the title compound (17.5 mg) as a colorless oil.
¹HNMR (400 MHz, CDCl₃, δppm): 1.77-1.83 (2H, m), 1.98-2.07 (2H, m), 2.40-2.48 (2H, m), 2.80-2.86 (2H, m), 3.57 (2H, s), 3.70 (3H, s), 5.05 (2H, s), 5.28 (1H, br s), 5.90 (1H, br s), 6.98-7.02 (1H, m), 7.05-7.11 (2H, m), 7.13-7.19 (3H, m), 7.35 (2H, d, J = 8.0 Hz), 8.44 (1H, s), 8.50 (1H, d, J = 4.8 Hz).
ESI-MS Found: m/z 480[M+H]⁺

### Example 1-13

### Synthesis of N'-{(3,4-difluorophenyl)[4-(1H,1'H-spiro[furo[3,4-c]pyridine-3,4'-piperidin]-1'-ylmethyl)phenyl]methyl}-N,N-dimethylurea

The same operation as in Example 1-1 was performed using the compound obtained in Reference Example 1-5 (20.0 mg) and dimethylcarbamoyl chloride, to give the title compound (6.1 mg) as a white amorphous substance.
¹HNMR (400 MHz, CDCl₃, δppm): 1.77-1.83 (2H, m), 2.00-2.09 (2H, m), 2.41-2.50 (2H, m), 2.82-2.88 (2H, m), 2.95 (6H, s), 3.58 (2H, s), 4.85 (1H, d, J = 6.3 Hz), 5.06 (2H, s), 6.07 (1H, d, J = 6.3 Hz), 6.98-7.04 (1H, m), 7.06-7.14 (2H, m), 7.17-7.20 (3H, m), 7.34 (2H, d, J = 7.8 Hz), 8.45 (1H, br s), 8.51 (1H, d, J = 4.9 Hz).
ESI-MS Found: m/z 493[M+H]⁺

### Example 1-14

### Synthesis of N-{(3,4-difluorophenyl)[4-(1H,1'H-spiro[furo[3,4-c]pyridine-3,4'-piperidin]-1'-ylmethyl)phenyl]methyl}pyrrolidine-1-carboxamide

The same operation as in Example 1-1 was performed using the compound obtained in Reference Example 1-5 (20.0 mg) and 1-pyrrolidinecarbonyl chloride, to give the title compound (9.0 mg) as a white amorphous substance.
¹HNMR (400 MHz, CDCl₃, δppm): 1.77-1.83 (2H, m), 1.90-1.95 (4H, m), 1.97-2.10 (2H, m), 2.39-2.50 (2H, m), 2.80-2.90 (2H, m), 3.35-3.40 (4H, m), 3.58 (2H, s), 4.69 (1H, d, J = 7.0 Hz), 5.06 (2H, s), 6.11 (1H, d, J = 7.0 Hz), 6.99-7.04 (1H, m), 7.06-7.14 (2H, m), 7.17-7.21 (3H, m), 7.34 (2H, d, J = 7.8 Hz), 8.45 (1H, s), 8.51 (1H, d, J = 4.9 Hz).
ESI-MS Found: m/z 519[M+H]⁺

### Example 1-15

### Synthesis of N-{(3,4-difluorophenyl)[4-(1H,1'H-spiro[furo[3,4-c]pyridine-3,4'-piperidin]-1'-ylmethyl)phenyl]methyl}morpholine-4-carboxamide

The same operation as in Example 1-1 was performed using the compound obtained in Reference Example 1-5 (20.0 mg) and 4-morpholinecarbonyl chloride, to give the title compound (10.6 mg) as a white amorphous substance.
¹HNMR (400 MHz, CDCl₃, δppm): 1.77-1.83 (2H, m), 1.98-2.08 (2H, m), 2.40-2.49 (2H, m), 2.81-2.88 (2H, m), 3.38-3.41 (4H, m), 3.58 (2H, s), 3.69-3.72 (4H, m), 4.91 (1H, d, J = 6.3 Hz), 5.06 (2H, s), 6.07 (1H, d, J = 6.3 Hz), 6.98-7.03 (1H, m), 7.05-7.20 (5H, m), 7.35 (2H, d, J = 8.3 Hz), 8.44 (1H, d, J = 1.0 Hz), 8.50 (1H, d, J = 4.9 Hz).
ESI-MS Found: m/z 535[M+H]⁺

### Example 1-16

### Synthesis of N-{(3,4-difluorophenyl)[4-(1H,1'H-spiro[furo[3,4-c]pyridine-3,4'-piperidin]-1'-ylmethyl)phenyl]methyl}methanesulfonamide

The same operation as in Example 1-1 was performed using the compound obtained in Reference Example 1-5 (20.0 mg) and methanesulfonyl chloride, to give the title compound (15.6 g).
¹HNMR (400 MHz, CDCl₃, δppm): 1.77-1.83 (2H, m), 1.97-2.07 (2H, m), 2.41-2.49 (2H, m), 2.75 (3H, s), 2.79-2.86 (2H, m), 3.59 (2H, s), 5.06 (2H, s), 5.15-5.22 (1H, m), 5.73 (1H, d, J = 6.8 Hz), 7.07-7.20 (4H, m), 7.25 (2H, d, J = 8.0 Hz), 7.39 (2H, d, J = 8.0 Hz), 8.42 (1H, s), 8.50 (1H, d, J = 4.9 Hz).
ESI-MS Found: m/z 500[M+H]⁺

### Example 1-17

### Synthesis of N-{(3,4-difluorophenyl)[4-(1H,1'H-spiro[furo[3,4-c]pyridine-3,4'-piperidin]-1'-ylmethyl)phenyl]methyl}cyclopropanesulfonamide

The same operation as in Example 1-1 was performed using the compound obtained in Reference Example 1-5 (20.0 mg) and cyclopropanesulfonyl chloride, to give the title compound (3.60 mg) as a yellow oil.
¹HNMR (400 MHz, CDCl₃, δppm): 0.72-0.85 (2H, m), 0.99-1.16 (2H, m), 1.77-1.83 (2H, m), 1.98-2.08 (2H, m), 2.12-2.19 (1H, m), 2.41-2.48 (2H, m), 2.79-2.86 (2H, m), 3.59 (2H, s), 5.00 (1H, d, J = 7.0 Hz), 5.05 (2H, s), 5.71 (1H, d, J = 7.0 Hz), 7.08-7.22 (4H, m), 7.25 (2H, d, J = 8.0 Hz), 7.38 (2H, d, J = 8.0 Hz), 8.43 (1H, s), 8.50 (1H, d, J = 4.9 Hz).
ESI-MS Found: m/z 526[M+H]⁺

### Example 1-18

### Synthesis of N-{(3,4-difluorophenyl)[4-(1H,1'H-spiro[furo[3,4-c]pyridine-3,4'-piperidin]-1'-ylmethyl)phenyl]methyl}-2-methylpropanamide

To a DMF solution (1.00 mL) of the compound obtained in Reference Example 1-5 (20.0 mg) were added triethylamine (37.0 µL), isobutyric acid (5.24 µL), and 1-hydroxybenzotriazole hydrate (HOBt·H₂O) (7.64 mg). WSC·HCl (10.8 mg) was added at 0°C, and stirred at room temperature overnight. An aqueous sodium hydrogen carbonate solution was added to the reaction mixture, followed by extraction with chloroform. The organic layer was washed with water and saturated brine, and then dried over anhydrous sodium sulfate. Toluene was added to the organic layer, followed by azeotropy and concentration under reduced pressure. The residue was then purified by preparative thin-layer chromatography (chloroform:methanol = 94:6) to give the title compound (16.0 mg) as a colorless oil.
¹HNMR (400 MHz, CDCl₃, δppm): 1.20 (3H, d, J = 4.8 Hz), 1.21 (3H, d, J = 4.8 Hz), 1.77-1.82 (2H, m), 1.99-2.08 (2H, m), 2.40-2.49 (3H, m), 2.81-2.88 (2H, m), 3.58 (2H, s), 5.06 (2H, s), 5.96 (1H, d, J = 7.6 Hz), 6.16 (1H, d, J = 7.6 Hz), 6.94-7.05 (2H, m), 7.09-7.14 (1H, m), 7.15 (2H, d, J = 8.0 Hz), 7.19 (1H, d, J = 4.8 Hz), 7.35 (2H, d, J = 8.0 Hz), 8.44 (1H, s), 8.51 (1H, d, J = 4.8 Hz).
ESI-MS Found: m/z 492[M+H]⁺

### Example 1-19

### Synthesis of 3-{(3,4-difluorophenyl)[4-(1H,1'H-spiro[furo[3,4-c]pyridine-3,4'-piperidin]-1'-ylmethyl)phenyl]methyl}-1,3-oxazolidin-2-one

To a methylene chloride solution (1.00 mL) of the compound obtained in Reference Example 1-5 (50.0 mg) was added triethylamine (76.0 µL). Subsequently, 2-chloroethyl chloroformate (16.9 µL) was added thereto at 0°C, and stirred at 0°C for 30 minutes. An aqueous sodium hydrogen carbonate solution was added to the reaction mixture, followed by extraction with ethyl acetate, and the organic layer was dried over anhydrous magnesium sulfate. The organic layer was concentrated under reduced pressure to give a crude product of 2-chloroethyl {(3,4-difluorophenyl)[4-(1H,1'H-spiro[furo[3,4-c]pyridine-3,4'-piperidin]-1'-ylmethyl)phenyl]methyl}carbamate. Potassium carbonate (15.1 mg) was added to a 2-butanone solution (1.00 mL) of the obtained compound, heated under reflux overnight, and then cooled to room temperature. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by preparative thin-layer chromatography (chloroform:methanol = 90:10) to give the title compound (23.5 mg) as a colorless oil.
¹HNMR (400 MHz, CDCl₃, δppm): 1.78-1.85 (2H, m), 1.99-2.09 (2H, m), 2.41-2.50 (2H, m), 2.81-2.88 (2H, m), 3.32-3.41 (2H, m), 3.60 (2H, s), 4.33-4.42 (2H, m), 5.07 (2H, s), 6.31 (1H, s), 6.96-7.07 (2H, m), 7.14-7.21 (4H, m), 7.39 (2H, d, J = 7.8 Hz), 8.46 (1H, d, J = 1.0 Hz), 8.51 (1H, d, J = 4.9 Hz).
ESI-MS Found: m/z 492[M+H]⁺

### Example 1-20

### Synthesis of 1'-{4-[(3,4-difluorophenyl)(1,1-dioxideisothiazolidin-2-yl)methyl]benzyl}-5-methyl-3,5-dihydro-6H-spiro[furo[3,4-c]pyridine-1,4'-piperidin]-6-one

The same operation as in Example 1-19 was performed using the compound obtained in Reference Example 1-9 (50.0 mg) and 3-chloropropanesulfonyl chloride (13.0 µL), to give the title compound (10.0 mg) as a colorless oil.
¹HNMR (400 MHz, CDCl₃, δppm): 1.73-1.79 (2H, m), 1.83-1.93 (2H, m), 2.28-2.43 (4H, m), 2.76-2.83 (2H, m), 3.03-3.25 (4H, m), 3.53 (3H, s), 3.55 (2H, s), 4.82 (2H, s), 5.92 (1H, s), 6.35 (1H, s), 7.06-7.19 (4H, m), 7.24 (2H, d, J = 8.3 Hz), 7.34 (2H, d, J = 8.3 Hz).
ESI-MS Found: m/z 556[M+H]⁺

### Example 1-21

### Synthesis of 1-{(3,4-difluorophenyl)[5-(1H,1'H-spiro[furo[3,4-c]pyridine-3,4'-piperidin]-1'-ylmethyl)pyridin-2-yl]methyl}-3,3-dimethylpyrrolidine-2,5-dione

To a benzene solution (1.00 mL) of 2,2-dimethylsuccinic acid (20.8 mg) were added pyridine (23.9 µL) and oxalyl dichloride (37.3 µL), and the mixture was heated under reflux for 30 minutes. The reaction mixture was cooled to room temperature, followed by concentration under reduced pressure, to give a mixture of 4-chloro-2,2-dimethyl-4-oxobutanoic acid and 2,2-dimethylsuccinyl dichloride. A benzene solution (1.00 mL) of the compound obtained in Reference Example 2-2 (50.0 mg) and pyridine (119 µL) were added to a benzene solution (1.00 mL) of the obtained mixture at 0°C, and heated under reflux for 30 minutes. The reaction mixture was cooled to room temperature, followed by concentration under reduced pressure, to give the title compound and a cyclization precursor thereof as a mixture. Sodium acetate (29.1 mg) was added to an acetic anhydride solution (2.00 mL) of the obtained mixture, and stirred at room temperature for 30 minutes. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by preparative thin-layer chromatography (chloroform:methanol = 90:10) to give the title compound (3.50 mg) as a white amorphous substance.
¹HNMR (400 MHz, CDCl₃, δppm): 1.35 (6H, s), 1.76-1.83 (2H, m), 1.98-2.07 (2H, m), 2.44-2.52 (2H, m), 2.63 (2H, s), 2.80-2.88 (2H, m), 3.60 (2H, s), 5.06 (2H, s), 6.44 (1H, s), 7.02 (1H, d, J = 8.3 Hz), 7.10-7.23 (3H, m), 7.36 (1H, ddd, J = 11.2, 7.8, 2.0 Hz), 7.67 (1H, dd, J = 8.3, 2.0 Hz), 8.45 (1H, s), 8.50-8.52 (2H, m).
ESI-MS Found: m/z 533[M+H]⁺

### Example 1-22

### Synthesis of 3-{(3,4-difluorophenyl)[4-(1H,1'H-spiro[furo[3,4-c]pyridine-3,4'-piperidin]-1'-ylmethyl)phenyl]methyl}-1,3-oxazolidine-2,4-dione

To a THF solution (1.50 mL) of the compound obtained in Reference Example 1-5 (100 mg) was added triethylamine (131 µL). Subsequently, acetoxyacetyl chloride (24.0 µL) was added thereto at 0°C, and the mixture was stirred at 0°C for 30 minutes. An aqueous sodium hydrogen carbonate solution was added to the reaction mixture, followed by extraction with ethyl acetate, and the organic layer was dried over anhydrous magnesium sulfate. The organic layer was concentrated under reduced pressure to give a crude product of 2-({(3,4-difluorophenyl)[4-(1H,1'H-spiro[furo[3,4-c]pyridine-3,4'-piperidin]-1'-ylmethyl)phenyl]methyl}amino)-2-oxoethyl acetate. A 5.00 M aqueous sodium hydroxide solution (100 µL) was added to a methanol solution (1.00 mL) of the obtained crude product, and stirred at room temperature for 1 hour. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by silica gel column chromatography (chloroform:methanol = 100:0 to 80:20), to give N-{(3,4-difluorophenyl)[4-(1H,1'H-spiro[furo[3,4-c]pyridine-3,4'-piperidin]-1'-ylmethyl)phenyl]methyl}-2-hydroxyacetamide (76.3 mg) as a yellow oil. Triethylamine (36.0 µL) and 1,1'-carbonyldiimidazole (11.0 mg) were added to a methylene chloride solution (2.00 mL) of the obtained compound (25.0 mg), and stirred at room temperature overnight. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by preparative thin-layer chromatography (chloroform:methanol = 90:10) to give the title compound (19.1 mg) as a colorless oil.
¹HNMR (400 MHz, CDCl₃, δppm): 1.77-1.83 (2H, m), 1.98-2.08 (2H, m), 2.40-2.49 (2H, m), 2.80-2.88 (2H, m), 3.59 (2H, s), 4.74 (2H, s), 5.06 (2H, s), 6.40 (1H, s), 7.05-7.11 (1H, m), 7.12-7.22 (3H, m), 7.29 (2H, d, J = 8.0 Hz), 7.39 (2H, d, J = 8.0 Hz), 8.46 (1H, s), 8.51 (1H, d, J = 4.9 Hz).
ESI-MS Found: m/z 506[M+H]⁺

### Example 1-23

### Synthesis of 1-{(3,4-difluorophenyl)[4-(1H,1'H-spiro[furo[3,4-c]pyridine-3,4'-piperidin]-1'-ylmethyl)phenyl]methyl}-3-methylimidazolidin-2-one

To a methanol solution (2.00 mL) of the compound obtained in Reference Example 1-5 (100 mg) were added triethylamine (130 µL) and tert-butyl methyl(2-oxoethyl)carbamate (198 mg), and the mixture was stirred at room temperature for 30 minutes. Sodium borohydroxide (14.2 mg) was added to the reaction mixture, and stirred at room temperature for 30 minutes. An aqueous sodium hydrogen carbonate solution was added to the reaction mixture, followed by extraction with ethyl acetate, and the organic layer was dried over anhydrous magnesium sulfate. The organic layer was concentrated under reduced pressure to give a crude product of tert-butyl [2-({(3,4-difluorophenyl)[4-(1H,1'H-spiro[furo[3,4-c]pyridine-3,4'-piperidin]-1'-ylmethyl)phenyl]methyl}amino)ethyl]methylcarbamate. A 5.00 M aqueous hydrochloric acid solution (500 µL) was added to an ethyl acetate solution (1.00 mL) of the obtained crude product, and stirred at room temperature overnight. The reaction mixture was concentrated under reduced pressure, and then the residue was subjected to azeotropy with ethyl acetate, to give a crude product of N-{(3,4-difluorophenyl)[4-(1H,1'H-spiro[furo[3,4-c]pyridine-3,4'-piperidin]-1'-ylmethyl)phenyl]methyl}-N'-methylethane-1,2-diamine tetrahydrochloride. Triethylamine (260 µL) and triphosgene (55.9 mg) were added to a THF solution (2.00 mL) of the obtained crude product, and stirred at room temperature for 2 hours. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by preparative thin-layer chromatography (chloroform:methanol = 20:1) to give the title compound (7.40 mg) as a colorless oil.
¹HNMR (400 MHz, CDCl₃, δppm): 1.79-1.85 (2H, m), 1.95-2.20 (2H, m), 2.40-2.60 (2H, m), 2.84-2.85 (3H, m), 2.88-2.96 (2H, m), 3.08-3.14 (2H, m), 3.29-3.35 (2H, m), 3.60-3.68 (2H, m), 5.06 (2H, s), 6.38 (1H, s), 6.94-6.99 (1H, m), 7.00-7.07 (1H, m), 7.09-7.20 (4H, m), 7.33-7.43 (2H, m), 8.47 (1H, s), 8.52 (1H, d, J = 4.9 Hz).
ESI-MS Found: m/z 505[M+H]⁺

### Example 1-24

### Synthesis of (R)- or (S)-N-{(3,4-difluorophenyl)[4-(1H,1'H-spiro[furo[3,4-c]pyridine-3,4'-piperidin]-1'-ylmethyl)phenyl]methyl}cyclopropanecarboxamide

The same operation as in Example 1-1 was performed using the compound obtained in Reference Example 1-5 (100 mg) and cyclopropanecarbonyl chloride, to give a racemate (66.1 mg) of the title compound. Further, the product was purified by high-performance liquid chromatography (CHIRALPAK™ AD-H, hexane:ethanol:diethylamine = 60:40:0.04) to give the title compound (32.6 mg, faster) as a white amorphous substance. ¹HNMR (400 MHz, CDCl₃, δppm): 0.76-0.81 (2H, m), 0.99-1.02 (2H, m), 1.40-1.47 (1H, m), 1.77-1.82 (2H, m), 1.99-2.08 (2H, m), 2.40-2.49 (2H, m), 2.80-2.87 (2H, m), 3.58 (2H, s), 5.06 (2H, s), 6.18-6.22 (2H, m), 6.98-7.02 (1H, m), 7.03-7.14 (2H, m), 7.15-7.20 (3H, m), 7.35 (2H, d, J = 8.0 Hz), 8.44 (1H, s), 8.50 (1H, d, J = 4.8 Hz).
ESI-MS Found: m/z 490[M+H]⁺

### Example 1-25

### Synthesis of (R)- or (S)-N-{(3,4-difluorophenyl)[4-(1H,1'H-spiro[furo[3,4-c]pyridine-3,4'-piperidin]-1'-ylmethyl)phenyl]methyl}-2,2-difluoroacetamide

The same operation as in Example 1-1 was performed using the compound obtained in Reference Example 1-5 (100 mg) and difluoroacetic anhydride, to give a racemate (76.8 mg) of the title compound. Further, the product was purified by high-performance liquid chromatography (CHIRALPAK™ AS-H, hexane:ethanol:diethylamine = 80:20:0.02) to give the title compound (32.4 mg, faster) as a white amorphous substance.
¹HNMR (400 MHz, CDCl₃, δppm): 1.78-1.82 (2H, m), 1.99-2.08 (2H, m), 2.41-2.50 (2H, m), 2.80-2.87 (2H, m), 3.59 (2H, s), 5.06 (2H, s), 5.97 (1H, t, J = 14.4 Hz), 6.19 (1H, d, J = 8.4 Hz), 6.84 (1H, br s), 6.98-7.08 (2H, m), 7.12-7.20 (4H, m), 7.39 (2H, d, J = 8.0 Hz), 8.44 (1H, s), 8.50 (1H, d, J = 4.8 Hz).
ESI-MS Found: m/z 500[M+H]⁺

### Example 1-26

### Synthesis of (R)- or (S)-N-{(3,4-difluorophenyl)[5-(1H,1'H-spiro[furo[3,4-c]pyridine-3,4'-piperidin]-1'-ylmethyl)pyridin-2-yl]methyl}-2,2-difluoroacetamide

The same operation as in Example 1-25 was performed using the compound obtained in Reference Example 2-2 (30.0 mg), to give a racemate (21.7 mg) of the title compound. Further, the product was purified by high-performance liquid chromatography (CHIRALPAK™ AS-H, hexane:ethanol:diethylamine = 60:40:0.04) to give the title compound (6.40 mg, slower) as a colorless oil.
¹HNMR (400 MHz, CDCl₃, δppm): 1.77-1.84 (2H, m), 1.96-2.06 (2H, m), 2.44-2.52 (2H, m), 2.77-2.84 (2H, m), 3.60 (2H, s), 5.06 (2H, s), 5.94 (1H, t, J = 14.0 Hz), 6.03 (1H, d, J = 6.8 Hz), 7.10-7.20 (5H, m), 7.73 (1H, dd, J = 7.2, 2.4 Hz), 8.45 (1H, s), 8.52 (1H, d, J = 5.2 Hz), 8.57 (1H, d, J = 2.4 Hz), 8.67 (1H, d, J = 7.2 Hz).
ESI-MS Found: m/z 501[M+H]⁺

### Example 1-27

### Synthesis of (R)- or (S)-N-{(3,4-difluorophenyl) [5-(1H,1'H-spiro[furo[3,4-c]pyridine-3,4'-piperidin]-1'-ylmethyl)pyridin-2-yl]methyl}-2,2-difluoro-N-methylacetamide

The same operation as in Example 1-25 was performed using the compound obtained in Reference Example 3-2 (41.0 mg), to give a racemate (39.6 mg) of the title compound. Further, the product was purified by high-performance liquid chromatography (CHIRALPAK™ AS-H, hexane:ethanol:diethylamine = 80:20:0.02) to give the title compound (18.3 mg, faster) as a colorless oil.
¹HNMR (400 MHz, CDCl₃, δppm): 1.79-1.86 (2H, m), 2.00-2.10 (2H, m), 2.46-2.55 (2H, m), 2.80-2.88 (2H, m), 2.91 (2/3H, s), 3.08 (7/3H, s), 3.61 (2H, s), 5.07 (2H, s), 6.21 (7/9H, t, J = 13.6 Hz), 6.27 (2/9H, t, J = 13.6 Hz), 6.50 (2/9H, s), 6.90 (7/9H, s), 6.92-7.00 (1H, m), 7.02-7.09 (1H, m), 7.12-7.21 (2H, m), 7.23-7.28 (1H, m), 7.73-7.81 (1H, m), 8.46 (1H, s), 8.52 (1H, d, J = 4.8 Hz), 8.60 (1H, d, J = 2.0 Hz).
ESI-MS Found: m/z 515[M+H]⁺

### Example 1-28

### Synthesis of (R)- or (S)-N-{(3,4-difluorophenyl)[5-(1H,1'H-spiro[furo[3,4-c]pyridine-3,4'-piperidin]-1'-ylmethyl)pyridin-2-yl]methyl}-N-(2-hydroxy-2-methylpropyl)propanamide

The same operation as in Example 1-3 was performed under heating conditions using the compound obtained in Reference Example 3-7 (37.8 mg) and propionic anhydride, to give a racemate (31.4 mg) of the title compound. Further, the product was purified by high-performance liquid chromatography (CHIRALPAK™ AD-H, hexane:ethanol:diethylamine = 60:40:0.04) to give the title compound (13.9 mg, slower) as a colorless oil.
¹HNMR (400 MHz, CDCl₃, δppm): 1.10 (3H, t, J = 7.2 Hz), 1.28 (3H, s), 1.30 (3H, s), 1.78-1.85 (2H, m), 1.97-2.07 (2H, m), 2.35-2.53 (4H, m), 2.78-2.87 (2H, m), 3.33 (1H, d, J = 15.2 Hz), 3.55-3.65 (3H, m), 5.06 (2H, s), 6.15 (4/5H, s), 6.41 (1/5H, s), 6.90-7.23 (5H, m), 7.65-7.80 (1H, m), 8.46 (1H, s), 8.46-8.48 (4/5H, m), 8.51 (1H, d, J = 4.8 Hz), 8.60-8.63 (1/5H, m). ESI-MS Found: m/z 551[M+H]⁺

### Example 1-29

### Synthesis of (R)- or (S)-N-{(5-chloropyridin-2-yl)[4-(1H,1'H-spiro[furo[3,4-c]pyridine-3,4'-piperidin]-1'-ylmethyl)phenyl]methyl}propanamide

The same operation as in Example 1-1 was performed using the compound obtained in Reference Example 3-9 (33.8 mg) and propionic anhydride, to give a racemate (33.6 mg) of the title compound. Further, the product was purified by high-performance liquid chromatography (CHIRALPAK™ AS-H, hexane:ethanol:diethylamine = 80:20:0.02) to give the title compound (15.4 mg, faster) as a white solid.
¹HNMR (400 MHz, CDCl₃, δppm): 1.18 (3H, t, J = 7.6 Hz), 1.73-1.80 (2H, m), 1.95-2.04 (2H, m), 2.32 (2H, q, J = 7.6 Hz), 2.35-2.44 (2H, m), 2.78-2.83 (2H, m), 3.52 (2H, s), 5.04 (2H, s), 6.16 (1H, d, J = 7.2 Hz), 7.17 (1H, d, J = 4.8 Hz), 7.22 (1H, d, J = 8.0 Hz), 7.24-7.35 (5H, m), 7.61 (1H, dd, J = 8.0, 2.4 Hz), 8.44 (1H, s), 8.50 (1H, d, J = 4.8 Hz), 8.53 (1H, d, J = 2.4 Hz). ESI-MS Found: m/z 477, 479[M+H]⁺

### Example 1-30

### Synthesis of (R)- or (S)-N-{3,4-difluorophenyl)[4-(1H,1'H-spiro[furo[3,4-c]pyridine-3,4'-piperidin]-1'-ylmethyl)phenyl]methyl}-N-(2-hydroxy-2-methylpropyl)acetamide

The same operation as in Example 1-3 was performed under heating conditions using the compound obtained in Reference Example 3-6 (21.0 mg), to give a racemate (14.5 mg) of the title compound. Further, the product was purified by high-performance liquid chromatography (CHIRALPAK™ AD-H, hexane:isopropanol:diethylamine = 70:30:0.03) to give the title compound (5.90 mg, faster) as a colorless oil.
¹HNMR (400 MHz, CDCl₃, δppm): 0.62 (3H, s), 0.75 (3H, s), 1.79-1.83 (2H, m), 1.99-2.08 (2H, m), 2.24 (3H, s), 2.41-2.50 (2H, m), 2.80-2.86 (2H, m), 3.47 (1H, d, J = 14.8 Hz), 3.57 (1H, d, J = 14.8 Hz), 3.61 (2H, s), 5.06 (2H, s), 5.48 (1H, s), 6.22 (1H, s), 6.92-7.03 (2H, m), 7.13 (2H, d, J = 8.0 Hz), 7.15-7.22 (2H, m), 7.40 (2H, d, J = 8.0 Hz), 8.46 (1H, s), 8.51 (1H, d, J = 4.8 Hz).
ESI-MS Found: m/z 536[M+H]⁺

### Example 1-31

### Synthesis of (R)- or (S)-N-{3,4-difluorophenyl)[4-(1H,1'H-spiro[furo[3,4-c]pyridine-3,4'-piperidin]-1'-ylmethyl)phenyl]methyl}-N-methoxyacetamide

The same operation as in Example 1-3 was performed using the compound obtained in Reference Example 6-3 (20.2 mg), to give a racemate (20.2 mg) of the title compound. Further, the product was purified by high-performance liquid chromatography (CHIRALPAK™ AS-H, hexane:ethanol:diethylamine = 90:10:0.01) to give the title compound (8.0 mg, slower) as a colorless oil.
¹HNMR (400 MHz, CDCl₃, δppm): 1.78-1.82 (2H, m), 2.01-2.08 (2H, m), 2.26 (3H, s), 2.42-2.48 (2H, m), 2.84-2.86 (2H, m), 3.23 (3H, s), 3.60 (2H, s), 5.06 (2H, s), 6.71 (1H, s), 7.02-7.06 (1H, m), 7.09-7.18 (2H, m), 7.18 (1H, d, J = 4.8 Hz), 7.24 (2H, d, J = 8.0 Hz), 7.36 (2H, d, J = 8.0 Hz), 8.46 (1H, s), 8.51 (1H, d, J = 4.8 Hz).
ESI-MS Found: m/z 494[M+H]⁺

### Example 1-32

### Synthesis of (R)- or (S)-N-{3,4-difluorophenyl)[4-(1H,1'H-spiro[furo[3,4-c]pyridine-3,4'-piperidin]-1'-ylmethyl)phenyl]methyl}-N-(2-hydroxy-2-methylpropoxy)acetamide

The same operation as in Example 1-3 was performed using the compound obtained in Reference Example 6-5 (31.1 mg), to give a racemate (22.7 mg) of the title compound. Further, the product was purified by high-performance liquid chromatography (CHIRALPAK™ OD-H, hexane:isopropanol:diethylamine = 80:20:0.02) to give the title compound (9.9 mg, slower) as a colorless oil.
¹HNMR (400 MHz, CDCl₃, δppm): 1.04 (3H, s), 1.07 (3H, s), 1.77-1.82 (2H, m), 1.98-2.07 (2H, m), 2.25 (3H, s), 2.40-2.50 (2H, m), 2.79-2.86 (2H, m), 3.04 (1H, d, J = 8.4 Hz), 3.24 (1H, d, J = 8.4 Hz), 3.61 (2H, s), 5.05 (2H, s), 6.64 (1H, br s), 7.04-7.09 (1H, m), 7.10-7.20 (3H, m), 7.21-7.28 (2H, m), 7.35 (2H, d, J = 8.4 Hz), 8.44 (1H, s), 8.51 (1H, d, J = 4.8 Hz).
ESI-MS Found: m/z 552[M+H]⁺

### Example 1-33

### Synthesis of (R)- or (S)-N-((3,4-difluorophenyl){4-[(5-methyl-6-oxo-5,6-dihydro-1'H,3H-sbiro[furo[3,4-c]pyridine-1,4'-piberidin]-1'-yl)methyl]phenyl}methyl)-2,2-dimethylpropanamide

The same operation as in Example 1-1 was performed using the compound obtained in Reference Example 1-9 (40.0 mg) and 2,2-dimethylpropanoyl chloride (14.0 µL), to give a racemate (33.0 mg) of the title compound. Further, the product was purified by high-performance liquid chromatography (CHIRALPAK™ AD-H, hexane:ethanol:diethylamine = 50:50:0.05) to give the title compound (15.6 mg, slower) as a white amorphous substance. ¹HNMR (400 MHz, CDCl₃, δppm): 1.25 (9H, s), 1.72-1.79 (2H, m), 1.82-1.92 (2H, m), 2.33-2.41 (2H, m), 2.76-2.82 (2H, m), 3.53 (3H, s), 3.54 (2H, s), 4.81 (2H, s), 6.11-6.14 (2H, m), 6.33 (1H, s), 6.93-6.97 (1H, m), 7.01 (1H, ddd, J = 11.0, 7.6, 2.2 Hz), 7.08-7.17 (4H, m), 7.32 (2H, d, J = 8.3 Hz).
ESI-MS Found: m/z 536[M+H]⁺

### Example 1-34

### Synthesis of (R)- or (S)-N-[(3,4-difluorophenyl)(4-{[4-(6-fluoropyridin-3-yl)piperidin-1-yl]methyl}phenyl)methyl]-2-hydroxy-2-methylpropanamide

The same operation as in Example 1-1 was performed using the compound obtained in Reference Example 1-6 (50.0 mg) and 2-chloro-1,1-dimethyl-2-oxoethyl acetate, to give 2-{[(3,4-difluorophenyl)(4-{[4-(6-fluoropyridin-3-yl)piperidin-1-yl]methyl}phenyl)methyl]amino}-1,1-dimethyl-2-oxoethyl acetate (48.1 mg). Further, a 5.00 M aqueous sodium hydroxide solution (1 mL) was added to an ethanol solution (5.00 mL) of the obtained compound, and stirred at room temperature for 2 hours. The reaction mixture was extracted with chloroform. The organic layer was washed with water and saturated brine, and dried over anhydrous sodium sulfate. The organic layer was concentrated under reduced pressure, and then the residue was purified by preparative thin-layer chromatography (chloroform:methanol = 95:5) to give a racemate (39.4 mg) of the title compound. Further, the product was purified by high-performance liquid chromatography (CHIRALPAK™ AD-H, hexane:isopropanol:diethylamine = 80:20:0.02) to give the title compound (16.4 mg, slower) as a colorless oil.
¹HNMR (400 MHz, CDCl₃, δppm): 1.49 (3H, s), 1.50 (3H, s), 1.72-1.84 (4H, m), 2.04-2.13 (2H, m), 2.19 (1H, br s), 2.50-2.60 (1H, m), 2.98-3.04 (2H, m), 3.53 (2H, s), 6.12 (1H, d, J = 8.0 Hz), 6.86 (1H, dd, J = 8.0, 2.8 Hz), 6.94-7.06 (2H, m), 7.08-7.16 (1H, m), 7.15 (2H, d, J = 8.4 Hz), 7.32 (2H, d, J = 8.4 Hz), 7.32-7.36 (1H, m), 7.64 (1H, ddd, J = 8.0, 8.0, 2.8 Hz), 8.06 (1H, d, J = 2.8 Hz).
ESI-MS Found: m/z 498[M+H]⁺

### Example 1-35

### Synthesis of (R)- or (S)-N-{(3,4-difluorophenyl)[4-(1H,1'H-spiro[furo[3,4-c]pyridine-3,4'-piperidin]-1'-ylmethyl)phenyl]methyl}-2-hydroxy-N,2-dimethylpropanamide

The same operation as in Example 1-34 was performed using the compound obtained in Reference Example 3-1 (134 mg), to give a racemate (49.4 mg) of the title compound. Further, the product was purified by high-performance liquid chromatography (CHIRALPAK™ AD-H, hexane:ethanol:diethylamine = 60:40:0.04) to give the title compound (21.7 mg, slower) as a colorless oil.
¹HNMR (400 MHz, CDCl₃, δppm): 1.58 (6H, s), 1.78-1.85 (2H, m), 2.00-2.09 (2H, m), 2.42-2.50 (2H, m), 2.78 (3/5H, br s), 2.82-2.89 (2H, m), 2.93 (12/5H, br s), 3.59 (2H, s), 4.43 (1H, br s), 5.07 (2H, s), 6.90-6.96 (1H, m), 6.97-7.10 (2H, m), 7.12 (2H, d, J = 8.4 Hz), 7.14-7.20 (2H, m), 7.37 (2H, d, J = 8.4 Hz), 8.44 (1H, s), 8.51 (1H, d, J = 4.4 Hz).
ESI-MS Found: m/z 522[M+H]⁺

### Example 1-36

### Synthesis of (R)- or (S)-N-[(3,4-difluorophenyl)(5-{[4-(6-fluoropyridin-3-yl)piperidin-1-yl]methyl}pyridin-2-yl)methyl]-2-hydroxy-2-methylpropanamide

The same operation as in Example 1-34 was performed using the compound obtained in Reference Example 2-3 (40.0 mg), to give a racemate (33.0 mg) of the title compound. Further, the product was purified by high-performance liquid chromatography (CHIRALPAK™ AD-H, hexane:ethanol:diethylamine = 70:30:0.03) to give the title compound (14.2 mg, slower) as a colorless oil.
¹HNMR (400 MHz, CDCl₃, δppm): 1.46 (3H, s), 1.48 (3H, s), 1.69-1.86 (4H, m), 2.09-2.17 (2H, m), 2.50-2.62 (2H, m), 2.96-3.02 (2H, m), 3.54 (2H, s), 6.01 (1H, d, J = 7.2 Hz), 6.87 (1H, dd, J = 8.4, 2.8 Hz), 7.04-7.16 (3H, m), 7.19 (1H, d, J = 8.0 Hz), 7.63 (1H, ddd, J = 8.0, 8.0, 2.8 Hz), 7.67 (1H, dd, J = 8.0, 2.0 Hz), 8.06 (1H, d, 2.8 Hz), 8.53 (1H, d, J = 2.0 Hz), 8.55 (1H, d, J = 7.2 Hz).
ESI-MS Found: m/z 499[M+H]⁺

### Example 1-37

### Synthesis of (R)- or (S)-N-[(3,4-difluorophenyl)(5-{[4-(6-fluoropyridin-3-yl)piperidin-1-yl]methyl}pyridin-2-yl)methyl]-2-hydroxy-N,2-dimethylpropanamide

The same operation as in Example 1-34 was performed using the compound obtained in Reference Example 3-3 (35.2 mg), to give a racemate (37.5 mg) of the title compound. Further, the product was purified by high-performance liquid chromatography (CHIRALPAK™ AD-H, hexane:ethanol:diethylamine = 60:40:0.04) to give the title compound (14.4 mg, slower) as a colorless oil.
¹HNMR (400 MHz, CDCl₃, δppm): 1.56 (6H, s), 1.71-1.88 (4H, m), 2.10-2.20 (2H, m), 2.52-2.62 (1H, m), 2.78 (3/4H, br s), 2.98-3.06 (2H, m), 3.07 (9/4H, br s), 3.57 (2H, s), 4.43 (1H, br s), 6.88 (1H, dd, J = 8.0, 2.8 Hz, ), 6.90-6.96 (1H, m), 6.97-7.06 (2H, m), 7.12-7.20 (1H, m), 7.22 (1H, d, J = 7.6 Hz), 7.65 (1H, ddd, J = 8.0, 8.0, 2.8 Hz), 7.73 (1H, d, J = 7.6 Hz), 8.08 (1H, d, J = 2.8 Hz), 8.57 (1H, s).
ESI-MS Found: m/z 513[M+H]⁺

### Example 1-38

### Synthesis of (R)- or (S)-N-{(3,4-difluorophenyl)[5-(1H,1'H-spiro[furo[3,4-c]pyridine-3,4'-piperidin]-1'-ylmethyl)pyridin-2-yl]methyl}-N-ethyl-2-hydroxy-2-methylpropanamide

The same operation as in Example 1-34 was performed using the compound obtained in Reference Example 3-5 (46.3 mg), to give a racemate (22.0 mg) of the title compound. Further, the product was purified by high-performance liquid chromatography (CHIRALPAK™ AD-H, hexane:ethanol:diethylamine = 75:25:0.025) to give the title compound (9.9 mg, faster) as a colorless oil. ¹HNMR (400 MHz, CDCl₃, δppm): 0.48-0.56 (9/5H, m), 0.88-0.98 (6/5H, m), 1.55 (6H, s), 1.78-1.86 (2H, m), 1.98-2.08 (2H, m), 2.44-2.53 (2H, m), 2.80-2.86 (2H, m), 3.35-3.80 (4H, m),

4.32-4.49 (1H, m), 5.06 (2H, s), 6.90-7.28 (6H, m), 7.68-7.80 (1H, m), 8.44 (1H, s), 8.51 (1H, d, J = 4.8 Hz), 8.56-8.62 (1H, m).
ESI-MS Found: m/z 537[M+H]⁺

### Example 1-39

### Synthesis of (R)- or (S)-N-cyclopropyl-N-{(3,4-difluorophenyl)[5-(1H,1'H-spiro[furo[3,4-c]pyridine-3,4'-piperidin]-1'-ylmethyl)pyridin-2-yl]methyl}-2-hydroxy-2-methylpropanamide

The same operation as in Example 1-34 was performed using the compound obtained in Reference Example 3-8 (30.1 mg), to give a racemate (13.0 mg) of the title compound. Further, the product was purified by high-performance liquid chromatography (CHIRALPAK™ AD-H, hexane:ethanol:diethylamine = 50:50:0.05) to give the title compound (5.92 mg, slower) as a white amorphous substance.
¹HNMR (400 MHz, CDCl₃, δppm): 0.64-0.70 (1H, m), 0.76-0.94 (3H, m), 1.59 (3H, s), 1.60 (3H, s), 1.78-1.84 (2H, m), 1.98-2.07 (2H, m), 2.43-2.52 (2H, m), 2.79-2.86 (2H, m), 3.59 (2H, s), 4.44 (1H, s), 5.06 (2H, s), 6.48 (1H, s), 6.95-7.00 (1H, m), 7.06-7.20 (4H, m), 7.68 (1H, dd, J = 8.0, 2.2 Hz), 8.45 (1H, s), 8.51 (1H, d, J = 4.9 Hz), 8.53 (1H, d, J = 1.5 Hz).
ESI-MS Found: m/z 549[M+H]⁺

### Example 1-40

### Synthesis of (R)- or (S)-N-{(3,4-difluorophenyl)[5-(1H,1'H-spiro[furo[3,4-c]pyridine-3,4'-piperidin]-1'-ylmethyl)pyrimidin-2-yl]methyl}-2-hydroxy-N,2-dimethylpropanamide

The same operation as in Example 1-34 was performed using the compound obtained in Reference Example 3-4 (40.9 mg), to give a racemate (22.0 mg) of the title compound. Further, the product was purified by high-performance liquid chromatography (CHIRALPAK™ AD-H, hexane:isopropanol:diethylamine = 50:50:0.05) to give the title compound (10.3 mg, slower) as a colorless oil.
¹HNMR (400 MHz, CDCl₃, δppm): 1.56 (3H, s), 1.59 (3H, s), 1.80-1.88 (2H, m), 2.00-2.10 (2H, m), 2.50-2.60 (2H, m), 2.80-2.90 (2H, m), 3.08 (3H, s), 3.62 (2H, s), 4.48 (1H, br s), 5.07 (2H, s), 6.87-6.92 (1H, m), 6.93-7.00 (1H, m), 7.10 (1H, s), 7.10-7.18 (1H, m), 7.20 (1H, d, J = 4.8 Hz), 8.46 (1H, s), 8.53 (1H, d, J = 4.8 Hz), 8.75 (2H, s).
ESI-MS Found: m/z 524[M+H]⁺

### Example 1-41

### Synthesis of (R)- or (S)-N-((3,4-difluorophenyl){4-[(4-pyrazolo[1,5-b]pyridazin-3-ylpiperidin-1-yl)methyl]phgnyl}methyl)-1-hydroxycyclopropanecarboxamide

The same operation as in Example 1-18 was performed using the compound obtained in Reference Example 1-7 (50.0 mg) and 1-hydroxycyclopropanecarboxylic acid, to give a racemate (53.4 mg) of the title compound. Further, the product was purified by high-performance liquid chromatography (CHIRALPAK™ AD-H, hexane:ethanol:diethylamine = 60:40:0.04) to give the title compound (23.3 mg, slower) as a colorless oil.
¹HNMR (400 MHz, CDCl₃, δppm): 0.99-1.10 (2H, m), 1.30-1.40 (2H, m), 1.80-1.92 (4H, m), 2.10-2.20 (2H, m), 2.75-2.82 (1H, m), 2.95-3.02 (2H, m), 3.50 (1H, d, J = 13.2 Hz), 3.54 (1H, d, J = 13.2 Hz), 6.18 (1H, d, J = 8.0 Hz), 6.91 (1H, dd, J = 8.8, 4.0 Hz), 6.96-7.12 (3H, m), 7.16 (2H, d, J = 8.0 Hz), 7.30 (2H, d, J = 8.0 Hz), 7.62 (1H, d, J = 8.0 Hz), 7.83 (1H, s), 7.93 (1H, dd, J = 8.8, 1.6 Hz), 8.17 (1H, dd, J = 4.0, 1.6 Hz).
ESI-MS Found: m/z 518[M+H]⁺

### Example 1-42

### Synthesis of (R)- or (S)-N-[(3,4-difluorophenyl)(5-{[4-(6-fluoropyridin-3-yl)piperidin-1-yl]methyl}pyridin-2-yl)methyl]-1-hydroxy-N-methylcyclopropanecarboxamide

The same operation as in Example 1-41 was performed using the compound obtained in Reference Example 3-3 (20.6 mg), to give a racemate (23.0 mg) of the title compound. Further, the product was purified by high-performance liquid chromatography (CHIRALPAK™ AD-H, hexane:ethanol:diethylamine = 50:50:0.05) to give the title compound (11.2 mg, faster) as a colorless oil.
¹HNMR (400 MHz, CDCl₃, δppm): 1.00-1.04 (2H, m), 1.04-1.14 (1H, m), 1.14-1.20 (1H, m), 1.70-1.86 (4H, m), 2.12-2.19 (2H, m), 2.52-2.62 (1H, m), 2.90-3.04 (5H, m), 3.57 (2H, s), 6.88 (1H, dd, J = 8.0, 2.8 Hz), 6.92-6.98 (1H, m), 7.01-7.07 (2H, m), 7.15 (1H, dd, J = 17.2, 8.0 Hz), 7.24 (1H, d, J = 7.6 Hz), 7.64 (1H, ddd, J = 8.0, 8.0, 2.4 Hz), 7.73 (1H, d, 7.6 Hz), 8.07 (1H, d, J = 2.4 Hz), 8.56 (1H, s).
ESI-MS Found: m/z 511[M+H]⁺

### Example 1-43

### Synthesis of (R)- or (S)-N-{(3,4-difluorophenyl)[5-(1H,1'H-spiro[furor3,4-c]pyridine-3,4'-piperidin]-1'-ylmethyl)pyridin-2-yl]methyl}-1-methylcyclopropanecarboxamide

The same operation as in Example 1-18 was performed using the compound obtained in Reference Example 2-2 (40.0 mg) and 1-methylcyclopropanecarboxylic acid (13.9 mg), to give a racemate (36.0 mg) of the title compound. Further, the product was purified by high-performance liquid chromatography (CHIRALPAK™ AD-H, hexane:isopropanol:diethylamine = 60:40:0.04) to give the title compound (9.60 mg, faster) as a white amorphous substance.
¹HNMR (400 MHz, CDCl₃, δppm): 0.57-0.61 (2H, m), 1.17-1.21 (2H, m), 1.46 (3H, s), 1.77-1.83 (2H, m), 1.97-2.06 (2H, m), 2.43-2.51 (2H, m), 2.78-2.84 (2H, m), 3.58 (2H, s), 5.06 (2H,
s), 6.03 (1H, d, J = 6.3 Hz), 7.00-7.19 (5H, m), 7.69 (1H, dd, J = 8.0, 2.2 Hz), 8.07 (1H, d, J = 6.3 Hz), 8.44-8.45 (1H, m), 8.51 (1H, d, J = 4.9 Hz), 8.56 (1H, d, J = 2.2 Hz).
ESI-MS Found: m/z 505[M+H]⁺

### Example 1-44

### Synthesis of (R)- or (S)-N-{(3,4-difluorophenyl)[5-(1H,1'H-spiro[furo[3,4-c]pyridine-3,4'-biberidin]-1'-ylmethyl)pyridin-2-yl]methyl}cyclobutanecarboxamide

The same operation as in Example 1-18 was performed using the compound obtained in Reference Example 2-2 (40.0 mg) and cyclobutanecarboxylic acid (13.0 µL), to give a racemate (30.5 mg) of the title compound. Further, the product was purified by high-performance liquid chromatography (CHIRALPAK™ AS-H, hexane:ethanol:diethylamine = 80:20:0.02) to give the title compound (12.0 mg, faster) as a white amorphous substance. ¹HNMR (400 MHz, CDCl₃, δppm): 1.75-2.05 (6H, m), 2.13-2.35 (4H, m), 2.43-2.51 (2H, m), 2.77-2.83 (2H, m), 3.09-3.18 (1H, m), 3.58 (2H, s), 5.06 (2H, s), 6.05 (1H, d, J = 6.8 Hz), 7.03-7.15 (3H, m), 7.17-7.20 (2H, m), 7.51 (1H, d, J = 6.8 Hz), 7.69 (1H, dd, J = 7.8, 2.4 Hz), 8.44 (1H, s), 8.51 (1H, d, J = 4.9 Hz), 8.53 (1H, d, J = 2.4 Hz).
ESI-MS Found: m/z 505[M+H]⁺

### Example 1-45

### Synthesis of (R)- or (S)-N-{(3,4-difluorophenyl)[5-(1H,1'H-spiro[furo[3,4-c]pyridine-3,4'-piperidin]-1'-ylmethyl)pyridin-2-yl]methyl}-3-hydroxy-3-methylbutaneamide

The same operation as in Example 1-18 was performed using the compound obtained in Reference Example 2-2 (40.0 mg) and 3-hydroxy-3-methylbutanoic acid, to give a racemate (34.9 mg) of the title compound. Further, the product was purified by high-performance liquid chromatography (CHIRALPAK™ AD-H, hexane:isopropanol:diethylamine = 65:35:0.035) to give the title compound (13.8 mg, faster) as a colorless oil.
¹HNMR (400 MHz, CDCl₃, δppm): 1.23 (3H, s), 1.27 (3H, s), 1.78-1.83 (2H, m), 1.97-2.06 (2H, m), 2.43-2.52 (4H, m), 2.78-2.82 (2H, m), 3.59 (2H, s), 4.63 (1H, br s), 5.06 (2H, s), 6.10 (1H, d, J = 7.2 Hz), 7.08-7.20 (5H, m), 7.71 (1H, dd, J = 8.0, 2.0 Hz), 7.74 (1H, d, J = 7.2 Hz), 8.44 (1H, s), 8.51 (1H, d, J = 4.8 Hz), 8.52 (1H, d, J = 2.0 Hz).
ESI-MS Found: m/z 523[M+H]⁺

### Example 1-46

### Synthesis of (R)- or (S)-N-{(3,4-difluorophenyl)[5-(1H,1'H-spiro[furo[3,4-c]pyridine-3,4'-biberidin]-1'-ylmethyl)pyridin-2-yl]methyl}-3-hydroxy-N,3-dimethylbutaneamide

The same operation as in Example 1-45 was performed using the compound obtained in Reference Example 3-2 (35.4 mg), to give a racemate (27.8 mg) of the title compound. Further, the product was purified by high-performance liquid chromatography (CHIRALPAK™ AD-H, hexane:isopropanol:diethylamine = 60:40:0.04) to give the title compound (12.4 mg, slower) as a colorless oil.
¹HNMR (400 MHz, CDCl₃, δppm): 1.30 (3H, s), 1.33 (3H, s), 1.79-1.85 (2H, m), 1.98-2.10 (2H, m), 2.45-2.55 (2H, m), 2.54 (1H, d, J = 15.6 Hz), 2.61 (1H, d, J = 15.6 Hz), 2.77 (1/2H, s), 2.80-2.87 (2H, m), 2.94 (5/2H, s), 3.61 (2H, br s), 5.07 (2H, s), 5.21 (5/6H, br s), 5.37 (1/6H, br s), 6.87-6.96 (1H, m), 6.96-7.06 (1H, m), 7.10 (1H, s), 7.12-7.16 (1H, m), 7.19 (1H, d, J = 4.8 Hz), 7.23 (1H, d, J = 8.0 Hz), 7.70-7.80 (1H, m), 8.46 (1H, s), 8.52 (1H, d, J = 4.8 Hz), 8.57 (5/6H, d, J = 1.6 Hz), 8.61 (1/6H, d, J = 1.6 Hz).
ESI-MS Found: m/z 537[M+H]⁺

### Example 1-47

### Synthesis of (R)- or (S)-(2S)-N-{(3,4-difluorophenyl)[5-(1H,1'H-spiro[furo[3,4-c]pyridine-3,4'-biberidin]-1'-ylmethyl)pyridin-2-yl]methyl}-2-hydroxy-3,3-dimethylbutaneamide

The same operation as in Example 1-18 was performed using the compound obtained in Reference Example 2-2 (40.0 mg) and (2S)-2-hydroxy-3,3-dimethylbutanoic acid, to give the title compound (16.0 mg, polar) as a colorless oil.
¹HNMR (400 MHz, CDCl₃, δppm): 0.98 (9H, s), 1.73-1.83 (2H, m), 1.96-2.06 (2H, m), 2.43-2.52 (2H, m), 2.76-2.82 (2H, m), 3.31 (1H, br s), 3.58 (2H, s), 3.80 (1H, s), 5, 05 (2H, s), 6.09 (1H, d, J = 7.2 Hz), 7.06-7.22 (5H, m), 7.69 (1H, dd, J = 8.0, 2.0 Hz), 8.27 (1H, d, J = 7.2 Hz), 8.42 (1H, s), 8.50 (1H, d, J = 4.8 Hz), 8.53 (1H, d, J = 2.0 Hz).
ESI-MS Found: m/z 537[M+H]⁺

### Example 1-48

### Synthesis of (R)- or (S)-(2S)-N-{(3,4-difluorophenyl)[5-(1H,1'H-spiro[furo[3,4-c]pyridine-3,4'-biberidin]-1'-ylmethyl)pyridin-2-yl]methyl}-2-hydroxy-N,3,3-trimethylbutaneamide

The same operation as in Example 1-47 was performed using the compound obtained in Reference Example 3-2 (35.4 mg), to give the title compound (2.1 mg, less polar) as a colorless oil.
¹HNMR (400 MHz, CDCl₃, δppm): 0.95 (9H, s), 1.78-1.85 (2H, m), 1.98-2.07 (2H, m), 2.45-2.53 (2H, m), 2.80-2.86 (2H, m), 2.97 (3H, s), 3.27 (1H, d, J = 9.6 Hz), 3.61 (2H, s), 4.25 (1H, d, J = 9.6 Hz), 5.07 (2H, s), 6.95-7.00 (1H, m), 7.03-7.20 (4H, m), 7.24 (1H, d, J = 8.0 Hz), 7.73 (1H, dd, J = 8.0, 2.0 Hz), 8.46 (1H, s), 8.52 (1H, d, J = 4.8 Hz), 8.57 (1H, d, J = 2.0 Hz). ESI-MS Found: m/z 551[M+H]⁺

### Example 1-49

### Synthesis of (R)- or (S)-1-[(3,4-difluorophenyl)(5-{[4-(6-fluoropyridin-3-yl)piperidin-1-yl]methyl}pyridin-2-yl)methyl]pyrrolidin-2-one

To a methylene chloride solution (2.00 mL) of the compound obtained in Reference Example 2-3 (95.6 mg) were added triethylamine (162 µL) and 4-chlorobutanoyl chloride (39.0 µL), and the mixture was stirred at room temperature for 1 hour. An aqueous sodium hydrogen carbonate solution was added to the reaction mixture, followed by extraction with ethyl acetate, and the organic layer was dried over anhydrous magnesium sulfate. The organic layer was concentrated under reduced pressure to give a crude product of 4-chloro-N-[(3,4-difluorophenyl)(5-{[4-(6-fluoropyridin-3-yl)piperidin-1-yl]methyl}pyridin-2-yl)methyl]butaneamide. A 5.00 M aqueous sodium hydroxide solution (400 µL) was added to an ethanol solution (2.00 mL) of the obtained crude product, and stirred at room temperature overnight. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by silica gel column chromatography (chloroform:methanol = 100:0 to 80:20), to give a racemate (86.1 mg) of the title compound. Further, the product was purified by high-performance liquid chromatography (CHIRALPAK™ AD-H, hexane:ethanol:diethylamine = 40:60:0.06) to give the title compound (32.0 mg, faster) as a colorless oil.
¹HNMR (400 MHz, CDCl₃, δppm): 1.70-1.87 (4H, m), 1.95-2.08 (2H, m), 2.10-2.18 (2H, m),
2.44-2.50 (2H, m), 2.52-2.62 (1H, m), 2.98-3.04 (2H, m), 3.17-3.24 (1H, m), 3.56 (2H, s), 3.60-3.67 (1H, m), 6.54 (1H, s), 6.87 (1H, dd, J = 8.0, 2.9 Hz), 6.93-6.98 (1H, m), 7.01-7.07 (1H, m), 7.09-7.16 (1H, m), 7.23 (1H, d, J = 7.8 Hz), 7.64 (1H, td, J = 8.0, 2.0 Hz), 7.70 (1H, dd, J = 8.0, 2.0 Hz), 8.07 (1H, d, J = 2.0 Hz), 8.56 (1H, d, J = 2.0 Hz).
ESI-MS Found: m/z 481[M+H]⁺

### Example 1-50

### Synthesis of (R)- or (S)-N-{(3,4-difluorophenyl)[5-(1H,1'H-spiro[furo[3,4-c]pyridine-3,4'-piperidin]-1'-ylmethyl)pyridin-2-yl]methyl}cyclopropanecarboxamide

The same operation as in Example 1-1 was performed using the compound obtained in Reference Example 7-7 (13.4 mg) and cyclopropanecarbonyl chloride, to give the title compound (6.80 mg) as a colorless oil.
¹HNMR (400 MHz, CDCl₃, δppm): 0.73-0.79 (2H, m), 0.94-0.99 (2H, m), 1.51-1.59 (1H, m), 1.77-1.83 (2H, m), 1.97-2.06 (2H, m), 2.43-2.51 (2H, m), 2.78-2.84 (2H, m), 3.58 (2H, s), 5.06 (2H, s), 6.08 (1H, d, J = 6.8 Hz), 7.03-7.19 (5H, m), 7.69 (1H, dd, J = 8.0, 2.2 Hz), 7.78 (1H, d, J = 6.8 Hz), 8.45 (1H, s), 8.51 (1H, d, J = 4.9 Hz), 8.55 (1H, d, J = 2.2 Hz).
ESI-MS Found: m/z 491[M+H]⁺

### Example 1-51

### Synthesis of (R)- or (S)-N-{1-(3,4-difluorophenyl)-1-[5-(1H,1'H-spirorfuro[3,4-c]pyridine-3,4'-piperidin]-1'-ylmethyl)pyridin-2-yl]ethyl}acetamide

The same operation as in Example 1-1 was performed using the compound obtained in Reference Example 7-12 (195 mg) and acetic anhydride, to give the title compound (190 mg) as a white amorphous substance.
¹HNMR (400 MHz, DMSO-d₆, δppm): 1.62-1.67 (2H, m), 1.90-1.99 (5H, m), 2.01 (3H, s), 2.30-2.38 (2H, m), 2.67-2.73 (2H, m), 3.53 (2H, s), 4.97 (2H, s), 7.09-7.14 (1H, m), 7.24-7.40 (4H, m), 7.69 (1H, dd, J = 8.2, 2.0 Hz), 8.45 (1H, d, J = 4.7 Hz), 8.47 (1H, d, J = 2.0 Hz), 8.53 (1H, s), 8.59 (1H, s).
ESI-MS Found: m/z 479[M+H]⁺

### Example 1-52

### Synthesis of (R)- or (S)-N-{1-(3,4-difluorobhenyl)-1-[5(1H,1'H-spiro[furo[3,4c]pyridine-3,4' piperidin-1'-ylmethyl)pyridin-2-yl]ethyl}-N-methylacetamide

The same operation as in Example 1-51 was performed using the compound obtained in Reference Example 7-32 (13.6 mg), to give title compound (9.52 mg) as a white solid. ¹HNMR (400 MHz, DMSO-d₆, δppm): 1.62-1.68 (2H, m), 1.91-1.97 (2H, m), 1.98 (3H, s), 2.00-2.09 (3H, m), 2.30-2.38 (2H, m), 2.68-2.74 (2H, m), 2.87 (3H, s), 3.52 (2H, s), 4.97 (2H, s), 7.09-7.14 (1H, m), 7.23-7.41 (4H, m), 7.61-7.65 (1H, m), 8.40 (1H, s), 8.45 (1H, d, J = 4.7 Hz), 8.53 (1H, s).
ESI-MS Found: m/z 493[M+H]⁺

### Example 1-53

### Synthesis of (R)- or (S)-N-{1-(3,4-difluorobhenyl)-1-[5-(1H,1'H-spiro[furo[3,4-c]pyridine-3,4' piperidin]-1'-ylmethyl)pyridin-2-yl)propyl}acetamide

The same operation as in Example 1-51 was performed using the compound obtained in Reference Example 7-35 (30.0 mg), to give the title compound (31.7 mg) as a colorless oil.
¹HNMR (400 MHz, DMSO-d₆, δppm): 0.51 (3H, t, J = 7.2 Hz), 1.58-1.64 (2H, m), 1.86-1.94 (2H, m), 2.26-2.34 (2H, m), 2.45-2.50 (2H, m), 2.62-2.68 (2H, m), 3.12 (3H, s), 3.50 (2H, s), 4.93 (2H, s), 7.13-7.18 (1H, m), 7.22-7.28 (2H, m), 7.28-7.30 (1H, m), 7.36-7.42 (1H, m), 7.66 (1H, dd, J = 8.4, 2.0 Hz), 8.41 (1H, d, J = 4.7 Hz), 8.44 (1H, d, J = 2.0 Hz), 8.48 (1H, s), 8.55 (1H, s).
ESI-MS Found: m/z 493[M+H]⁺

### Example 1-54

### Synthesis of (R)- or (S)-N-{cycloproyl(3,4-difluorophenyl)[5-(1H,1'H-spiro[furo[3,4-c]pyridine-3,4'-piperidin]-1'-ylmethyl)pyridin-2-yl]methyl}acetamide

The same operation as in Example 1-51 was performed using the compound obtained in Reference Example 7-36 (25.0 mg), to give the title compound (19.9 mg) as a colorless oil.
¹HNMR (400 MHz, DMSO-d₆, δppm): -0.05-0.00 (1H, m), 0.02-0.09 (1H, m), 0.37-0.47 (1H, m), 0.53-0.61 (1H, m), 1.62-1.67 (2H, m), 1.90-1.98 (5H, m), 2.17-2.24 (1H, m), 2.30-2.38 (2H, m), 2.65-2.72 (2H, m), 3.54 (2H, s), 4.97 (2H, s), 6.88 (1H, d, J = 8.2 Hz), 7.25-7.30 (1H, m), 7.31-7.39 (2H, m), 7.45-7.52 (1H, m), 7.64 (1H, dd, J = 8.4, 2.2 Hz), 8.45 (1H, d, J = 5.1 Hz), 8.48-8.53 (3H, m).
ESI-MS Found: m/z 505[M+H]⁺

### Example 1-55

### Synthesis of (R)- or (S)-N-{1-(4-chloro-3,5-difluorophenyl)-1-[5-(1H,1'H-spiro[furo[3,4-c]pyridine-3,4'-piperidin]-1'-ylmethyl)pyridin-2-yl]ethyl}acetamide trifluoroacetate

The same operation as in Example 1-51 was performed using the compound obtained in Reference Example 7-20 (10.0 mg), to give a crude product of the title compound. The product was purified by reversed-phase high-performance liquid chromatography (YMC-ODS, 0.1% TFA-70% methanol-30 % acetonitrile:0.1% TFA-water = 37:63 to 67:33) to give the title compound (4.00 mg) as a colorless solid.
¹HNMR (300 MHz, CD₃OD, δppm): 1.89 (3H, s), 1.92 (3H, s), 1.98-2.06 (2H, m), 2.14-2.28 (2H, m), 3.20-3.42 (4H, m), 4.32 (2H, s), 5.12 (2H, s), 7.03 (2H, d, J = 8.7 Hz), 7.28 (1H, d, J = 8.4 Hz), 7.62 (1H, d, J = 5.4 Hz), 7.82 (1H, d, J = 8.1 Hz), 8.51-8.66 (3H, m).
ESI-MS Found: m/z 513, 515[M+H]⁺

### Example 1-56

### Synthesis of (R)- or (S)-N-{1-(3,4-difluorophenyl)-2,2-difluoro-1-[5-(1H,1'H-spiro[furo[3,4-c]pyridine-3,4'-piperidin]-1'-ylmethyl)pyridin-2-yl]ethyl}acetamide trifluoroacetate

To a THF solution (5.00 mL) of the compound obtained in Reference Example 7-25 (25.0 mg) was added a 1.00 M THF solution (0.20 mL) of lithium bis(trimethylsilyl)amide in a nitrogen atmosphere at -78°C, and the mixture was stirred for 30 minutes. Acetic anhydride (20.0 mg) was added to the reaction solution, and stirred for 30 minutes. The reaction mixture was added dropwise to ice water, followed by extraction with ethyl acetate. The organic layer was washed with water and then dried over anhydrous sodium sulfate. The organic layer was concentrated under reduced pressure, and then the residue was purified by reversed-phase high-performance liquid chromatography (YMC-ODS, 0.1% TFA-70% methanol-30% acetonitrile:0.1% TFA-water = 34:66 to 64:36) to give the title compound (5.00 mg) as a colorless solid.
¹HNMR (400 MHz, CD₃OD, δppm): 2.05 (3H, s), 2.11-2.15 (2H, m), 2.38-2.45 (2H, m), 3.45-3.59 (4H, m), 4.52 (2H, s), 5.29 (2H, s), 6.98-7.27 (4H, m), 7.41 (1H, d, J = 8.4 Hz), 7.81 (1H, d, J = 9.2 Hz), 8.02 (1H, dd, J = 8.4, 2.0 Hz), 8.68-8.72 (2H, m), 8.81 (1H, s).
ESI-MS Found: m/z 515[M+H]⁺

### Example 1-57

### Synthesis of (R)- or (S)-N-{1-(3,4-difluorophenyl)-2,2,2-trifluoro-1-[5-(1H,1'H-spirorfuror3,4-c]pyridine-3,4'-piperidin]-1'-ylmethyl)pyridin-2-yl]ethyl}acetamide trifluoroacetate

The same operation as in Example 1-56 was performed using the compound obtained in Reference Example 7-29 (25.0 mg), to give a crude product of the title compound. The product was purified by reversed-phase high-performance liquid chromatography (YMC-ODS, 0.1% TFA-acetonitrile:0.1% TFA-water = 30:70 to 60:40) to give the title compound (5.00 mg) as a colorless solid.
¹HNMR (400 MHz, CD₃OD, δppm): 2.11 (3H, s), 2.11-2.15 (2H, m), 2.40-2.45 (2H, m), 3.45-3.55 (4H, m), 4.53 (2H, s), 5.30 (2H, s), 7.24-7.28 (2H, m), 7.34-7.40 (2H, m), 7.84 (1H, d, J = 5.4 Hz), 8.03 (1H, d, J = 6.4 Hz), 8.70-8.74 (2H, m), 8.84 (1H, s).
ESI-MS Found: m/z 533[M+H]⁺

### Example 1-58

### Synthesis of (R)- or (S)-N-[1-(3,4-difluorophenyl)-1-(5-{[4-(6-fluoropyridin-3-yl)piperidin-1-yl]methyl}pyridin-2-yl)ethyl]-2,2-difluoroacetamide

The same operation as in Example 1-1 was performed using the compound obtained in Reference Example 7-39 (20.0 mg) and difluoroacetic anhydride, to give the title compound (22.9 mg) as a colorless oil.
¹HNMR (400 MHz, CDCl₃, δppm): 1.69-1.86 (4H, m), 2.10-2.18 (5H, m), 2.52-2.61 (1H, m), 2.94-3.01 (2H, m), 3.55 (2H, s), 5.85 (1H, t, J = 54.4 Hz), 6.87 (1H, dd, J = 8.8, 2.9 Hz), 6.93 (1H, d, J = 8.3 Hz), 7.06-7.18 (3H, m), 7.61-7.68 (2H, m), 8.06 (1H, d, J = 2.4 Hz), 8.50 (1H, d, J = 1.5 Hz), 9.86 (1H, s).
ESI-MS Found: m/z 505[M+H]⁺

### Example 1-59

### Synthesis of (R)- or (S)-N-(1-(3,4-difluorophenyl)-1-{5-[5-methyl-6-oxo-5,6-dihydro-1H',3H-spiro[furo[3,4-c]pyridine-1,4'-piperidin]-1'-yl)methyl]pyridin-2-yl}ethyl)-2,2-difluoroacetamide

The same operation as in Example 1-58 was performed using the compound obtained in Reference Example 7-13 (26.1 mg), to give the title compound (21.6 mg) as a colorless oil.
¹HNMR (400 MHz, CDCl₃, δppm): 1.73-1.79 (2H, m), 1.81-1.90 (2H, m), 2.12 (3H, s), 2.38-2.46 (2H, m), 2.72-2.79 (2H, m), 3.53 (3H, s), 3.56 (2H, s), 4.82 (2H, s), 5.85 (1H, t, J = 54.6 Hz), 6.34 (1H, s), 6.94 (1H, d, J = 8.3 Hz), 7.06-7.18 (4H, m), 7.67 (1H, dd, J = 8.3, 2.0 Hz), 8.49 (1H, d, J = 2.0 Hz), 9.86 (1H, s).
ESI-MS Found: m/z 545[M+H]⁺

### Example 1-60

### Synthesis of (R)- or (S)-N-{1-(3,4-difluorophenyl)-1-[5-(1H,1'H-spiro[furo[3,4-c]pyridine-3,4'-piperidin]-1'-ylmethyl)pyridin-2-yl]ethyl}-2-hydroxy-2-methylpropanamide

The same operation as in Example 1-34 was performed using the compound obtained in Reference Example 7-12 (28.0 mg), to give the title compound (14.4 mg) as a white amorphous substance.
¹HNMR (400 MHz, DMSO-d₆, δppm): 1.20 (3H, s), 1.24 (3H, s), 1.61-1.67 (2H, m), 1.90-1.98 (2H, m), 2.04 (3H, s), 2.30-2.38 (2H, m), 2.65-2.71 (2H, m), 3.54 (2H, s), 4.96 (2H, s), 7.15-7.20 (1H, m), 7.25 (1H, d, J = 8.3 Hz), 7.28-7.36 (2H, m), 7.38-7.45 (1H, m), 7.72 (1H, dd, J = 8.3, 2.2 Hz), 8.45 (1H, d, J = 5.1 Hz), 8.49-8.53 (2H, m), 9.72 (1H, s).
ESI-MS Found: m/z 523[M+H]⁺

### Example 2-1

### Synthesis of 1-{(3,4-difluorophenyl)[4-(1H,1'H-spiro[furo[3,4-c]pyridine-3,4'-piperidin]-1'-ylmethyl)phoyl]methyl}pyrrolidin-2-one

To an acetic acid solution (1.00 mL) of the compound obtained in Reference Example 4-1 (52.0 mg) were added 2-pyrrolidone (182 µL) and concentrated sulfuric acid (18.7 µL) at room temperature, and the mixture was stirred at 130°C for 2 days. The reaction mixture was cooled to room temperature and then concentrated under reduced pressure. The residue was diluted with ethyl acetate, and then an aqueous sodium hydrogen carbonate solution was added thereto to make it alkaline. The mixed solution was extracted with ethyl acetate, and the organic layer was dried over anhydrous sodium sulfate. The organic layer was concentrated under reduced pressure, and then the residue was purified by preparative thin-layer chromatography (chloroform:methanol = 90:10) to give the title compound (38.5 mg) as a colorless oil.
¹HNMR (400 MHz, CDCl₃, δppm): 1.79-1.85 (2H, m), 2.03-2.09 (2H, m), 2.13-2.25 (2H, m), 2.48-2.54 (2H, m), 2.56-2.65 (2H, m), 2.99-3.07 (2H, m), 3.16-3.21 (2H, m), 3.74 (2H, s), 5.07 (2H, s), 6.56 (1H, s), 6.89-6.95 (1H, m), 6.95-7.01 (1H, m), 7.11-7.18 (3H, m), 7.20-7.23 (1H, m), 7.39 (2H, d, J = 8.0 Hz), 8.48 (1H, s), 8.53 (1H, d, J = 5.1 Hz).
ESI-MS Found: m/z 490[M+H]⁺

### Example 2-2

### Synthesis of 1-[[4-(1H,1'H-spiro[furo[3,4-c]pyridine-3,4'-piberidin]-1'-ylmethyl)phenyl(2,4,5-trifluorophenyl)methyl]pyrrolidin-2-one

The same operation as in Example 2-1 was performed using the compound obtained in Reference Example 5-4 (75.1 mg), to give the title compound (38.1 mg) as a white amorphous substance.
¹HNMR (400 MHz, CDCl₃, δppm): 1.79-1.82 (2H, m), 2.00-2.11 (4H, m), 2.42-2.52 (4H, m), 2.83-2.85 (2H, m), 3.17-3.23 (2H, m), 3.58 (2H, s), 5.06 (2H, s), 6.64 (1H, s), 6.91-6.99 (2H, m), 7.08 (2H, d, J = 8.0 Hz), 7.18 (1H, d, J = 4.0 Hz), 7.35 (2H, d, J = 8.0 Hz), 8.45 (1H, s), 8.51 (1H, d, J = 4.0 Hz).
ESI-MS Found: m/z 508[M+H]⁺

### Example 2-3

### Synthesis of 2-{(3,4-difluorophenyl)[4-(1H,1'H-spiro[furo[3,4-c]pyridine-3,4'-piperidin]-1'-ylmethyl)phenyl]methyl}isoindolin-1-one

To a TFA solution (1.00 mL) of the compound obtained in Reference Example 4-1 (30.3 mg) was added isoindolin-1-one (14.3 mg) at room temperature, and the mixture was stirred using microwaves at 150°C for 30 minutes. The reaction mixture was cooled to room temperature and then concentrated under reduced pressure. The residue was diluted with ethyl acetate, and then an aqueous sodium hydrogen carbonate solution was added thereto to make it alkaline. The mixed solution was extracted with ethyl acetate, followed by drying over anhydrous sodium sulfate. The organic layer was concentrated under reduced pressure, and then the residue was purified by preparative thin-layer chromatography (chloroform:methanol = 90:10) to give the title compound (19.5 mg) as a colorless oil.
¹HNMR (400 MHz, CDCl₃, δppm): 1.77-1.83 (2H, m), 1.98-2.08 (2H, m), 2.40-2.49 (2H, m), 2.82-2.88 (2H, m), 3.59 (2H, s), 4.16-4.28 (2H, m), 5.06 (2H, s), 6.84 (1H, s), 6.93-6.98 (1H, m), 6.99-7.06 (1H, m), 7.10-7.20 (4H, m), 7.36-7.41 (3H, m), 7.47-7.58 (2H, m), 7.92 (1H, d, J = 7.3 Hz), 8.45 (1H, s), 8.51 (1H, d, J = 5.4 Hz).
ESI-MS Found: m/z 538[M+H]⁺

### Example 2-4

### Synthesis of (R)- or (S)-(3S)-1-{(3,4-difluorophenyl)[4-(1H,1'H-spiro[furo[3,4-c]pyridine-3,4'-piperidin]-1'-ylmethyl)phoyl]methyl}-3-hydroxypyrrolidin-2-one

The same operation as in Example 2-1 was performed using the compound obtained in Reference Example 4-1 (220 mg) and (3S)-3-hydroxypyrrolidin-2-one (263 mg) to give a crude product of (3S)-1-{(3,4-difluorophenyl)[4-(1H,1'H-spiro[furo[3,4-c]pyridine-3,4'-piperidin]-1'-ylmethyl)phenyl]methyl}-2-oxopyrrolidin-3-yl acetate. A 5.00 M aqueous sodium hydroxide solution (4.00 µL) was added to an ethanol solution (4.00 mL) of the obtained crude product, and stirred at room temperature for 1 hour. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by silica gel column chromatography (chloroform:methanol = 100:0 to 80:20), to give a racemate(236 mg) of the title compound. Further, the product was purified by high-performance liquid chromatography (CHIRALPAK™ AS-H, hexane:ethanol:diethylamine = 60:40:0.04) to give the title compound (102 mg, slower) as a yellow solid.
¹HNMR (400 MHz, CDCl₃, δppm): 1.78-1.84 (2H, m), 1.95-2.08 (3H, m), 2.42-2.51 (3H, m), 2.82-2.89 (2H, m), 3.03-3.10 (1H, m), 3.23-3.29 (1H, m), 3.60 (2H, s), 4.41-4.46 (1H, m), 5.06 (2H, s), 6.50 (1H, s), 6.91-7.03 (2H, m), 7.07-7.19 (4H, m), 7.37 (2H, d, J = 7.8 Hz), 8.45 (1H, s), 8.51 (1H, d, J = 4.9 Hz).
ESI-MS Found: m/z 506[M+H]⁺

### Example 2-5

### Synthesis of (3R)- or (3 5)-[(R)- or (S)-1-{(3,4-difluorophenyl)[4-(1H,1'H-spiro[furo[3,4-c]pyridine-3,4'-piperidin]-1'-ylmethyl)phenyl]methyl}]-3-fluoropyrrolidin-2-one

The same operation as in Example 2-1 was performed using the compound obtained in Reference Example 4-1 (30.0 mg) and the compound obtained in Reference Example 9-3, to give a diastereomeric mixture (36.0 mg) of the title compound. Further, the product was purified by high-performance liquid chromatography (CHIRALPAK™ AD-H, hexane:ethanol:diethylamine = 50:50:0.05) to give the title compound (5.91 mg, second fraction) as a colorless oil.
¹HNMR (400 MHz, CDCl₃, δppm): 1.78-1.84 (2H, m), 1.99-2.09 (2H, m), 2.17-2.33 (1H, m), 2.40-2.53 (3H, m), 2.81-2.87 (2H, m), 3.07-3.14 (1H, m), 3.32-3.38 (1H, m), 3.59 (2H, s), 5.06 (2H, s), 5.17 (1H, ddd, J = 52.4, 7.6, 6.1 Hz), 6.53 (1H, s), 6.90-6.95 (1H, m), 6.95-7.01 (1H, m), 7.12 (2H, d, J = 7.8 Hz), 7.15-7.20 (2H, m), 7.38 (2H, d, J = 7.8 Hz), 8.45 (1H, s), 8.51 (1H, d, J = 4.9 Hz).
ESI-MS Found: m/z 508[M+H]⁺

### Example 3-1

### Synthesis of 3-{(3,4-difluorophenyl)[4-(1H,1'H-spiro[furo[3,4-c]pyridine-3,4'-piperidin]-1'-ylmethyl)phenyl]methyl}-1-methylimidazolidine-2,4-dione

To a THF solution (1.00 mL) of the compound obtained in Reference Example 4-1 (20.0 mg) and 1-methylimidazolidine-2,4-dione (16.2 mg) were added tri-n-butylphosphine (35.0 µL) and 1,1'-azobis(N,N-dimethylformamide) (12.2 mg) at room temperature, and the mixture was stirred for 2 hour. An aqueous sodium hydrogen carbonate solution was added to the reaction mixture, followed by extraction with ethyl acetate, and the organic layer was dried over anhydrous magnesium sulfate. The organic layer was concentrated under reduced pressure, and then the residue was purified by preparative thin-layer chromatography (chloroform:methanol = 90:10) to give the title compound (3.00 mg) as a white amorphous substance.
¹HNMR (400 MHz, CDCl₃, δppm): 1.77-1.83 (2H, m), 1.97-2.08 (2H, m), 2.38-2.48 (2H, m), 2.81-2.89 (2H, m), 3.01 (3H, s), 3.58 (2H, s), 3.90 (2H, s), 5.06 (2H, s), 6.41 (1H, s), 7.05-7.24 (4H, m), 7.28-7.38 (4H, m), 8.45 (1H, s), 8.51 (1H, d, J = 4.9 Hz).
ESI-MS Found: m/z 519[M+H]⁺

### Example 3-2

### Synthesis of 1-{(3,4-difluorophenyl)[4-(1H,1'H-spiro[furo[3,4-c]pyridine-3,4'-piperidin]-1'-ylmethyl)phenyl]methyl}pyridin-2(1H)-one

The same operation as in Example 3-1 was performed using the compound obtained in Reference Example 4-1 (50.0 mg) and 2-hydroxypyridine (24.0 µL), to give the title compound (10.6 mg) as a white amorphous substance.
¹HNMR (400 MHz, CDCl₃, δppm): 1.77-1.83 (2H, m), 1.98-2.09 (2H, m), 2.41-2.51 (2H, m), 2.81-2.88 (2H, m), 3.59 (2H, s), 5.06 (2H, s), 6.16 (1H, td, J = 6.7, 1.1 Hz), 6.63 (1H, d, J = 9.3 Hz), 6.86-6.91 (1H, m), 6.92-6.98 (1H, m), 7.07-7.19 (5H, m), 7.32-7.41 (3H, m), 7.44 (1H, s), 8.45 (1H, s), 8.51 (1H, d, J = 4.9 Hz).
ESI-MS Found: m/z 500[M+H]⁺

### Example 3-3

### Synthesis of (R)- or (S)-1-{(3,4-difluorophenyl)[4-(1H,1'H-spiro[furo[3,4-c]pyridine-3,4'-piperidin1-1'-ylmethyl)phenyl]methyl}-3-methoxypyridin-2(1H)-one

The same operation as in Example 3-1 was performed using the compound obtained in Reference Example 4-1 (90.0 mg) and 3-methoxy-2(1H)-pyridone (26.7 mg), to give a racemate (22.4 mg) of the title compound. Further, the product was purified by high-performance liquid chromatography (CHIRALPAK™ AD-H, hexane:ethanol:diethylamine = 20:80:0.08) to give the title compound (6.5 mg, faster) as a white solid.
¹HNMR (400 MHz, CDCl₃, δppm): 1.77-1.83 (2H, m), 1.97-2.07 (2H, m), 2.40-2.48 (2H, m), 2.80-2.86 (2H, m), 3.57 (2H, s), 3.83 (3H, s), 5.06 (2H, s), 6.09 (1H, t, J = 7.1 Hz), 6.61 (1H, dd, J = 7.3, 1.6 Hz), 6.74 (1H, dd, J = 7.1, 1.6 Hz), 6.87-6.93 (1H, m), 6.94-6.97 (1H, m), 7.08-7.19 (4H, m), 7.36 (2H, d, J = 8.3 Hz), 7.52 (1H, s), 8.45 (1H, s), 8.51 (1H, d, J = 4.9 Hz).
ESI-MS Found: m/z 530[M+H]⁺

### Example 3-4

### Synthesis of (R)- or (S)-1-{(3,4-difluorophenyl)[4-(1H,1'H-spiro[furo[3,4-c]pyridine-3,4'-piperidin]-1'-ylmethyl)phenyl]methyl}-3-methoxypyridin-2(1H)-one

The same operation as in Example 3-1 was performed using the compound obtained in Reference Example 4-1 (90.0 mg) and 4-methoxy-2(1H)-pyridone (26.7 mg), to give a racemate (21.9 mg) of the title compound. Further, the product was purified by high-performance liquid chromatography (CHIRALPAK™ AD-H, hexane:isopropanol:diethylamine = 20:80:0.08) to give the title compound (4.7 mg, slower) as a white solid.
¹HNMR (400 MHz, CDCl₃, δppm): 1.77-1.83 (2H, m), 1.98-2.06 (2H, m), 2.40-2.48 (2H, m), 2.80-2.87 (2H, m), 3.58 (2H, s), 3.78 (3H, s), 5.06 (2H, s), 5.89 (1H, dd, J = 7.8, 2.9 Hz), 5.95 (1H, d, J = 2.9 Hz), 6.85-6.97 (3H, m), 7.07-7.19 (4H, m), 7.36-7.38 (3H, m), 8.45 (1H, s), 8.51 (1H, d, J = 5.4 Hz).
ESI-MS Found: m/z 530[M+H]⁺

### Example 4-1

### Synthesis of 1-((3,4-difluorophenyl){4-[(6-fluoro-1H,1'H-spiro[furo[3,4-c]pyridine-3,4'-piperidin]-1'-yl)methyl]phenyl}methyl)pyrrolidin-2-one

The same operation as in Reference Example 1-4 was performed using the compound obtained in Reference Example 8-2 (25.0 mg) and 6-fluoro-1H-spiro[furo[3,4-c]pyridine-3,4'-piperidine] hydrochloride (19.3 mg), to give the title compound (13.9 g) as a white amorphous substance.
¹HNMR (400 MHz, CDCl₃, δppm): 1.22-1.28 (2H, m), 1.79-1.85 (2H, m), 1.96-2.09 (4H, m), 2.40-2.45 (4H, m), 3.16-3.21 (2H, m), 3.58 (2H, s), 5.04 (2H, s), 6.55 (1H, s), 6.75-6.78 (1H, m), 6.89-6.94 (1H, m), 6.94-7.01 (1H, m), 7.10-7.17 (3H, m), 7.35 (2H, d, J = 7.8 Hz), 7.99 (1H, s).
ESI-MS Found: m/z 508[M+H]⁺

### Example 4-2

### Synthesis of 1-((3,4-difluorophenyl){4-[(4-[1,2,4]triazolo[4,3-a]pyridin-7-ylpiperidin-1-yl)methyl]phenyl}methyl)pyrrolidin-2-one

The same operation as in Reference Example 1-4 was performed using the compound obtained in Reference Example 8-2 (25.0 mg) and 7-piperidin-4-yl[1,2,4]triazolo[4,3-a]pyridine hydrochloride (21.7 mg), to give the title compound (15.3 mg) as a white amorphous substance.
¹HNMR (400 MHz, CDCl₃, δppm): 1.78-1.93 (4H, m), 2.01-2.09 (2H, m), 2.10-2.18 (2H, m), 2.47-2.52 (2H, m), 2.54-2.63 (1H, m), 3.02-3.08 (2H, m), 3.16-3.21 (2H, m), 3.56 (2H, s), 6.55 (1H, s), 6.79 (1H, dd, J = 7.1, 1.3 Hz), 6.89-7.01 (2H, m), 7.10-7.18 (3H, m), 7.34 (2H, d, J = 8.0 Hz), 7.57 (1H, s), 8.04 (1H, d, J = 7.1 Hz), 8.75 (1H, s).
ESI-MS Found: m/z 502[M+H]⁺

### Example 4-3

### Synthesis of (R)- or (S)-N-((3,4-difluorophenyl){5-[(4-pyrazolo[1,5-b]pyridazin-3-ylpiperidin-1-yl)methyl]pyridin-2-yl}methyl)-2-hydroxy-N,2-dimethylpropanamide

The same operation as in Reference Example 1-4 was performed using the compound obtained in Reference Example 8-5 (72.7 mg) and 3-piperidin-4-ylpyrazolo[1,5-b]pyridazine hydrochloride, to give 2-[((3,4-difluorophenyl){5-[(4-pyrazolo[1,5-b]pyridazin-3-ylpiperidin-1-yl)methyl]pyridin-2-yl}methyl)(methyl)amino]-1,1-dimethyl-2-oxoethyl acetate (79.0 mg). Further, a 5.00 M aqueous sodium hydroxide solution (1.00 mL) was added to an ethanol solution (5.00 mL) of the obtained compound, and stirred at room temperature for 2 hours. The reaction mixture was extracted with chloroform. The organic layer was washed with water and saturated brine, and dried over anhydrous sodium sulfate. The organic layer was concentrated under reduced pressure, and then the residue was purified by preparative thin-layer chromatography (chloroform:methanol = 95:5) to give a racemate (66.7 mg) of the title compound. Further, the product was purified by high-performance liquid chromatography (CHIRALPAK™ AD-H, hexane:ethanol:diethylamine = 60:40:0.04) to give the title compound (29.4 mg, slower) as a colorless oil.
¹HNMR (400 MHz, CDCl₃, δppm): 1.56 (6H, s), 1.82-1.96 (4H, m), 2.15-2.23 (2H, m), 2.75-2.85 (1H, m), 2.98-3.05 (2H, m), 3.07 (3H, s), 3.59 (2H, s), 4.46 (1H, br s), 6.91 (1H, dd, J = 8.8, 4.8 Hz), 6.91-6.96 (1H, m), 6.98-7.04 (2H, m), 7.12-7.20 (1H, m), 7.22 (1H, d, J = 7.6 Hz), 7.74 (1H, d, J = 7.6 Hz), 7.90 (1H, s), 7.94 (1H, dd, J = 8.8, 1.6 Hz), 8.23 (1H, dd, J = 4.8, 1.6 Hz), 8.57 (1H, s).
ESI-MS Found: m/z 535[M+H]⁺

### INDUSTRIAL APPLICABILITY

The compound of the invention has MCH-1R antagonism, and is useful as a preventive or a remedy for metabolic diseasessuch as obesity, diabetes, hormone secretion disorder, hyperlipemia, gout, fatty liver, hepatitis, liver cirrhosis, and like; circulatory diseases such as angina pectoris, acute/congestive cardiac insufficiency, myocardial infarction, coronary arteriosclerosis, hypertension, nephropathy, electrolyte abnormality, and like; central and peripheral nervous system diseases such as bulimia, affective disorder, depression, anxiety, epilepsy, delirium, dementia, schizophrenia, attention deficit/hyperactivity disorder, dysmnesia, somnipathy, cognitive impairment, dyskinesia, dysesthesia, dysosmia, morphine resistance, drug dependence, alcohol dependence, and like; reproductive system diseases such as infertility, premature delivery, sexual dysfunction, and like; and other conditions including digestive diseases, respiratory diseases, cancer, chromatosis, and the like. The compound of the invention is especially useful as a preventive or a remedy for obesity, diabetes, fatty liver, bulimia, depression, or anxiety.

## Claims

1. A diarylmethylamide derivative represented by formula (I) or a pharmaceutically acceptable salt thereof: 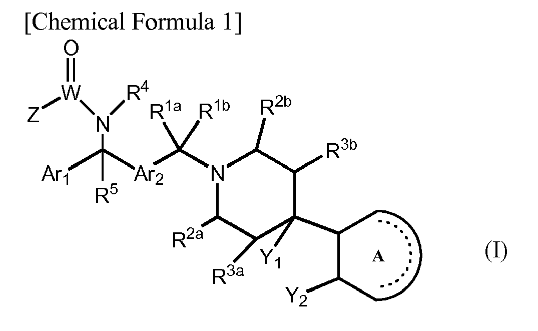 wherein:
R^{1a} and R^{1b} independently represent a hydrogen atom or C₁₋₆ alkyl,
R^{2a} and R^{2b} independently represent a hydrogen atom or C₁₋₆ alkyl,
R^{3a} and R^{3b} independently represent a hydrogen atom or C₁₋₆ alkyl,
R⁴ represents a hydrogen atom, hydroxy, C₁₋₆ alkyl, C₃₋₆ cycloalkyl, or C₁₋₆ alkoxy, wherein the alkyl, cycloalkyl, or alkoxy being optionally substituted with halogen, hydroxy, or C₁₋₆ alkoxy,
R⁵ represents a hydrogen atom, C₁₋₆ alkyl, or C₃₋₆ cycloalkyl, wherein the alkyl or cycloalkyl being optionally substituted with halogen, hydroxy, or C₁₋₆ alkoxy,
Z represents C₁₋₆ alkyl, C₃₋₆ cycloalkyl, C₁₋₆ alkoxy, aryl, heteroaryl, or N(R^{6a})(R^{6b}), wherein the alkyl, cycloalkyl, alkoxy, aryl, or heteroaryl being optionally substituted with halogen, hydroxy, C₁₋₆ alkyl, halo C₁₋₆ alkyl, C₁₋₆ alkoxy, or halo C₁₋₆ alkoxy, or,
R⁴ and Z together form, together with the nitrogen atom to which R⁴ is bonded, a 4- to 6-membered nitrogen-containing hetero ring, wherein the nitrogen-containing hetero ring optionally containing a double bond in the ring and optionally further containing a heteroatom selected from the group consisting of nitrogen, oxygen, and sulfur, the nitrogen-containing hetero ring being optionally fused with an aryl ring or a heteroaryl ring, and the nitrogen-containing hetero ring being optionally substituted with halogen, hydroxy, C₁₋₆ alkyl, halo C₁₋₆ alkyl, C₁₋₆ alkoxy, halo C₁₋₆ alkoxy, or oxo,
R^{6a} and R^{6b} independently represent a hydrogen atom or C₁₋₆ alkyl, or, R^{6a} and R^{6b} together form, together with the nitrogen atom to which they are bonded, a 5- to 6-membered nitrogen-containing hetero ring, wherein the nitrogen-containing hetero ring optionally further containing a heteroatom selected from the group consisting of nitrogen, oxygen, and sulfur and being optionally substituted with halogen, hydroxy, C₁₋₆ alkyl, halo C₁₋₆ alkyl, C₁₋₆ alkoxy, halo C₁₋₆ alkoxy, or oxo,
Y₁ represents H or -OR^{7a},
Y₂ represents H, or Y₁ and Y₂ together form -O-C(R^{7b})(R^{7c})-,
R^{7a}, R^{7b}, and R^{7c} each independently represent a hydrogen atom or C₁₋₆ alkyl,
W represents C, S, or SO,
Ar₁ represents 6-membered aryl or 6-membered nitrogen-containing heteroaryl, wherein the aryl or nitrogen-containing heteroaryl being optionally substituted with a substituent selected from the group consisting of group α,
Ar₂ is a divalent group and represents 6-membered aryl or 5- to 6-membered heteroaryl, wherein the aryl or heteroaryl being optionally substituted with a substituent selected from the group consisting of group α, and
a formula: 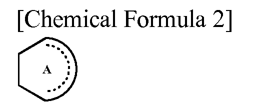 represents a 6-membered aryl ring or a 5- to 6-membered nitrogen-containing hetero ring, wherein the aryl ring or nitrogen-containing hetero ring being optionally fused with a 5- to 6-membered aryl ring or heteroaryl ring, and wherein the aryl ring or nitrogen-containing hetero ring being optionally substituted with halogen, cyano, hydroxy, C₁₋₆ alkyl, halo C₁₋₆ alkyl, C₁₋₆ alkoxy, halo C₁₋₆ alkoxy, C₁₋₆ alkylcarbonylamino, or oxo. Substituents of group α are:
halogen, cyano, hydroxy, amino, mono C₁₋₆ alkylamino, di-C₁₋₆ alkylamino, C₁₋₆ alkyl, halo C₁₋₆ alkyl, C₁₋₆ alkoxy, halo C₁₋₆ alkoxy, C₁₋₆ alkoxy C₁₋₆ alkyl, C₁₋₆ alkoxycarbonyl, C₁₋₆ alkoxycarbonylamino, C₁₋₆ alkoxycarbonyl(C₁₋₆ alkyl)amino, C₁₋₆ alkylcarbonyl, C₁₋₆ alkylcarbonyloxy, C₁₋₆ alkylcarbonylamino, C₁₋₆ alkylcarbonyl(C₁₋₆ alkyl)amino, carbamoyl, mono-C₁₋₆ alkylcarbamoyl, di-C₁₋₆ alkylcarbamoyl, carbamoylamino, mono-C₁₋₆ alkylcarbamoylamino, di-C₁₋₆ alkylcarbamoylamino, mono-C₁₋₆ alkylcarbamoyl(C₁₋₆ alkyl)amino, di-C₁₋₆ alkylcarbamoyl(C₁₋₆ alkyl)amino, carbamoyloxy, mono-C₁₋₆ alkylcarbamoyloxy, di-C₁₋₆ alkylcarbamoyloxy, C₁₋₆ alkylsulfonyl, C₁₋₆ alkylsulfonylamino, C₁₋₆ alkylsulfonyl(C₁₋₆ alkyl)amino, sulfamoyl, mono-C₁₋₆ alkylsulfamoyl, di-C₁₋₆ alkylsulfamoyl, sulfamoylamino, mono-C₁₋₆ alkylsulfamoylamino, di-C₁₋₆ alkylsulfamoylamino, mono-C₁₋₆ alkylsulfamoyl(C₁₋₆ alkyl)amino, and di-C₁₋₆ alkylsulfamoyl(C₁₋₆ alkyl)amino.

2. The compound or the pharmaceutically acceptable salt thereof according to claim 1, wherein R^{1a} and R^{1b} are independently a hydrogen atom or methyl.

3. The compound or the pharmaceutically acceptable salt thereof according to claim 1 or 2, wherein R^{2a} and R^{2b} are both hydrogen atoms.

4. The compound or the pharmaceutically acceptable salt thereof according to any one of claims 1 to 3, wherein R^{3a} and R^{3b} are both hydrogen atoms.

5. The compound or the pharmaceutically acceptable salt thereof according to any one of claims 1 to 4, wherein R⁴ is a hydrogen atom, methyl, 2-hydroxy-2-methylpropyl, cyclopropyl, or 2-hydroxy-2-methylpropyloxy.

6. The compound or the pharmaceutically acceptable salt thereof according to any one of claims 1 to 5, wherein W is C or SO.

7. The compound or the pharmaceutically acceptable salt thereof according to any one of claims 1 to 6, wherein Z is methyl, ethyl, isopropyl, difluoromethyl, 1-hydroxy,-1-methylethyl, 1-hydroxycyclopropyl, phenyl, or isoxazolyl.

8. The compound or the pharmaceutically acceptable salt thereof according to any one of claims 1 to 4, wherein R⁴ and Z together form, together with the nitrogen atom to which R⁴ is bonded, one of the following rings: 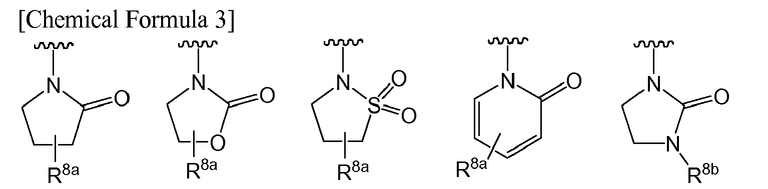 wherein R^{8a} represents a hydrogen atom, halogen, hydroxy, C₁₋₆ alkyl, halo C₁₋₆ alkyl, C₁₋₆ alkoxy, or halo C₁₋₆ alkoxy, and R^{8b} represents a hydrogen atom, C₁₋₆ alkyl, or halo C₁₋₆ alkyl.

9. The compound or the pharmaceutically acceptable salt thereof according to claim 8, wherein R^{8a} is a hydrogen atom, a fluorine atom, hydroxy, or methoxy, and R^{8b} is a hydrogen atom or methyl.

10. The compound or the pharmaceutically acceptable salt thereof according to any one of claims 1 to 9, wherein R⁵ is a hydrogen atom, methyl, ethyl, fluoromethyl, difluoromethyl, trifluoromethyl, or cyclopropyl.

11. The compound or the pharmaceutically acceptable salt thereof according to any one of claims 1 to 10, wherein Y₁ and Y₂ are both hydrogen atoms.

12. The compound or the pharmaceutically acceptable salt thereof according to any one of claims 1 to 10, wherein Y₁ and Y₂ together form -O-CH₂-.

13. The compound or the pharmaceutically acceptable salt thereof according to any one of claims 1 to 12, wherein Ar₂ is phenylenediyl, pyridinediyl, or pyrimidinediyl.

14. The compound or the pharmaceutically acceptable salt thereof according to claim 13, wherein Ar₂ is 1,4-phenylenediyl, pyridine-2,5-diyl, or pyrimidine-2,5-diyl.

15. The compound or the pharmaceutically acceptable salt thereof according to any one of claims 1 to 14, wherein Ar₁ is 6-membered aryl substituted with one to three fluorine atoms or chlorine atoms or is 6-membered nitrogen-containing heteroaryl substituted with one to three fluorine atoms or chlorine atoms.

16. The compound or the pharmaceutically acceptable salt thereof according to claim 15, wherein Ar₁ is phenyl substituted with one to three fluorine atoms or chlorine atoms or is pyridyl substituted with one to three fluorine atoms or chlorine atoms.

17. The compound or the pharmaceutically acceptable salt thereof according to any one of claims 1 to 16, wherein the formula: 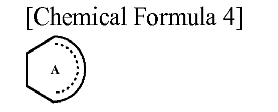 is selected from the group consisting of the following formulae: 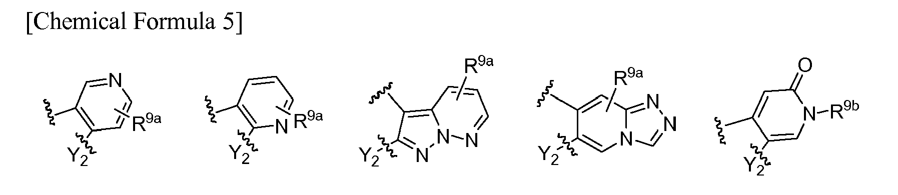 wherein R^{9a} represents a hydrogen atom, halogen, cyano, hydroxy, C₁₋₆ alkyl, halo C₁₋₆ alkyl, C₁₋₆ alkoxy, halo C₁₋₆ alkoxy, or C₁₋₆ alkylcarbonylamino, R^{9b} represents a hydrogen atom, C₁₋₆ alkyl, or halo C₁₋₆ alkyl, and Y₂ is as defined above.

18. The compound or the pharmaceutically acceptable salt thereof according to claim 17, wherein R^{9a} is a hydrogen atom or a fluorine atom, and R^{9b} is a hydrogen atom or methyl.

19. The compound or the pharmaceutically acceptable salt thereof according to claim 1, wherein the compound represented by formula (I) is selected from the group consisting of:
N-{(3,4-difluorophenyl)[4-(1H,1'H-spiro[furo[3,4-c]pyridine-3,4'-piperidin]-1'-ylmethyl)phenyl]methyl} acetamide,
N-{(3,4-difluorophenyl)[5-(1H,1'H-spiro[furo[3,4-c]pyridine-3,4'-piperidin]-1'-ylmethyl)pyridin-2-yl]methyl}-N-(2-hydroxy-2-methylpropyloxy)acetamide,
N-{(3,4-difluorophenyl)[5-(1H,1'H-spiro[furo[3,4-c]pyridine-3,4'-piperidin]-1'-ylmethyl)pyridin-2-yl]methyl}-N-(2-hydroxy-2-methylpropyl)-2-methylpropanamide,
N-{(3,4-difluorophenyl)[4-(1H,1'H-spiro[furo[3,4-c]pyridine-3,4'-piperidin]-1'-ylmethyl)phenyl]methyl}benzamide,
N-{(3,4-difluorophenyl)[4-(1H,1'H-spiro[furo[3,4-c]pyridine-3,4'-piperidin]-1'-ylmethyl)phenyl]methyl}isoxazol-5-carboxamide,
3-{(3,4-difluorophenyl)[4-(1H,1'H-spiro[furo[3,4-c]pyridine-3,4'-piperidin]-1'-ylmethyl)phenyl]methyl}-1,3-oxazolidin-2-one,
1'-{4-[(3,4-difluorophenyl)(1,1-dioxideisothiazolidin-2-yl)methyl]benzyl}-5-methyl-3,5-dihydro-6H-spiro[furo[3,4-c]pyridine-1,4'-piperidin]-6-one,
1-{(3,4-difluorophenyl)[4-(1H,1'H-spiro[furo[3,4-c]pyridine-3,4'-piperidin]-1'-ylmethyl)phenyl]methyl}-3-methylimidazolidin-2-one,
(R)- or (S)-N-{(5-chloropyridin-2-yl)[4-(1H,1'H-spiro[furo[3,4-c]pyridine-3,4'-piperidin]-1'-ylmethyl)phenyl]methyl}propanamide,
(R)- or (S)-N-[(3,4-difluorophenyl)(5-{[4-(6-fluoropyridin-3-yl)piperidin-1-yl]methyl}pyridin-2-yl)methyl]-2-hydroxy-N,2-dimethylpropanamide,
(R)- or (S)-N-cyclopropyl-N-{(3,4-difluorophenyl)[5-(1H,1'H-spiro[furo[3,4-c]pyridine-3,4'-piperidin]-1'-ylmethyl)pyridin-2-yl]methyl}--2-hydroxy-2-methylpropanamide, (R)- or (S)-N-{(3,4-difluorophenyl)[5-(1H,1'H-spiro[furo[3,4-c]pyridine-3,4'-piperidin]-1'-ylmethyl)pyrimidin-2-yl]methyl}--2-hydroxy-N,2-dimethylpropanamide,
(R)- or (S)-N-((3,4-difluorophenyl){4-[(4-pyrazolo[1,5-b]pyridazin-3-ylpiperidin-1-yl)methyl]phenyl}methyl)-1-hydroxycyclopropanecarboxamide,
(R)- or (S)-N-{cyclopropyl(3,4-difluorophenyl)[5-(1H,1'H-spiro[furo[3,4-c]pyridine-3,4'-piperidin]-1'-ylmethyl)pyridin-2-yl]methyl}acetamide,
(R)- or (S)-N-[1-(3,4-difluorophenyl)-1-(5-{[4-(6-fluoropyridin-3-yl)piperidin-1-yl]methyl}pyridin-2-yl)ethyl]-2,2-difluoroacetamide,
1-{(3,4-difluorophenyl)[4-(1H,1'H-spiro[furo[3,4-c]pyridine-3,4'-piperidin]-1'-ylmethyl)phenyl]methyl}pyrrolidin-2-one,
1-[[4-(1H,1'H-spiro[furo[3,4-c]pyridine-3,4'-piperidin]-1'-ylmethyl)phenyl](2,4,5-trifluorophenyl)methyl]pyrrolidin-2-one,
(3R)- or (3S)-[(R)- or (S)-1-{(3,4-difluorophenyl)[4-(1H,1'H-spiro[furo[3,4-c]pyridine-3,4'-piperidin]-1'-ylmethyl)phenyl]methyl}]-3-fluoropyrrolidin-2-one,
1-{(3,4-difluorophenyl)[4-(1H,1'H-spiro[furo[3,4-c]pyridine-3,4'-piperidin]-1'-ylmethyl)phenyl]methyl}pyridin-2(1H)-one,
1-((3,4-difluorophenyl){4-[(6-fluoro-1H,1'H-spiro[furo[3,4-c]pyridine-3,4'-piperidin]-1'-yl)methyl]phenyl}methyl)pyrrolidin-2-one,
1-((3,4-difluorophenyl){4-[(4-[1,2,4]triazolo[4,3-a]pyridin-7-ylpiperidin-1-yl)methyl]phenyl}methyl)pyrrolidin-2-one,
(R)- or (S)-N-{1-(4-chloro-3,5-difluorophenyl)-1-[5-(1H,1'H-spiro[furo[3,4-c]pyridine-3,4'-piperidin]-1'-ylmethyl)pyridin-2-yl]ethyl}acetamide,
(R)- or (S)-N-{1-(3,4-difluorophenyl)-2,2-difluoro-1-[5-(1H,1'H-spiro[furo[3,4-c]pyridine-3,4'-piperidin]-1'-ylmethyl)pyridin-2-yl]ethyl}acetamide, and
(R)- or (S)-N-{1-(3,4-difluorophenyl)-2,2,2-trifluoro-1-[5-(1H,1'H-spiro[furo[3,4-c]pyridine-3,4'-piperidin]-1'-ylmethyl)pyridin-2-yl]ethyl}acetamide.

20. A melanin-concentrating hormone receptor antagonist comprising as an active ingredient the compound or the pharmaceutically acceptable salt thereof according to any one of claims 1 to 19.

21. A pharmaceutical composition comprising a pharmaceutically acceptable additive and the compound or the pharmaceutically acceptable salt thereof according to any one of claims 1 to 19.

22. A preventive or remedy for obesity, diabetes, fatty liver, bulimia, depression, or anxiety, comprising as an active ingredient the compound or the pharmaceutically acceptable salt thereof according to any one of claims 1 to 19.

23. A medicine based on MCH receptor antagonism, comprising as an active ingredient the compound or the pharmaceutically acceptable salt thereof according to any one of claims 1 to 19.
